# EUROPEAN PATENT SPECIFICATION

(11) **EP 3 793 517 B1**
(45) Date of publication and mention of the grant of the patent: **19.04.2023**
(21) Application number: 19728321.1
(22) Date of filing: 15.05.2019
(51) Int. Cl.: A61K 9/00, A61K 9/70, A61K 47/36, A61K 31/352

(54) **PHARMACEUTICAL FORMULATION**
PHARMAZEUTISCHE FORMULIERUNG
FORMULATION PHARMACEUTIQUE

(30) Priority: 16.05.2018 GB 201807942
(43) Date of publication of application: 24.03.2021
(73) Proprietor: Klaria Pharma Holding AB, 754 50 Uppsala (SE)
(72) Inventor: BOYER, Scott, 754 50 Uppsala (SE); HÜBINETTE, Fredrik, 754 50 Uppsala (SE); SINGH, Shalini, 754 50 Uppsala (SE)
(74) Representative: J A Kemp LLP
(86) International application number: PCT/EP2019/062534
(87) International publication number: WO 2019/219773

(56) References cited:
- WO-A1-03/101357
- WO-A1-2007/073346
- WO-A1-2017/180707
- WO-A2-2007/125533
- US-A1- 2016 051 510

## Description

### Field of the Invention

The present invention relates to a film comprising an alginate salt of a monovalent cation or a mixture of alginate salts containing at least one alginate salt of a monovalent cation, and one or more cannabinoids, such as Δ⁹-tetrahydrocannabinol (THC) or cannabidiol (CBD), or pharmaceutically acceptable salts thereof. The present invention further relates to methods for manufacturing such a film, and the use of such a film in the treatment of disease.

### Background to the Invention

Cannabinoids are a diverse class of chemical compounds which act on cannabinoid receptors in cells. Cannabinoid receptors are membrane-bound G-protein coupled receptors (GPCRs). At present, there are two recognised types of cannabinoid receptors, CB₁ and CB₂ receptors, although there is also evidence of further cannabinoid receptor types. [1]

CB₁ receptors are primarily located in the central nervous system (CNS) and peripheral nervous system (PNS), although are also found in both male and female reproductive systems. CB₁ receptors have been implicated in various conditions, including the maintenance of homeostasis, the function of the stress response, gastrointestinal activity, cardiovascular activity, neuronal plasticity, depression, motor control, and drug and behaviour addictions. Selective antagonists of the CB₁ receptor have been developed, such as rimonabant, and are used in patients for (e.g.) weight reduction and smoking cessation.

CB₂ receptors are primarily located in the immune system, or immune-derived cells, with the greatest density in the spleen. CB₂ receptors are thought to effect the immunological activity of leukocytes; specifically, these receptors are implicated in a variety of modulatory functions, such as immune suppression, induction of apoptosis, and induction of cell migration. [2] They have also been implicated in the regulation of homing and retention of marginal zone B cells. [3] Modulation of CB₂ receptor activity may have possible therapeutic roles in the treatment of neurodegenerative disorders (such as Alzheimer's disease), cardiovascular disease, gastrointestinal conditions, liver disease, kidney disease, psychiatric disease, bone disease, skin disease, autoimmune diseases, lung disorders, pain, and cancer.

Cannabinoids may act as agonists, inverse agonists or antagonists of any of the different cannabinoid receptors, such as CB₁ and CB₂ receptors.

The cannabinoids can be broadly grouped into three different classes based on their origin, although these classes contain within them a wide variety of chemical structures: (i) phytocannabinoids; (ii) endocannabinoids; and (iii) synthetic cannabinoids.

Phytocannabinoids (sometimes referred to as "classical cannabinoids") are cannabinoids which can be obtained from plants, notably the *Cannabis* plant genus (comprising *Cannabis sativa, Cannabis indica,* and *Cannabis ruderalis*)*.* They are produced in the glandular trichromes of the plant as a viscous resin. At least 113 compounds have been isolated from the *Cannabis* plant. The most prevalent and well-studied of these compounds include tetrahydrocannabinol (THC) and cannabidiol (CBD). THC is the primary psychoactive component of the *Cannabis* plant. Delta-9-THC (Δ⁹-THC) and delta-8-THC (Δ⁸-THC) are CB₁ agonists, and are thought to induce effects such as euphoria, cognitive impairment and anxiety through activation of CB₁ receptors in the brain. Δ⁹-THC is used medically as an appetite stimulant, anti-emetic and analgesic. In contrast, CBD is a non-psychotropic agent with little affinity for CB₁ and CB₂ receptors, but may act as an indirect antagonist of cannabinoid agonists, mitigating the effects associated with such agonists. [4]

Endocannabinoids are substances produced within the body that activate cannabinoid receptors. Common endocannabinoids include anandamide (AEA), 2-arachidonylglycerol (2-AG), 2-arachidonyl glyceryl ether (noladin ether), N-arachidonoyl dopamine (NADA), virodhamine (OAE) and lysophosphatidylinositol (LPI). Endocannabinoids are signalling molecules which serve as intracellular "lipid messengers" that are released from one cell and activate cannabinoid receptors on other nearby cells. In contrast to many neurotransmitters, endocannabinoids can act as retrograde transmitters, which travel in the opposite direction to ordinary synaptic transmitter flow. Thus, endocannabinoids are in effect released from a postsynaptic neurone and act on the presynaptic neurone, where the target receptors are often densely concentrated on axonal terminals in the zones from which conventional neurotransmitters are released. Activation of cannabinoid receptors temporarily reduces the amount of conventional neurotransmitter released. The downstream effect of this signalling depends on the neurotransmitter secretion being inhibited. For example, if endocannabinoid activation decreases the release of the inhibitory transmitter GABA, the net effect is an increase in the excitation of the (endocannabinoid-releasing) postsynaptic neurone.

Cannabinoids can also be produced synthetically. Many synthetic cannabinoids are structural analogues of naturally-occurring cannabinoids, such as THC. Other synthetic cannabinoids are more structurally disparate (so-called "nonclassical" cannabinoids or "cannabimimetics"), including aminoalkylindoles, 1,5-diarylpyrazoles, quinolones, and arylsulfonamides as well as icosanoids (which are related to endocannabinoids). Synthetic cannabinoids include nabilone (an analog of marinol), rimonabant (a selective CB₁ receptor inverse agonist used as an anti-obesity drug and to encourage smoking cessation), JWH-018, JWH-073, CP-55940, dimethylheptylpyran, HU-210, HU-331 (a potential anti-cancer drug derived from CBD that specifically inhibits topoisomerase II), SR144528 (a CB₂ receptor antagonist), WIN 55,212-2, JWH-133 (a selective CB₂ receptor agonist), levonantradol (also known as nantodolum, an anti-emetic and analgesic), AM-2201, and ajulenic acid.

Cannabinoids may play a role in homeostasis and treatment of disease by reducing excitotoxicity, reducing oxidative damage, and enhancing anandamine levels. [5] Cannabinoids present an interesting therapeutic potential in several areas, including their possible use as antiemetics, appetite stimulants (e.g. in debilitating diseases such as cancer and AIDS), analgesics, and in the treatment of multiple sclerosis, spinal cord injuries, Tourette's syndrome, epilepsy and glaucoma. [6]

However, cannabinoids are often highly lipophilic and generally have very low aqueous solubility and fat solubility, which poses difficulties for the administration of cannabinoids to patients. Cannabinoids can be stored in fat deposits in the body which reduces their bioavailability. Further, most cannabinoids are metabolised in the liver, especially by cytochrome P450 mixed-function oxidases. [7]

Cannabinoids derived from natural sources are often administered to a patient either in the form of dried plant material (e.g. via cigarettes) or as a concentrated oil. Commonly utilised methods for cannabinoid administration include inhalation (i.e. smoking or vapourising), oral administration and transdermal administration. For example, nabiximols is an oral spray which comprises both Δ⁹-THC and CBD, delivering a dose of 2.7 mg Δ⁹-THC and 2.5 mg CBD per spray.

Each of the existing methods of administration however suffers from significant drawbacks. Smoking cannabinoids is considered by many people to be unpleasant, and is associated with a low dose accuracy and a high degree of variation in the dose delivered to the lungs of patients. Further, many of the chemicals in smoke are known carcinogens, and smoking cannabis may be associated with bronchial irritation and bronchodilation. [8][9] Vapourising is considered to be safer than inhaling smoke, and allows for an improved control of dosage by the patient, but is still associated with a high variation in the dose delivered to the lungs, and is still often considered unpleasant. Oral formulations, meanwhile, are associated with very poor bioavailability and a high time to onset. A significant portion of the administered dose is hydrolysed in the acidic environment of the stomach, and yet more of the dose is subjected to first-pass metabolism in the liver. In particular, Δ⁹-THC is metabolised in the liver to Δ¹¹-THC. Δ¹¹-THC possesses enhanced psychoactivity in comparison to Δ⁹-THC, but is less easily uptaken across the blood-brain barrier. Thus, patients who take edible cannabis are at risk of overconsumption which can cause an undesirably high psychoactive effect. Transdermal delivery (in the form of a patch or gel applied directly to the skin) is more pleasant for the patient than inhaled or oral administration. However, transdermal delivery is associated with a high time to onset (>1 hour). [10]

In summary, no cannabinoid formulation is currently available which can be administered in fashion that is not unpleasant to the patient, does not carry the risks of carcinogen inhalation, gives a short time to onset, and which results in acceptable bioavailability and blood plasma concentrations of cannabinoid with low variability between patients.

WO 2017/180707 describes mucosally dissolvable films containing a matrix and one or more active agents from hemp or cannabis. WO 03/101357 describes a method of transmucosally delivering a cannabinoid to a subject. US 2016/051510 describes orally dissolvable films the include botanical extract. WO 2007/125533 describes orally administrable mucoadhesive films comprising one or more bioactive ingredients and at least one alginate capable of forming a low viscosity aqueous solution. WO 2007/073346 describes the use of a specific type of sodium alginate in forming oral transmucosal films.

### Summary of the Invention

The present invention is based on the unexpected finding that formulations of cannabinoids or pharmaceutically acceptable salts thereof, in a film suitable for administration to an oral cavity can provide a desirable balance of properties for use in the treatment of a wide range of conditions.

Hence, the invention provides for the first time a film suitable for administration to an oral cavity comprising one or more cannabinoids or pharmaceutically acceptable salts thereof, its use in the treatment of patients suffering from conditions which can be treated or alleviated by cannabinoids, and methods for its manufacture.

In one aspect, the present invention provides a film suitable for administration to an oral cavity comprising:
(i) an alginate salt of a monovalent cation or a mixture of alginate salts containing at least one alginate salt of a monovalent cation; and
(ii) an active pharmaceutical ingredient (API) which is one or more cannabinoids or pharmaceutically acceptable salts thereof,
wherein the alginate salt of a monovalent cation comprises from 25 to 35% by weight of β-D-mannuronate and from 65 to 75% by weight of α-L-guluronate.

In another aspect, the present invention provides a film according to the invention for use in the treatment of a human patient.

In another aspect, the present invention provides a film according to the invention for use in the treatment of a disease or condition in a human patient, wherein the disease or condition is selected from the group consisting of: cancer; dementia; Alzheimer's disease; amyotrophic lateral sclerosis; dystonia; epilepsy; Huntington's disease; multiple sclerosis; Parkinson's disease; spasticity; Tourette's syndrome; irritable bowel disease (IBD); Crohn's disease; ulcerative colitis; anorexia; cachexia; cancer-induced nausea and/or vomiting; cancer-induced cachexia; glaucoma; chronic pain; cancer-induced pain; fibromyalgia; neuropathic pain; addiction; anxiety; bipolar disorder; post-traumatic stress disorder; psychosis; schizophrenia; scleroderma; and type I diabetes.

In another aspect, the present invention provides a method of manufacturing a film according to the invention, said method comprising the following steps:
(a) either the steps of:
   (i) optionally, mixing at least one antioxidant in water, or in a mixed aqueous/organic solvent, or in one or more organic solvents;
   (ii) mixing the API in water, or in a mixed aqueous/organic solvent, or in one or more organic solvents to which water is subsequently added, or in the solution obtained in step (i), optionally wherein the pH of the solution is adjusted either before or after the addition of the API to the desired level by addition of an appropriate acid or base, typically a diluted aqueous acid or alkali, more typically a diluted aqueous alkali, and preferably wherein the pH of the solution is adjusted to from 3.0 to 13.5;
   (iii) optionally, sonicating the solution;
   (iv) optionally, mixing one or more excipients, flavouring agents, buffering components, permeation enhancers, SEDDS (e.g. SMEDDS or SNEDDS), chelating agents, antioxidants, and/or antimicrobial agents into the solution obtained in step (ii) or step (iii); and
   (v) adding the alginate salt of monovalent cation under suitable conditions to result in the formation of a viscous cast;
   or alternatively the steps of:
   (i) mixing the alginate salt of monovalent cation in water, until a lump free dispersion is achieved, and optionally adding one or more excipients, flavouring agents, buffering components, permeation enhancers, SEDDS (e.g. SMEDDS or SNEDDS), chelating agents, antioxidants and/or antimicrobial agents to the aqueous solution either before or after the addition of the alginate salt;
   (ii) separately, dissolving the API in water, a mixed aqueous/organic solvent or one or more organic solvent(s), optionally wherein at least one antioxidant is pre-dissolved in the solvent, optionally wherein the pH of the solution is adjusted either before or after the addition of the API to the desired level by addition of an appropriate acid or base, typically a diluted aqueous acid or alkali, more typically a diluted aqueous alkali, and preferably wherein the pH of the solution is adjusted to from 3.0 to 13.5; and
   (iii) adding the solution obtained in step (i) to the solution obtained in step (ii) under suitable conditions to result in the formation of a viscous cast;
   or alternatively the steps of:
   (i) mixing the API in an oil phase;
   (ii) premixing a surfactant and a cosolvent, and then adding this to the solution obtained;
   (iii) optionally, adding one or more excipients, flavouring agents, buffering components, permeation enhancers, chelating agents, antioxidants and/or antimicrobial agents to water in step (i) under mixing;
   (iv) adding water, or the solution obtained in step (iii), to the solution obtained in step (ii) under stirring, preferably continuous stirring, and more preferably wherein the water or the solution obtained in step (iii) is added in a dropwise fashion; and
   (v) mixing the alginate salt of monovalent cation in the solution, until a lump free dispersion is achieved, and optionally adding further water to modulate the viscosity of the cast formed;
   or alternatively the steps of:
   (i) mixing the API in a solubilizing agent;
   (ii) adding the resultant solution to water, preferably under high shear mixing;
   (iii) optionally, adding one or more excipients, flavouring agents, buffering components, permeation enhancers, chelating agents, antioxidants and/or antimicrobial agents; and
   (iv) mixing the alginate salt of monovalent cation in the solution, until a lump free dispersion is achieved, and optionally adding further water to modulate the viscosity of the cast formed;
(b) optionally, adding one or more further excipients, flavouring agents, buffering components, permeation enhancers, SEDDS (e.g. SMEDDS or SNEDDS), chelating agents, antioxidants, and/or antimicrobial agents to the cast obtained in step (a);
(c) optionally, leaving the cast to de-aerate;
(d) pouring the cast onto a surface and spreading the cast out to the desired thickness;
(e) drying the cast layer, typically at a temperature of from 30 to 70 °C, until the residual water content of the film is from 0 to 20% by weight and a solid film is formed; and
(f) optionally, cutting the solid film into pieces of the desired size, further optionally placing these pieces into pouches, preferably wherein the pouches are made from PET-lined aluminium, sealing the pouches and further optionally, labelling them.

### Brief Description of the Figures

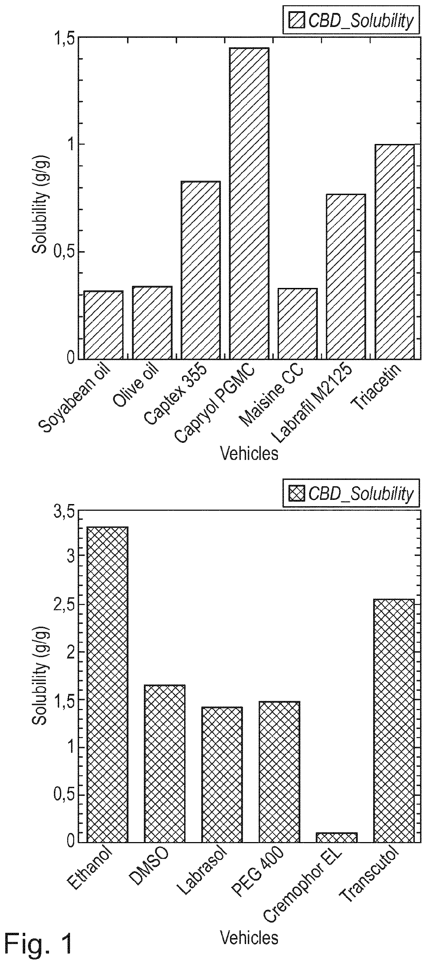

Fig. 1 shows bar charts that represent the solubility of CBD (g/g) in various different solvents.

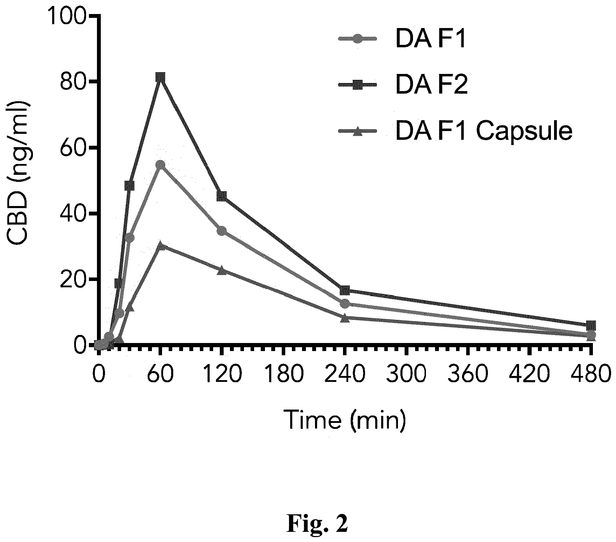

Fig. 2 shows dose-adjusted plasma levels of CBD in the blood plasma of adult beagle dogs (n=3) over a time period of 0 to 480 minutes after administration of an emulsion-based CBD film (F1), a non-emulsion-based CBD film (F2) or an emulsion-based CBD film encapsulated in a gelatin tablet for oral administration (F3). All dose levels were adjusted to 10 mg (9.93 mg actual) dose equivalents.

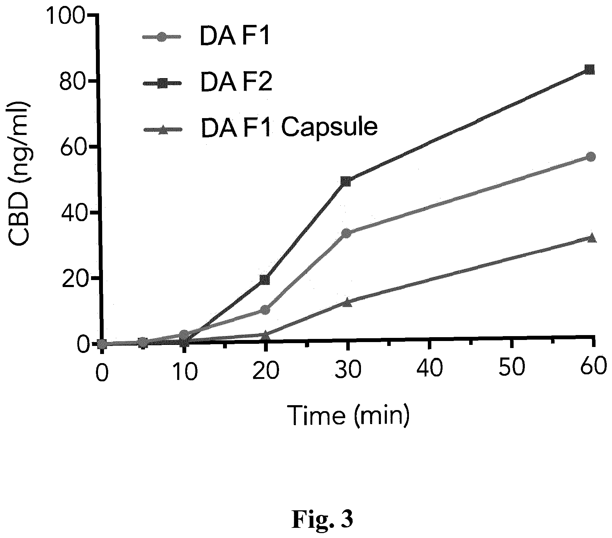

Fig. 3 shows dose-adjusted plasma levels of CBD in the blood plasma of adult beagle dogs (n=3) over a time period of 0 to 60 minutes after administration of an emulsion-based CBD film (F1), a non-emulsion-based CBD film (F2) or an emulsion-based CBD film encapsulated in a gelatin tablet for oral administration (F3). All dose levels were adjusted to 10 mg (9.93 mg actual) dose equivalents.

### Detailed Description of the Invention

The present invention is concerned with a film, suitable for administration to an oral cavity, which can be used for delivery of a cannabinoid or a pharmaceutically acceptable salt thereof to a human patient. Such a film may also be referred to as an oral dissolvable film (ODF) and/or an oral transmucosal film (OTF). The film is typically an alginate film which is applied by the patient themselves or another person, e.g. a medical practitioner, a nurse, a carer, a social worker, a colleague of the patient or a family member of the patient, to the mucosa of the oral cavity. The film is bioadhesive and adheres to the surface of the oral cavity upon application. After application, the alginate film begins to dissolve, releasing the active pharmaceutical ingredient. The present invention is useful in particular in the treatment of disease or conditions such as dementia, Alzheimer's disease, epilepsy, inflammatory bowel disease, Crohn's disease, ulcerative colitis, chronic pain, cancer-induced pain, fibromyalgia, and neuropathic pain.

For the avoidance of doubt, all alternative and preferred features relating to the film *per se* apply equally to the use of said film in the treatment of a human patient.

### Definitions

As defined herein, the term "alkyl" refers to a linear or branched saturated monovalent hydrocarbon radical having the number of carbon atoms indicated in the prefix. Thus, the term "C₁₋₆ alkyl" refers to a linear saturated monovalent hydrocarbon radical of one to six carbon atoms or a branched saturated monovalent hydrocarbon radical of three to six carbon atoms, e.g. methyl, ethyl, *n*-propyl, *iso*-propyl, *n*-butyl, *iso*-butyl, *tert*-butyl, *n*-pentyl and all structural isomers thereof, and *n*-hexyl and all structural isomers thereof.

As defined herein, the term "alkenyl" refers to a linear or branched saturated monovalent hydrocarbon radical having the number of carbon atoms indicated in the prefix and containing at least one double bond. Thus, the term "C₂₋₆ alkenyl" refers to a linear saturated monovalent hydrocarbon radical of one to six carbon atoms having at least one double bond, or a branched saturated monovalent hydrocarbon radical of three to six carbon atoms having at least one double bond, e.g. ethenyl, propenyl, 1,3-butadienyl, (CH₂)₂CH=C(CH₃)₂, CH₂CH=CHCH(CH₃)₂, and the like.

As defined herein, the term "acyl" refers to a -COR radical, wherein R is alkyl, haloalkyl, cycloalkyl, cycloalkylalkyl, aryl, aralkyl, heteroaryl, heteroaralkyl, heterocyclyl, orheterocyclylalkyl, each as defined herein, or poly(ethylene glycol), and wherein R is optionally further substituted with one, two, three, four or more substituents independently selected from alkyl, alkoxy, halo, haloalkoxy, -OH, -NH₂, alkylamino, -COOH, or alkoxycarbonyl.

As defined herein, the term "alkylene" refers to a linear saturated divalent hydrocarbon radical or a branched saturated divalent hydrocarbon radical, e.g. methylene, ethylene, propylene, 1-methylpropylene, 2-methylpropylene, butylene, pentylene, and the like.

As defined herein, the term "alkoxy" refers to an -OR radical where R is alkyl as defined above, e.g., methoxy, ethoxy, *n*-propoxy, *iso-*propoxy, *n*-butyl, *iso*-butyl, *tert*-butyl and the like.

As defined herein, the term "alkoxycarbonyl" refers to a -C(O)OR radical where R is alkyl, haloalkyl, cycloalkyl, cycloalkylalkyl, aryl, aralkyl, heteroaryl, heteroaralkyl, heterocyclyl, or heterocyclylalkyl, each as defined herein, or poly(ethylene glycol), and wherein R is optionally further substituted with one, two, three, four or more substituents independently selected from alkyl, alkoxy, halo, haloalkoxy, -OH, -NH₂, alkylamino, -COOH, or alkoxycarbonyl.

As defined herein, the term "alkylamino" refers to an -NHR radical where R is alkyl as defined above, e.g. methylamino, ehtylamino, n-propylamino, *iso*-propylamino, and the like.

As defined herein, the term "aryl" refers to a monovalent monocyclic or bicyclic aromatic hydrocarbon radical of 6 to 10 ring atoms, e.g. phenyl or naphthyl, and the like.

As defined herein, the term "aralkyl" refers to an -(alkylene)-R radical where R is aryl as defined above.

As defined herein, the term "cannabidiolic acid-Cs ester" refers to a moiety having the following structure: 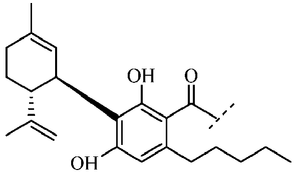 wherein the dashed line (-----) represents the point of contact to the remainder of the molecule.

As defined herein, the term "carbamate" refers to a -C(O)NR^{x}R^{y} radical where R^{x} and R^{y} are independently hydrogen, alkyl, haloalkyl, cycloalkyl, cycloalkylalkyl, aryl, aralkyl, heteroaryl, heteroaralkyl, heterocyclyl, or heterocyclylalkyl, each as defined herein, or poly(ethylene glycol), and wherein R^{x} and R^{y} are optionally further substituted with one, two, three, four or more substituents independently selected from alkyl, alkoxy, halo, haloalkoxy, -OH, -NH₂, alkylamino, -COOH, or alkoxycarbonyl.

As defined herein, the term "cycloalkyl" refers to a cyclic saturated monovalent hydrocarbon radical of three to ten carbon atoms wherein one or two carbon atoms may be replaced by an oxo group, e.g. cyclopropyl, cyclobutyl, cyclopentyl, or cyclohexyl, and the like.

As defined herein, the term "cycloalkylalkyl" refers to an -(alkylene)-R radical where R is cycloalkyl as defined above, e.g. cyclopropylmethyl, cyclobutylmethyl, cyclopentylethyl, or cyclohexylmethyl, and the like.

As defined herein, the term "halo" refers to fluoro, chloro, bromo, or iodo, preferably fluoro or chloro.

As defined herein, the term "haloalkyl" refers to an alkyl radical as defined above, which is substituted with one or more halogen atoms, preferably one to five halogen atoms, preferably fluorine or chlorine, including those substituted with different halogens, e.g. -CH₂Cl, -CF₃, -CHF₂, -CH₂CF₃, -CF₂CF₃, -CF(CH₃)₂, and the like.

As defined herein, the term "haloalkoxy" refers to an -OR radical where R is haloalkyl as defined above, e.g. -OCF₃, -OCHF₂, and the like.

As defined herein, the term "heteroaryl" refers to a monovalent monocyclic or bicyclic aromatic radical of 5 to 10 ring atoms where one or more, preferably one, two, or three, ring atoms are heteroatom selected from N, O, or S, the remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like.

As defined herein, the term "heteroaralkyl" refers to an -(alkylene)-R radical where R is heteroaryl as defined above.

As defined herein, the term "heterocycyl" refers to a saturated or unsaturated monovalent monocyclic group of 4 to 8 ring atoms in which one or two ring atoms are heteroatoms selected from N, O, or S(O)ₙ, where n is an integer from 0 to 2, the remaining ring atoms being C. The heterocyclyl ring is optionally fused to a (one) aryl or heteroaryl ring as defined herein provided the aryl and heteroaryl rings are monocyclic. Additionally, one or two ring carbon atoms in the heterocyclyl ring can optionally be replaced by a -CO-group. More specifically the term heterocyclyl includes, but is not limited to, pyrrolidino, piperidino, homopiperidino, 2-oxopyrrolidinyl, 2-oxopiperidinyl, morpholino, piperazino, tetrahydropyranyl, thiomorpholino, and the like. When the heterocyclyl ring is unsaturated it can contain one or two ring double bonds, provided that the ring is not aromatic.

As defined herein, the term "heterocycloalkyl" refers to an -(alkylene)-R radical where R is heterocyclyl ring as defined above, e.g. tetraydrofuranylmethyl, piperazinylmethyl, morpholinylethyl, and the like.

As defined herein, the term "oral cavity" is understood to mean the cavity of the mouth, and includes the inner upper and lower lips, all parts of the inner cheek, the sublingual area under the tongue, the tongue itself, as well as the upper and lower gums and the hard and soft palate.

As defined herein, the term "oral mucosa" is understood to mean the mucous membrane lining the inside of the mouth, and includes (but does not exclusively refer to) mucosa in the buccal, labial, sublingual, ginigival or lip areas, the soft palate and the hard palate.

As defined herein, a wavy line ( 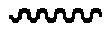 ) represents undefined stereochemistry about a particular atom.

### Films of the present invention

The present invention provides a film suitable for administration to an oral cavity comprising:
(i) an alginate salt of a monovalent cation or a mixture of alginate salts containing at least one alginate salt of a monovalent cation; and
(ii) an active pharmaceutical ingredient (API) which is one or more cannabinoids or pharmaceutically acceptable salts thereof,
wherein the alginate salt of a monovalent cation comprises from 25 to 35% by weight of β-D-mannuronate and from 65 to 75% by weight of α-L-guluronate.

The function of said alginate salt of a monovalent cation or mixture of alginate salts containing at least one alginate salt of a monovalent cation within the film is to act as a film-forming agent. As used herein, the term "film-forming agent" refers to a chemical or group of chemicals that form a pliable, cohesive and continuous covering when applied to a surface.

Alginate, the salt of alginic acid, is a linear polysaccharide naturally produced by brown seaweeds (*Phaeophyceae,* mainly *Laminaria*)*.* Typically the alginate employed in the present invention comprises from 100 to 3000 monomer residues linked together in a flexible chain. These residues are of two types, namely β-(1,4)-linked D-mannuronic acid (M) residues and α-(1,4)-linked L-guluronic acid (G) residues. Typically, at physiological pH, the carboxylic acid group of each residue in the polymer is ionised. The two residue types are epimers of one another, differing only in their stereochemistry at the C5 position, with D-mannuronic acid residues being enzymatically converted to L-guluronic acid residues after polymerization. However, in the polymer chain the two residue types give rise to very different conformations: any two adjacent D-mannuronic acid residues are ⁴C₁-diequatorially linked whilst any two adjacent L-guluronic acid residues are ⁴C₁-diaxially linked, as illustrated in Formula (A) below.

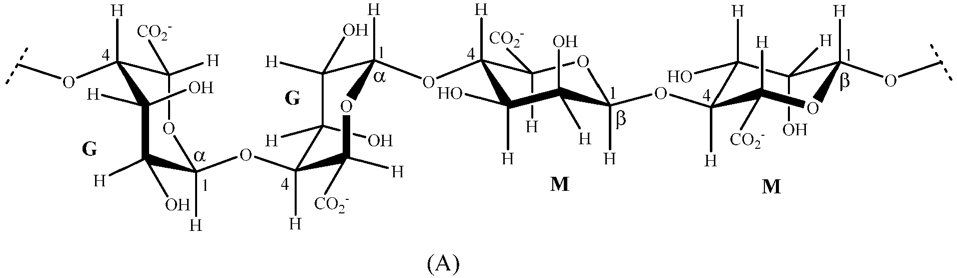

Typically in the alginate polymer, the residues are organised in blocks of identical or strictly alternating residues, e.g. MMMMM..., GGGGG... or GMGMGM.... Different monovalent and polyvalent cations may be present as counter ions to the negatively-charged carboxylate groups of the D-mannuronic acid and L-guluronic acid residues of the alginate polymer. Typically, the film comprises an alginate salt wherein the counter ions of the alginate polymer are monovalent cations. The cations which are the counterions of a single alginate polymer molecule may all be the same as one another or may be different to one another. Preferably, the counterions of the alginate polymer are selected from the group consisting of Na⁺, K⁺ and NH₄⁺. More preferably, the counterions of the alginate polymer are Na⁺. Alternatively, the film may comprise a mixture of alginate salts containing at least one alginate salt of a monovalent cation. The mixture of alginate salts may comprise an alginate salt of a cation selected from the group consisting of Na⁺, K⁺ and NH₄⁺.

Typically, the film comprises an alginate composition which has a dynamic viscosity, as measured on a 10% aqueous solution (w/w) thereof at a temperature of 20 °C with a Brookfield LVF viscometer (obtained from Brookfield Engineering Laboratories, Inc.), using a spindle No. 2 at a shear rate of 20 rpm, of 100-1000 mPa.s, or 200-800 mPa.s, or 300-700 mPa.s.

The film comprises an alginate composition having a mean guluronate (G) content of from 65 to 75% by weight and a mean mannuronate content of from 25 to 35% by weight. Thus, the film comprises an alginate composition having a mean guluronate (G) content of from 65 to 75% by weight. The film comprises an alginate composition having a mean mannuronate (M) content of from 25 to 35% by weight. Preferably, the film comprises an alginate composition having a mean molecular weight ranging from 30,000 g/mol to 90,000 g/mol, such as from 35,000 g/mol to 85,000 g/mol, or from 40,000 g/mol to 70,000 g/mol, or from 40,000 g/mol to 50,000 g/mol. Most preferably, the film comprises an alginate composition having a mean guluronate (G) content of from 65 to 75%, a mean mannuronate (M) content of from 25 to 35%, and a mean molecular weight ranging from 30,000 g/mol to 90,000 g/mol.

The alginate salt of a monovalent cation or the mixture of alginate salts containing at least one alginate salt of a monovalent cation may be the sole film-forming agent present in the film. Alternatively, the film may comprise one or more further film-forming agents in addition to the alginate salt of a monovalent cation or the mixture of alginate salts containing at least one alginate salt of a monovalent cation.

It is preferred that the film comprises Protanal^{®} LFR 5/60 or Protanal^{®} LF 10/60 (both commercially available sodium alginate products from FMC BioPolymer) as the alginate salt. Protonal^{®} LFR 5/60 is a low molecular weight and low viscosity sodium alginate extracted from the stem of *Laminaria hyperborean.* Protanal^{®} LF 10/60 is a sodium alginate having a G/M % ratio of 65-75/25-35 and a viscosity of from 20-70 mPas as measured on a 1% aqueous solution thereof at a temperature of 20 °C with a Brookfield LVF viscometer, using a spindle No. 2 at a shear rate of 20 rpm. Protanal^{®} LF 10/60 has both a higher mean molecular weight and a higher viscosity than Protanal^{®} LFR 5/60.

Without wishing to be bound by any particular theory, a film comprising a higher viscosity alginate salt is believed to have a longer residence time (i.e. dissolving time) after application to the oral cavity *via* adhesion to a mucous membrane of said cavity than a film comprising a lower viscosity alginate salt of a similar thickness. It is contemplated that the viscosity of the alginate composition within the film may be adjusted by mixing any number of alginates having different viscosities. Typically, a film of about 1 mm thickness comprising Protanal^{®} LFR 5/60 as the sole alginate component has a residence time of approximately 3-10 minutes after adhesion to a mucous membrane of the oral cavity. In contrast, a film of about 1 mm thickness comprising Protanal^{®} LF 10/60 as the sole alginate component has a residence time of approximately 30 minutes after adhesion to a mucous membrane of the oral cavity.

Therefore, if a long residence time of the film within the oral cavity is desired, it is generally preferred that the film comprises Protanal^{®} LF 10/60 as the alginate salt. However, compared to films comprising Protanal^{®} LFR 5/60 as the alginate salt, films comprising Protanal^{®} LF 10/60 as the alginate salt typically exhibit inferior adhesion properties when applied to a mucous membrane of the oral cavity. More generally, it is believed that film-forming agents having longer average chain lengths exhibit poorer adhesion to mucosa than film-forming agents having shorter average chain lengths. Without wishing to be bound by any particular theory, it is believed that better mucoadhesion of a film to the mucous membrane of the oral cavity enables a more efficient delivery of any active ingredients contained within the film to their site of action. Therefore, if a long residence time of the film within the oral cavity is not particularly necessary, it may be preferable to use Protanal^{®} LFR 5/60 as the alginate salt.

It is particularly preferred that the film comprises Protanal^{®} LFR 5/60 as the alginate salt.

The film may also comprise a film-forming agent other than the alginate salt of a monovalent cation or the mixture of alginate salts containing at least one alginate salt of a monovalent cation. Such other film-forming agents include agents such as poly(vinyl pyrrolidone) (PVP), pullulan, and so forth. However, if any other film-forming agent is present in the film in addition to the alginate salt of a monovalent cation or the mixture of alginate salts containing at least one alginate salt of a monovalent cation, then typically the alginate salt of a monovalent cation or the mixture of alginate salts containing at least one alginate salt of a monovalent cation will be present in the film in excess over any other film-forming agent present. Preferably, the ratio (by weight) of the alginate salt of a monovalent cation or the mixture of alginate salts containing at least one alginate salt of a monovalent cation present in the film to the combined total of all other film-forming agents (such as PVP and/or pullulan) present in the film is 1:1 or greater, or 2:1 or greater, or 3:1 or greater, or 4:1 or greater, or 5:1 or greater, or 10:1 or greater, or 20:1 or greater, or 50:1 or greater, or 100:1 or greater, or 500:1 or greater, or 1000:1 or greater, or 10000:1 or greater. Preferably, the alginate salt of a monovalent cation or the mixture of alginate salts containing at least one alginate salt of a monovalent cation will constitute at least 50% by weight of the total of the film-forming agents present in the film, more preferably at least 60% by weight, at least 70% by weight, at least 80% by weight, at least 90% by weight, at least 95% by weight, at least 98% by weight, at least 99% by weight, at least 99.5% by weight, at least 99.9% by weight, at least 99.95% by weight, or at least 99.99% by weight of the total of the film-forming agents present in the film.

Preferably, the alginate salt of a monovalent cation or the mixture of alginate salts containing at least one alginate salt of a monovalent cation is substantially the only film-forming agent present in the film. More preferably, the alginate salt of a monovalent cation or the mixture of alginate salts containing at least one alginate salt of a monovalent cation is the only film-forming agent present in the film. Alternatively, the film preferably does not comprise any, or substantially any, poly(vinyl pyrrolidone). Alternatively, the film preferably does not comprise any, or substantially any, pullulan.

As used herein, a reference to a film that does not comprise "substantially any" of a specified component refers to a film that may contain trace amounts of the specified component, provided that the specified component does not materially affect the essential characteristics of the film. Typically, therefore, a film that does not comprise substantially any of a specified component contains less than 5 wt% of the specified component, preferably less than 1 wt% of the specified component, most preferably less than 0.1 wt% of the specified component.

It is a finding of the present invention that the use of an alginate salt of a monovalent cation or a mixture of alginate salts containing at least one alginate salt of a monovalent cation as the film-forming agent has benefits over the use of alternative film-forming agents, such as PVP and/or pullulan. In particular, the use of alginate as the primary film-forming agent ensures that the films of the present invention have superior adhesive properties over films comprising primarily other film-forming agents such as PVP or pullulan. The films of the present invention are bioadhesive; that is to say that the films of the present invention can firmly adhere to a moist surface (i.e. mucosa) in the oral cavity of a mammal subject before it has fully dissolved. Films in which alginate is not the primary film-forming agent do not generally have this desirable property. A further advantageous finding of the present invention is that the choice of alginate as the primary film-forming agent enables therapeutically effective doses of an active pharmaceutical ingredient (e.g. Δ⁹-THC, CBD or mixtures thereof) to be loaded into the films whilst retaining homogeneity and other desirable physical properties of the films.

Typically, the film comprises from 25% to 99% by weight of the alginate salt of a monovalent cation or the mixture of alginate salts containing at least one alginate salt of a monovalent cation, preferably from 27% to 95% by weight, more preferably from 29% to 93% by weight, still more preferably from 30% to 91% by weight, and most preferably from 35% to 90% by weight.

The film according to the present invention may also contain a residual water content. Typically, the film comprises from 0% to 20% by weight of residual water. More typically, the film comprises from 5% to 15% by weight of residual water. Preferably, the film comprises from 9% to 11% by weight of residual water. Most preferably, the film comprises about 10% by weight of residual water.

The film according to the present invention also comprises an active pharmaceutical ingredient (API) which is one or more cannabinoids or pharmaceutically acceptable salts thereof. Typically the API is a mixture of from one to twenty cannabinoids or pharmaceutically acceptable salts thereof, preferably from one to ten cannabinoids or pharmaceutically acceptable salts thereof, more preferably from one to five cannabinoids or pharmaceutically acceptable salts thereof, and most preferably from one to three cannabinoids or pharmaceutically acceptable salts thereof. The API may be one cannabinoid or a pharmaceutically acceptable salt thereof. The API may be a mixture of two cannabinoids or pharmaceutically acceptable salts thereof. The API may be a mixture of three cannabinoids or pharmaceutically acceptable salts thereof.

Each cannabinoid is typically an agonist of a cannabinoid receptor, an inverse agonist of a cannabinoid receptor, or an antagonist of a cannabinoid receptor. Thus, typically, each cannabinoid is an agonist of a cannabinoid receptor. Alternatively, each cannabinoid is an inverse agonist of a cannabinoid receptor. Alternatively, each cannabinoid is an antagonist of a cannabinoid receptor. Each cannabinoid may act on one or more than one of the classes of cannabinoid receptor, e.g. cannabinoid receptor 1 (CB₁) and/or cannabinoid receptor 2 (CB₂). Thus, typically, each cannabinoid may be an agonist, inverse agonist or antagonist of CB₁. Alternatively, each cannabinoid may be an agonist, inverse agonist or antagonist of CB₂. Alternatively, each cannabinoid may be an agonist, inverse agonist or antagonist of both CB₁ and CB₂. Each cannabinoid may display selective activity for CB₁ over CB₂. Alternatively, each cannabinoid may display selective activity for CB₂ over CB₁. Alternatively, each cannabinoid may display little to no selectively for either CB₁ or CB₂ activity. A skilled person can easily identify and use suitable assays for determining whether a given compound has activity against CB₁ and/or CB₂ receptors, and would readily be able to classify any activity as agonistic, inverse agonistic, or antagonistic activity. For example, suitable assays that the skilled person can employ for this purpose include those reviewed by RG Pertwee. [11]

Each cannabinoid is typically a phytocannabinoid, an endocannabinoid or a synthetic cannabinoid. Thus, typically, each cannabinoid is a phytocannabinoid. Alternatively, each cannabinoid is an endocannabinoid. Alternatively, each cannabinoid is a synthetic cannabinoid.

Typically, one or more cannabinoids present in the film are selected from the group consisting of a compound of Formulae (I) to (XXI) 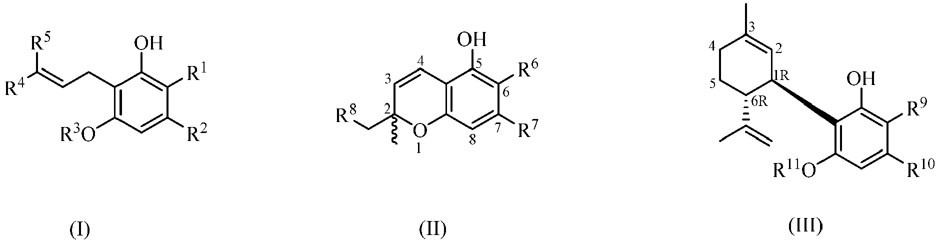 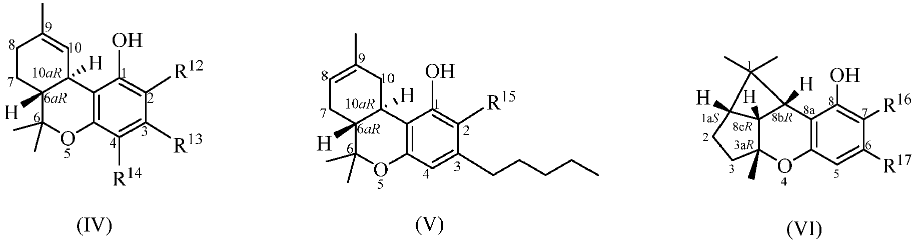 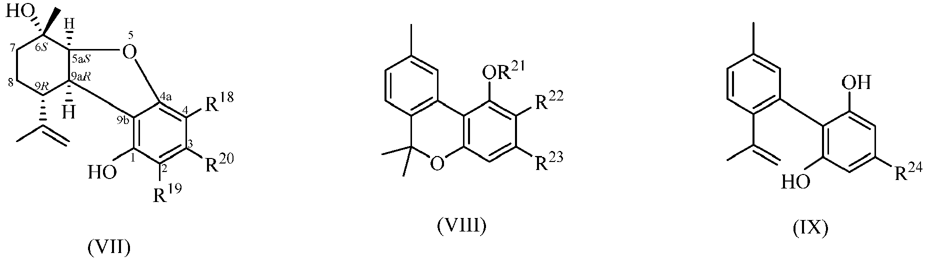 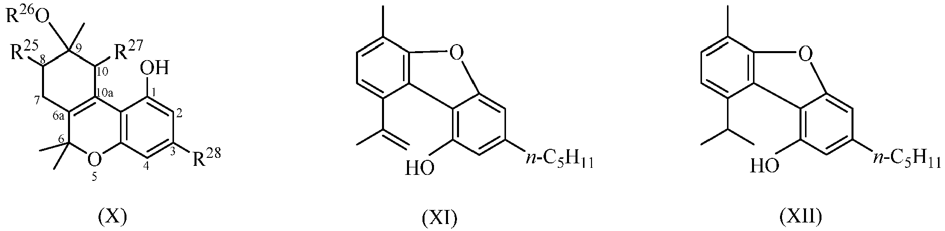 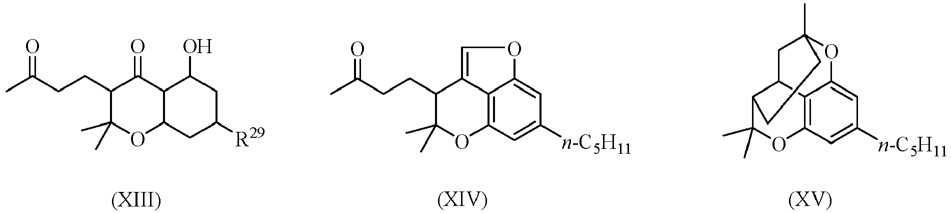 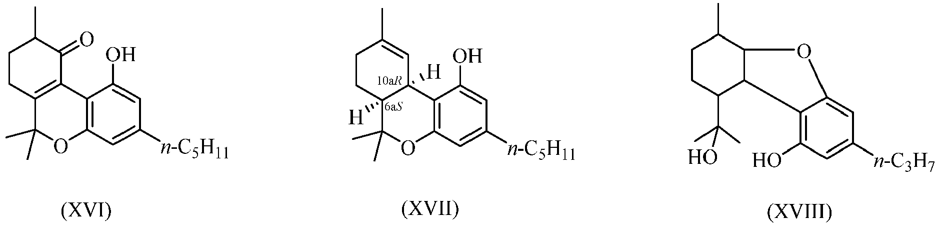 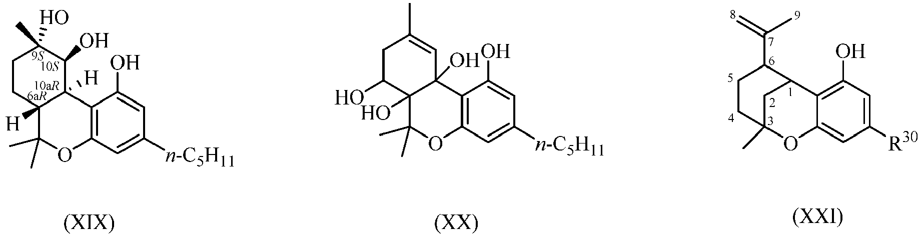 wherein:
R¹, R⁶, R⁹, R¹², R¹⁴, R¹⁵, R¹⁶, R¹⁸, R¹⁹ and R²² are each independently hydrogen or COOH;
R², R³, R⁷, R¹⁰, R¹¹, R¹³, R¹⁷, R²⁰, R²¹, R²³, R²⁴, R²⁸, R²⁹ and R³⁰ are each independently hydrogen or C₁₋₆ alkyl;
R⁴ and R⁵ are each independently C₁₋₆ alkyl or C₂₋₆ alkenyl;
R⁸ is C₂₋₆ alkenyl;
R²⁵ is hydrogen or OH;
R²⁶ is hydrogen or cannabidiolic acid-Cs ester; and
R²⁷ is hydrogen or OR³¹, wherein R³¹ is hydrogen or C₁₋₆ alkyl.

Formula (I) represents so-called "CBG" type cannabinoids. Formula (II) represents so-called "CBC" type cannabinoids. Formula (III) represents so-called "CBD" type cannabinoids. Formula (IV) represents so-called "Δ⁹-THC" type cannabinoids. Formula (V) represents so-called "Δ⁸-THC" type cannabinoids. Formula (VI) represents so-called "CBL" type cannabinoids. Formula (VII) represents so-called "CBE" type cannabinoids. Formula (VIII) represents so-called "CBN" type cannabinoids. Formula (IX) represents so-called "CBND" type cannabinoids. Formula (X) represents so-called "CBT" type cannabinoids. Formulae (XI) to (XXI) represent so-called "misc" type cannabinoids.

Preferably, R² is C₁₋₆ alkyl, more preferably C₃₋₅ n-alkyl. Preferably, R³ is hydrogen or methyl. Preferably, R⁴ is methyl or C₆ alkenyl, more preferably methyl or (CH₂)₂CH=C(CH₃)₂. Preferably, R⁵ is methyl or C₆ alkenyl, more preferably methyl or (CH₂)₂CH=C(CH₃)₂. More preferably, one of R⁴ and R⁵ is methyl and the other is (CH₂)₂CH=C(CH₃)₂. Preferably, R⁷ is C₁₋₆ alkyl, more preferably C₃₋₅ alkyl. Preferably, R⁸ is C₆ alkenyl, more preferably (CH₂)₂CH=C(CH₃)₂ or CH₂CH=CHCH(CH₃)₂. Preferably, R¹⁰ is C₁₋₆ alkyl, more preferably C₁₋₅ n-alkyl. Preferably, R¹¹ is hydrogen or methyl. Preferably, R¹³ is C₁₋₆ alkyl, more preferably C₁₋₅ n-alkyl. Preferably, R¹⁷ is C₁₋₆ alkyl, more preferably C₃₋₅ n-alkyl. Preferably, no more than one of R¹⁸ and R¹⁹ is COOH. Preferably, R²⁰ is C₁₋₆ alkyl, more preferably C₃₋₅ n-alkyl. Preferably, R²¹ is hydrogen or methyl. Preferably, R²³ is C₁₋₆ alkyl, more preferably C₁₋₅ n-alkyl. Preferably, R²⁴ is C₁₋₆ alkyl, more preferably C₃₋₅ *n-*alkyl. Preferably, at least one of R²⁵ and R²⁶ is hydrogen. Preferably, R²⁸ is C₁₋₆ alkyl, more preferably C₃₋₅ alkyl. Simultaneously, R²⁵ and R²⁶ may be hydrogen, R²⁷ may be -OH and R²⁸ may be -C₃H₇. Preferably, R²⁹ is C₁₋₆ alkyl, more preferably C₃₋₅ n-alkyl. Preferably, R³⁰ is C₁₋₆ alkyl, more preferably C₃₋₅ n-alkyl. Preferably, R³¹ is hydrogen, methyl or ethyl.

Preferably, the compound of Formulae (I) to (XXI) is selected from the group consisting of cannabigerolic acid A, cannabigerolic acid A monomethyl ether, cannabigerol, cannabigerol monomethyl ether, cannabigerovarinic acid A, cannabigerovarin, cannabinerolic acid A, (±)-cannabichromenic acid, (±)-cannabichromene, (±)-cannabichromevarinic acid, (±)-cannabivarichromene, (+)-cannabichromevarin, 2-methyl-2-(4-methyl-2-pentenyl)-7-propyl-2*H*-1-benzopyran-5-ol, cannabidiolic acid, (-)-cannabidiol (CBD), cannabidiol monomethyl ether, cannabidiol-C₄, cannabidivarinic acid, (-)-cannabidivarin, cannabidiorcol, tetrahydrocannabinolic acid A, tetrahydrocannabinolic acid B, tetrahydrocannabinol (Δ⁹-THC), tetrahydrocannabinolic acid-C₄, tetrahydrocannabinol-C₄, tetrahydrocannabivarinic acid A, tetrahydrocannabivarin, tetrahydrocannabiorcolic acid, tetrahydrocannabiorcol, (-)-Δ⁸*-trans-*(6a*R,*10aR)-tetrahydrocannabinolic acid A, (-)-Δ⁸*-trans-*(6a*R,*10a*R*)-tetrahydrocannabinol, (±)-(1a*S*,3a*R*,8b*R*,8c*R*)-cannabicyclolic acid, (±)-(1a*S*,3a*R*,8b*R*,8c*R*)-cannabicyclol, (±)-(1a*S*,3a*R*,8b*R*,8c*R*)-cannabicyclovarin, (5a*S*,6*S*,9*R*,9a*R*)-cannabielsoic acid A, (5a*S*,6*S*,9*R*,9a*R*)-cannabielsoic acid B, (5a*S*,6*S*,9*R*,9a*R*)-C₃-cannabielsoic acid B, (5a*S*,6*S*,9*R*,9a*R*)-cannabielsoin, (5a*S*,6*S*,9*R*,9a*R*)-C₃-cannabielsoin, cannabinolic acid A, cannabinol, cannabinol methyl ether, cannabinol-C₄, cannabivarin, cannabinol-C₂, cannabiorcol-C₁, cannabinodiol, cannabinodivarin, (-)-*trans*-cannabitriol, (+)*-trans-*cannabitriol, (±)-*cis*-cannabitriol, (±)-*trans*-cannabitriol-C₃, (-)-*trans*-10-ethoxy-9-hydroxy-Δ^{6a(10a)}-tetrahydrocannabinol, *trans*-10-ethoxy-9-hydroxy-Δ^{6a(10a)}-tetrahydrocannabivarin-C₃, 8,9-dihydroxy-Δ^{6a(10a)}-tetrahydrocannabinol, cannabidiolic acid tetrahydrocannabitriol ester, dehydrocannabifuran, cannabifuran, cannabichromanone, cannabichromanone-C₃, cannabicoumarinone-Cs, cannabicitran, 10-oxo- Δ^{6a(10a)}-tetrahydrocannabinol, (-)- Δ⁹-(6a*S*,10a*R*-*cis*)-tetrahydrocannabinol, cannabiglendol-C₃, (-)-(6a*R*,9*S*,10*S*,10a*R*)-9,10-dihydroxyhexahydrocannabinol, (-)-6a,7,10a-Trihydroxy-Δ⁹-tetrahydrocannabinol, (±)-Δ⁷-*cis*-(1*R*,3*R*,6*S*)-isotetrahydrocannabivarin-C3, (-)-Δ⁷*-trans-*(1*R,*3*R,*6*R*)-isotetrahydrocannabivarin-C₃, and (-)-Δ⁷-*trans*-(1*R*,3*R*,6*R*)-isotetrahydrocannabinol-C₅.

Most preferably, the compound of Formulae (I) to (XXI) is selected from the group consisting of Δ⁹-THC and CBD. Thus, preferably, the API is Δ⁹-THC. Alternatively, preferably the API is CBD. Alternatively, preferably the API is a mixture of Δ⁹-THC and CBD. The ratio of Δ⁹-THC:CBD in said mixture is typically from 10,000:1 to 1:10,000, preferably from 1,000:1 to 1:1,000, more preferably from 100:1 to 1:100, still more preferably from 10:1 to 1:10, yet more preferably from 5:1 to 1:5, even more preferably from 3:1 to 1:3, more preferably still from 2:1 to 1:2, and most preferably about 1:1. The structures of Δ⁹-THC and CBD are set out below as Formulae (XXII) and (XXIII) respectively.

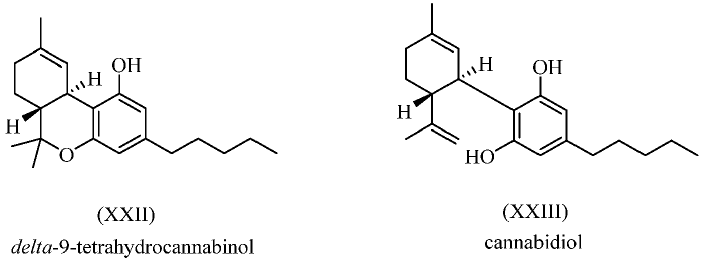

For the avoidance of doubt, the terms "tetrahydrocannabinol", "Δ⁹-tetrahydrocannabinol", "(-)-*trans*-Δ⁹-tetrahydrocannabinol", "(6a*R*, 10a*R*)- Δ⁹-tetrahydrocannabinol", "Marinol" and "dronabinol" all relate to the same chemical compound (having Formula (XXII)) and are interchangeable with one another, as used herein.

One or more cannabinoids present in the film may be endocannabinoids. In this case, preferably each endocannabinoid is selected from the group consisting of anandamide, 2-arachidonoylglycerol, 2-arachidonyl glyceryl ether, *N*-arachidonoyl dopamine, virodhamine, and lysophosphatidylinositol. The structures of anandamide, 2-arachidonoylglycerol, 2-arachidonyl glyceryl ether, *N*-arachidonoyl dopamine, virodhamine, and lysophosphatidylinositol are set out below as Formulae (XXIV) to (XXIX) respectively.

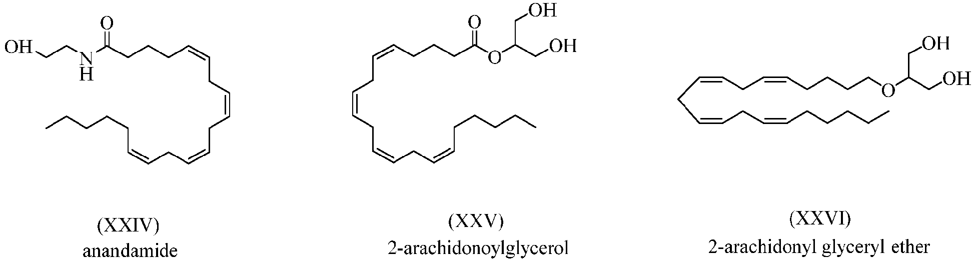

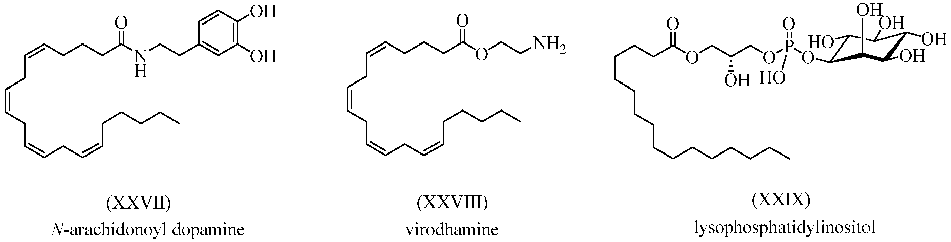

One or more cannabinoids present in the film may be selected from the group consisting of aminoalkylindoles, 1,5-diarylpyrazoles, quinolones, arylsulfonamides, icosanoids. In this case, more preferably the one or more cannabinoids are selected from the group consisting of nabilone, rimonabant, JWH-018, JWH-073, CP-55940, dimethylheptylpyran, HU-210, HU-331, SR144528, WIN 55,212-2, JWH-133, levonantradol, AM-2201, and ajulemic acid. As used herein, the term "nabilone" may refer to (*R,R*)-(-)-nabilone, (*S,S*)-(+)-nabilone, or a mixture thereof, including a racemic mixture thereof. The structures of (*R,R*)-(-)-nabilone, (*S,S*)-(+)-nabilone, rimonabant, JWH-018, JWH-073, CP-55940, dimethylheptylpyran, HU-210, HU-331, SR144528, WIN 55,212-2, JWH-133, levonantradol, AM-2201 and ajulemic acid are set out below as Formulae (XXX) to (XLIV) respectively.

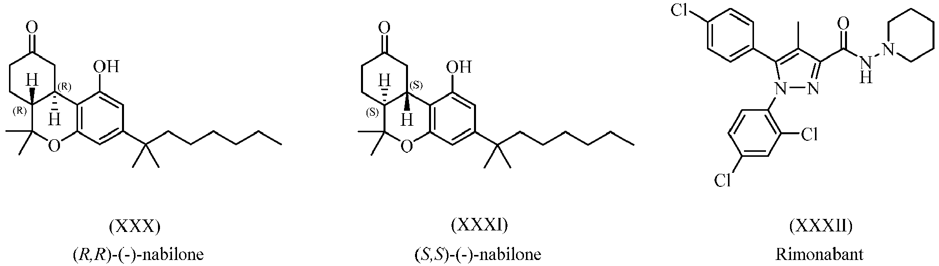

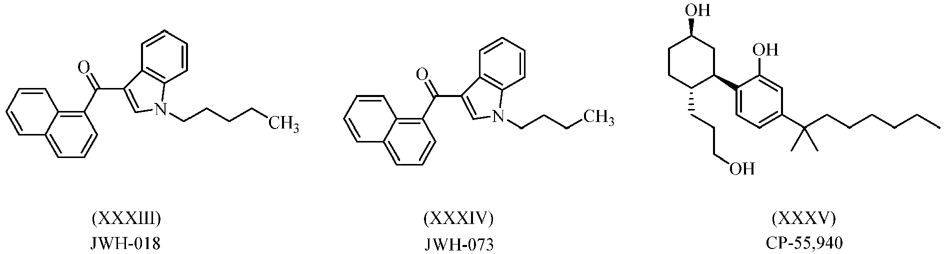

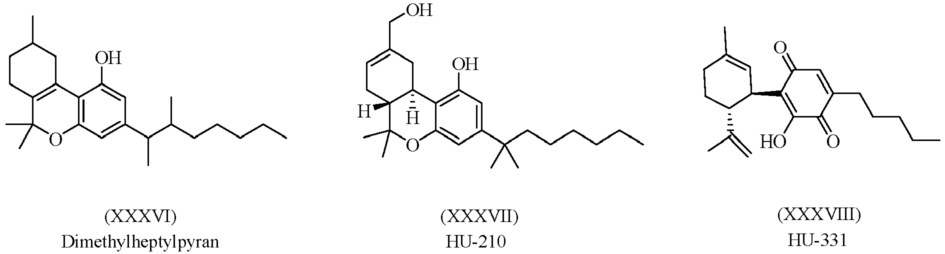

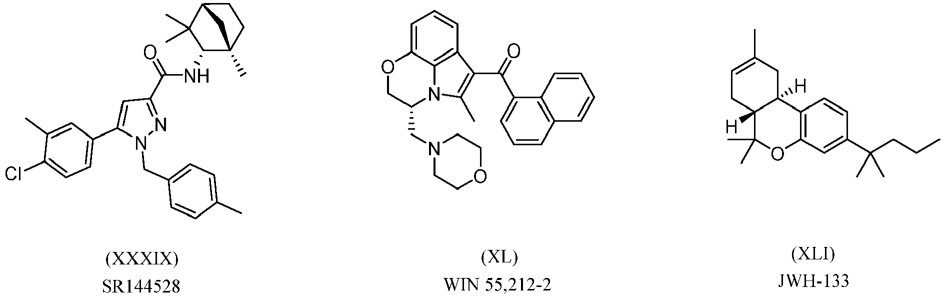

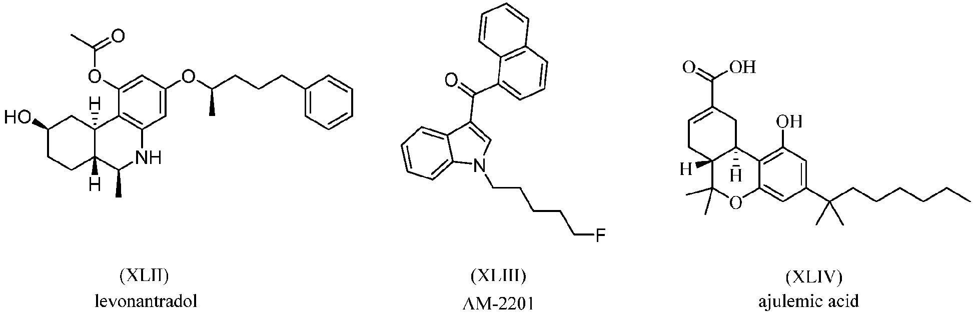

The API may be a pharmaceutically acceptable polymorph, co-crystal, hydrate or solvate of one or more cannabinoids or pharmaceutically acceptable salts, preferably a pharmaceutically acceptable polymorph, co-crystal, hydrate or solvate of Δ⁹-THC, CBD or a mixture of Δ⁹-THC and CBD, or pharmaceutically acceptable salts thereof.

Alternatively, the API may be a prodrug of one or more cannabinoids or pharmaceutically acceptable salts thereof, preferably a prodrug of Δ⁹-THC, CBD or a mixture of Δ⁹-THC and CBD, or pharmaceutically acceptable salts thereof. The term "prodrug" of a cannabinoid, as used herein, refers to any compound or pharmaceutically acceptable salt thereof which, after administration to the human body, may be metabolised *in vivo* to a cannabinoid. Typical prodrugs include acyl, ester, alkoxycarbonyl and carbamyl derivatives of a cannabinoid. For example, prodrugs of CBD include acyl and alkoxycarbonyl derivatives such as those described in US 8,293,786, the contents of which are incorporated herein in their entirety. For example, prodrugs of Δ⁹-THC include acyl and alkoxycarbonyl derivatives such as those described in US 8,227,627, the contents of which are incorporated herein in their entirety.

Thus, one or more cannabinoids present in the film may be selected from the group consisting of a compound of Formula (XLV) and a compound of Formula (XLVI) 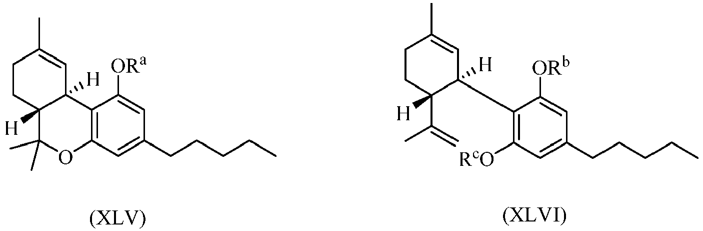 wherein:
R^{a} is a bio-labile linker, such as acyl, alkoxycarbonyl, carbamate, phosphate, diphosphate, or triphosphate; and
R^{b} and R^{c} are independently hydrogen or a bio-labile linker, such as acyl, alkoxycarbonyl, carbamate, phosphate, diphosphate, or triphosphate, provided that R^{b} and R^{c} are not both hydrogen.

The compounds of Formulae (I) to (XLVI) may contain one or more stereogenic centres. For example, in Formula (II), carbon 2 is a stereogenic centre. Thus, compounds of Formula (II) may be either the (*R*)-enantiomer, the (S)-enantiomer, or a mixture thereof, including a racemic mixture thereof. Certain compounds of Formulae (I) to (XLVI) may therefore be isolated in optically active or racemic forms. It is well-known in the art how to prepare optically active forms, such as by resolution of materials. For the avoidance of doubt, Formulae (I) to (XLVI) encompasses all enantiomeric, diastereomeric, and racemic forms of the compounds thereof, as well as all mixtures of enantiomers and diastereomers of the compounds thereof, unless stated otherwise (e.g. by the explicit use of dashed ( 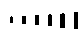 ) and/or wedged ( 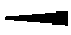 ) bonds to indicate a particular configuration about a given stereogenic centre).

Typically, the API is a one or more pharmaceutically acceptable salts of one or more cannabinoids. Typically, the pharmaceutically acceptable salt of each cannabinoid is selected from the group consisting of acetate, propionate, isobutyrate, benzoate, succinate, suberate, tartrate, citrate, fumarate, malonate, maleate, adipate, di-mesylate, sulfate, benzenesulfonate, nitrate, carbonate, hydrochloride, hydrobromide, phosphate, aluminium, ammonium, calcium, copper, ferric, ferrous, lithium, magnesium, manganic, manganous, potassium, sodium, zinc, arginine, betaine, caffeine, choline, *N,N'-* dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine and tromethamine salts of the cannabinoid. Preferred salt forms include aluminium, ammonium, calcium, copper, ferric, ferrous, lithium, magnesium, manganic, manganous, potassium, sodium, zinc, arginine, betaine, caffeine, choline, *N,N'-* dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine and tromethamine salts of the cannabinoid.

The API may be an extract obtained from a cannabis plant. Thus, the API may be an extract obtained from *Cannabis sativa.* Alternatively, the API may be an extract obtained from *Cannabis indica.* Alternatively, the API may be an extract obtained from *Cannabis ruderalis.*

The API may be present within the film in varying amounts. Typically, the film comprises from 0.001% to 75% by weight of the API, preferably from 0.01% to 60% by weight of the API, more preferably from 0.15% to 50% by weight of the API, still more preferably from 0.2% to 45% by weight of the API and most preferably from 0.25% to 40% by weight of the API. In one embodiment, the film comprises from 0.25% to 20% by weight of the API, preferably from 0.5% to 15% by weight of the API, and more preferably from 1% to 10% by weight of the API.

Typically, the one or more cannabinoids or pharmaceutically acceptable salts thereof constitutes the only API present in the film. However, the film may alternatively comprise one or more further active pharmaceutical ingredients in addition to cannabinoids or pharmaceutically acceptable salts thereof.

Preferably, the film comprises from 25% to 99% by weight of the alginate salt of a monovalent cation or the mixture of alginate salts containing at least one alginate salt of a monovalent cation, from 0% to 20% by weight of water, and from 0.001% to 75% by weight of the API. More preferably, the film comprises from 29% to 93% by weight of the alginate salt of a monovalent cation or the mixture of alginate salts containing at least one alginate salt of a monovalent cation, from 5% to 15% by weight of water, and from 0.15% to 50% by weight of the API. Even more preferably, the film comprises from 30% to 91% by weight of the alginate salt of a monovalent cation or the mixture of alginate salts containing at least one alginate salt of a monovalent cation, from 9% to 11% by weight of water, and from 0.2% to 45% by weight of the API. Still more preferable, the comprises from 30% to 91% by weight of the alginate salt of a monovalent cation or the mixture of alginate salts containing at least one alginate salt of a monovalent cation, from 9% to 11% by weight of water, and from 0.5% to 15% by weight of the API.

A film according to the present invention may optionally further comprise other components in addition to the API, water and the film-forming agent. Typically, a film according to the present invention further comprises one or more of the following:
(i) at least one pharmaceutically acceptable solvent;
(ii) at least one buffering component;
(iii) at least one excipient, such as one or more plasticizers or fillers;
(iv) at least one taste-masking agent or flavouring agent;
(v) at least one acidifying agent or basifying agent;
(vi) at least one permeation enhancer;
(vii) a self-emulsifying drug delivery system (SEDDS), such as a self-microemulsifying drug delivery system (SMEDDS) or a self-nanoemulsifying drug delivery system (SNEDDS);
(viii) at least one chelating agent;
(ix) at least one antioxidant;
(x) at least one antimicrobial agent; and
(xi) at least one inorganic salt.

The film may additionally comprise one or more pharmaceutically acceptable solvents. Such a solvent may be a non-aqueous solvent, or a combination of water and a non-aqueous solvent. Examples of non-aqueous solvents should be non-toxic and include, but are not limited to, ethanol, acetone, benzyl alcohol, diethylene glycol monoethyl ether, glycerine, hexylene glycol, isopropyl alcohol, polyethylene glycols, methoxypolyethylene glycols, diethyl sebacate, dimethyl isosorbide, propylene carbonate, dimethyl sulfoxide (DMSO), transcutol, triacetin, fatty acid esters, and oils such as soybean oil, peanut oil, olive oil, palm oil, rapeseed oil, corn oil, other vegetable oils and the like. Preferably, the solvent is a non-aqueous solvent. More preferably, the solvent is triacetin. The film may comprise from 0% to 15% of each non-aqueous solvent present, preferably from 0.001% to 10% by weight of each non-aqueous solvent, more preferably from 0.01% to 1% by weight of each non-aqueous solvent.

The film may additionally comprise any suitable buffering component. A "buffering component", as defined herein, refers to any chemical entity, which when dissolved in solution, enables said solution to resist changes in its pH following the subsequent addition of either an acid or a base. A suitable buffering component for use in the film of the present invention would be a buffering component which is an effective buffer within a pH range of from 3.0 to 13.5. Preferably, said buffering component is an effective buffer within a pH range of from 8.0 to 13.5, more preferably of from 10.0 to 13.0, and most preferably of from 12.5 to 13.0. Examples of suitable buffering components include, but are not limited to: phosphates, sulfates, citrates and acetates. The buffer may be a salt of a monovalent cation, such as sodium, potassium or ammonium salts. Particularly preferred buffering components include citric acid and sodium dihydrogen phosphate. Without wishing to be bound by any particular theory, it is believed that alginate tends to gel at a pH of less than 3.8. Thus, it is desirable that the pH of the film is greater than 3.8.

The film may comprise from 0.1% to 10% by weight of the buffering component, typically 0.2% to 8% by weight, typically from 0.3% to 6% by weight, typically from 0.5% to 5% by weight. Alternatively, the film may not additionally comprise a buffering component.

The film may additionally comprise any suitable excipient, such as one or more fillers or plasticizers. The film may comprise both a plasticizer and a filler. Alternatively, the film may comprise just one of a plasticizer or a filler. It is preferred that the film comprises a plasticizer. Under some circumstances it may be desirable that the film does not comprise a filler. It is particularly preferred that the film comprises a plasticizer but does not comprise a filler.

The plasticizer, when present, may be selected from the group consisting of polyethylene glycol, glycerol, sorbitol, xylitol, and a combination thereof. Typically, the film comprises a plasticizer which is selected from the group consisting of glycerol, sorbitol, xylitol, and a combination thereof. Preferably, the film comprises a plasticizer which is selected from the group consisting of glycerol, sorbitol, and a combination thereof. More preferably, the film comprises both glycerol and sorbitol as plasticizers. Yet more preferably, the film comprises all of glycerol, sorbitol and xylitol as plasticizers. The film may comprise from 0% to 40% by weight of each plasticizer present, preferably from 1% to 35% by weight of each plasticizer, more preferably from 2% to 30% by weight of each plasticizer, and most preferably from 3% to 25% by weight of each plasticizer.

The filler, when present, may be e.g. microcrystalline cellulose or titanium dioxide. A suitable amount of filler may be from 0% to 20% by weight, e.g. from 0.1% to 10% by weight, of the total pharmaceutical composition.

The film may additionally comprise a taste-masking agent or a flavouring agent. The taste-masking agent may be a sweetener. The flavouring agent, when present, may for example be selected from the group consisting of acacia, anise oil, caraway oil, cardamom, cherry syrup, cinnamon, citric acid syrup, clove oil, cocoa, coriander oil, ethyl vanillin, fennel oil, ginger, glycerine, glycyrrhiza, honey, lavender oil, lemon oil, mannitol, nutmeg oil, orange oil, orange flower water, peppermint oil, raspberry, rose oil, rosewater, rosemary oil, sarsaparilla syrup, spearmint oil, thyme oil, tolu balsam syrup, vanilla, wild cherry syrup, and mixtures thereof. The film may comprise from 0.001% to 10% by weight of each flavouring agent present, preferably from 0.01% to 5% by weight of each flavouring agent, and most preferably from 0.1% to 3% by weight of each flavouring agent.

The film may additionally comprise an acidifying agent or a basifying agent. An "acidifying agent", as defined herein, refers to a chemical compound that alone or in combination with other compounds can be used to acidify a pharmaceutical composition. A "basifying agent", as defined herein, refers to a chemical compound that alone or in combination with other compounds can be used to basify a pharmaceutical composition.

Typically, the film comprises an basifying agent. Typically, the basifying agent is an alkali. Examples of suitable basifying agents include, but are not limited to: sodium hydroxide, lithium hydroxide, potassium hydroxide, magnesium hydroxide, and calcium hydroxide. A preferable basifying agent is sodium hydroxide. Alternatively, the film may comprise an acidifying agent. Examples of suitable acidifying agents include, but are not limited to: acetic acid, dehydro acetic acid, ascorbic acid, benzoic acid, boric acid, citric acid, edetic acid, hydrochloric acid, isostearic acid, lactic acid, nitric acid, oleic acid, phosphoric acid, sorbic acid, stearic acid, sulfuric acid, tartaric acid, and undecylenic acid. A preferable acidifying agent is phosphoric acid.

A film according to the present invention is produced *via* the drying of a film-forming solution (*vide infra*)*.* Typically, a sufficient amount of acidifying agent or basifying agent is added to adjust the pH of the film-forming solution (before this is dried to form the film) to a pH of from 3.0 to 13.5, preferably to a pH of from 8.0 to 13.5, more preferably to a pH of from 10.0 to 13.0, most preferably to a pH of from 12.5 to 13.0.

The film may additionally comprise any suitable permeation enhancer. A "permeation enhancer", as defined herein, refers to a chemical compound that alone or in combination with other compounds can be used to aid the uptake of a further substance across an epithelium or other biological membrane. In particular, the term "permeation enhancer" is used herein to refer to a chemical compound that alone or in combination with other compounds can be used to aid the uptake of a further substance across the buccal mucosa. Permeation enhancers can typically be divided into two different categories, paracellular (para) or transcellular (trans) permeability enhancers, according to their mechanism of action. Paracellular permeation enhancers are those which aid the uptake of a further substance through the intercellular space between the cells in an epithelium or other biological membrane. Transcellular permeation enhancers are those which aid the uptake of a further substance through the cells in an epithelium or other biological membrane, wherein the further substance passes through both the apical and basolateral cell membranes in the epithelium or other biological membrane.

Typically, the film may comprise one or more paracellular permeation enhancers. Alternatively, the film may comprise one or more transcellular permeation enhancers. Alternatively, the film may comprise at least one paracellular permeation enhancer and at least one transcellular permeation enhancer.

Typically, the permeation enhancer, if present, is one or more compounds selected from the group consisting of: non-ionic, cationic, anionic or zwitterionic surfactants (e.g. caprylocaproyl polyoxyl-8 glyceride, sodium lauryl sulfate, cetyltrimetyl ammonium bromide, decyldimethyl ammonio propane sulfonate); bile salts (e.g. sodium deoxycholate); fatty acids (e.g. hexanoic acid, hetptanoic acid, oleic acid); fatty amines; fatty ureas; fatty acid esters (e.g. methyl laurate, methyl palmitate); substituted or unsubsituted nitrogen-containing heterocyclic compounds (e.g. methyl pyrrolidone, methyl piperazine, azone); terpenes (e.g. limonene, fenchone, menthone, cineole); sulfoxides (e.g. dimethylsulfoxide, DMSO); ethylenediaminetetraacetic acid (EDTA); and combinations thereof. Preferably, the permeation enhancer, if present, is selected from the group consisting of EDTA, oleic acid, and combinations thereof.

Typically, the film may comprise EDTA. Without wishing to be bound by any particular theory, EDTA is believed to act as a paracellular permeation enhancer by transiently affecting tight junctions interconnecting membrane cells, and subsequently increasing paracellular or pore transport. EDTA is also believed to act as a transcellular permeation enhancer by interaction with phospholipid headgroups and increasing membrane fluidity. [12] Alternatively, the film may comprise oleic acid. Without wishing to be bound by any particular theory, oleic acid is believed to act as a transcellular permeation enhancer by interacting with the polar head groups of phospholipids in or on cell membranes, and increasing cell membrane flexibility, thereby promoting transcellular drug permeability. Oleic acid has been shown to demonstrate enhanced permeability with porcine buccal epithelium at a concentration of 1-10%. [13]

The film may additionally comprise a self-emulsifying drug delivery system (SEDDS) or a resulting emulsion thereof. Such a system may preferably be a self-microemulsifying drug delivery system (SMEDDS) or a self-nanoemulsifying drug delivery system (SNEDDS) or resulting emulsion thereof. Self-microemulsifying drug delivery systems are microemulsion preconcentrates or anhydrous forms of microemulsion. Self-nanoemulsifying drug delivery systems are nanoemulsion preconcentrates or anhydrous forms of nanoemulsion. These systems are typically anhydrous isotropic mixtures of oil (e.g. tri-, di- or mono- glycerides or mixtures thereof) and at least one surfactant (e.g. Span, Tween), which, when introduced into aqueous phase under conditions of gentle agitation, spontaneously form an oil-in-water (O/W) microemulsion or nanoemulsion (respectively). SNEDDS systems typically for an emulsion with a globule size less than 200 nm. [14] SEDDS (e.g. SMEDDS or SNEDDS) may also contain coemulsifier or cosurfactant and/or solubilizer in order to facilitate emulsification (e.g. microemulsification or nanoemulsification) or improve the drug incorporation into the SEDDS (e.g. SMEDDS or SNEDDS).

Preferably, the oil phase is selected from olive oil, soyabean oil, Capryol PGMC, Maisine CC, Labrafil M2125, Captex 355 and triacetin. More preferably, the oil phase is Capryol PGMC. Preferably, the at least one surfactant is selected from Cremophor EL, Tween 80 and Labrasol. More preferably, the SEDDS comprises at least two surfactants, yet more preferably wherein said surfactants are selected from Cremophor EL, Tween 80 and Labrasol. Most preferably, the SEDDS comprises both Cremophor EL and Labrasol as surfactants. Preferably, the SEDDS further comprises a solubilizer (cosolvent). Typical solubilizers include transcutol, polyethylene glycol (PEG), DMSO and ethanol. A particularly preferred solubilizer is transcutol.

Typically, the SEDDS (e.g. SMEDDS or SNEDDS) components is selected from the group consisting of: a mixture of Tween with one or more glycerides and a hydrophilic cosolvent; a mixture of Tween with a low HLB (hydrophile-lipophile balance) cosurfactant and a hydrophilic cosolvent; a mixture of a polyethyleneglycol (PEG), Labrasol and Chremophore EL; a mixture of polyethyleneglycol (PEG), Labrasol and Kolliphore EL; a mixture of polyethyleneglycol (PEG), Labrasol, Chremophore EL and Chremophore RH40; a mixture of Capryol PGMC, Cremophor EL and transcutol; a mixture of Capryol PGMC, Cremophor EL and Labrasol; and a mixture of Capryol PGMC, Cremophor EL, Labrasol and transcutol. The PEG may be any suitable polyethyleneglycol such as PEG with an average molecular weight of from 100 to >1000 Da, preferably from 200 to 800 Da, more preferably from 300 to 600 Da, and most preferably about 400. More preferably, the SEDDS components is selected from the group consisting of: a mixture of Capryol PGMC, Cremophor EL and transcutol; a mixture of Capryol PGMC, Cremophor EL and Labrasol; and a mixture of Capryol PGMC, Cremophor EL, Labrasol and transcutol.

The term "glyceride", as defined herein, refers to any ester formed between glycerol and one or more fatty acids. The term "glyceride" may be used interchangeably with the term "acylglycerol". Typically, the glyceride is a monoglyceride, a diglyceride or a triglyceride. Preferably, the glyceride is a triglyceride. Typically, the glyceride is a simple glyceride. The term "simple glyceride" refers to a diglyceride in which the two fatty acids are the same as one another, or a triglyceride in which the three fatty acids are the same as one another. Alternatively, the glyceride is a mixed glyceride. The term "mixed glyceride" refers to a diglyceride in which the two fatty acids are different one another, or a triglyceride in which either one of the three fatty acids is different to the other two, or all three of the fatty acids are different to one another. Therefore, the glyceride is typically a monoglyceride, a simple diglyceride, a simple triglyceride, a mixed diglyceride, or a mixed triglyceride. Preferably, the glyceride is a simple triglyceride or a mixed triglyceride.

A "hydrophilic cosolvent", as defined herein, is any solvent that is miscible with water. Examples of suitable hydrophilic cosolvents include, but are not limited to: glycerol, ethanol, 2-(2-ethoxyethoxyethanol), PEG-400 and propylene glycol.

The term "low HLB cosurfactant", as defined herein, refers to any lipid falling within class IIIA, IIIB or IV of the lipid formulation classification system described by C.W. Pouton [15].

Typically, the film may additionally comprise any suitable chelating agent. A chelating agent may be added to the film to act as a preservative. A "chelating agent", as defined herein, refers to a chemical compound that is a multidentate ligand that is capable of forming two or more separate bonds to a single central atom, typically a metal ion. Examples of suitable chelating agents include, but are not limited to: ethylenediaminetetraacetic acid (EDTA), ethylene glycol-bis(β-aminoethyl ether)-*N,N,N',N'*-tetraacetic acid (EGTA), 1,2-bis(*ortho*-aminophenoxy)ethane-*N,N,N',N'*-tetraacetic acid (BAPTA), citric acid, phosphonic acid, glutamic acid, histidine, malate, and derivatives thereof. Preferably, the chelating agent, if present, is ethylenediaminetetraacetic acid (EDTA). Preferably, the film may comprise from 0.001% to 4% by weight of each chelating agent present, more preferably from 0.001% to 0.1% by weight of each chelating agent present.

The film may additionally comprise any suitable antioxidant. An "antioxidant", as defined herein, is any compound that inhibits the oxidation of other chemical species. Examples of suitable antioxidants include, but are not limited to: ascorbic acid; citric acid; sodium bisulfite; sodium metabisulfite; ethylenediaminetetraacetic acid (EDTA); butyl hydroxitoluene; and combinations thereof. Preferably, the antioxidant, if present, is ascorbic acid, sodium bisulfite, or a combination thereof. More preferably, the antioxidant, if present, is ascorbic acid. Most preferably, both ascorbic acid and sodium bisulfite are present as antioxidants. Preferably, the film may comprise from 0.001% to 4% by weight of each antioxidant present, more preferably from 0.001% to 0.1% by weight of each antioxidant present.

Typically, the film may additionally comprise any suitable antimicrobial agent. An "antimicrobial agent", as defined herein, is any compound that kills microorganisms or prevents their growth. Examples of suitable antimicrobial agents include, but are not limited to: benzyl alcohol; benzalkonium chloride; benzoic acid; methyl-, ethyl- or propyl- paraben; and quarternary ammonium compounds. Preferably, the film may comprise from 0.001% to 4% by weight of each antimicrobial agent present, more preferably from 0.001% to 0.1% by weight of each antimicrobial agent present.

EDTA may therefore be present in a film according to the present invention as an antioxidant, as a permeation enhancer or as a chelating agent. Typically, if EDTA is present, the EDTA acts as all of an antioxidant, a permeation enhancer and a chelating agent. Alternatively, if EDTA is present, the EDTA may act only as an antioxidant. Alternatively, if EDTA is present, the EDTA may act only as a permeation enhancer. Alternatively, if EDTA is present, the EDTA may act only as a chelating agent.

Typically, the film may additionally comprise at least one inorganic salt. Said inorganic salt may be any salt acceptable for use in the preparation of a medicament. Examples of such salts include, but are not limited to, the halides, oxides, hydroxides, sulfates, carbonates, phosphates, nitrates, acetates and oxamates of the alkali metals, alkaline earth metals, aluminium, zinc and ammonium. Typically, said inorganic salt may be selected from the group consisting of sodium chloride, potassium chloride, magnesium chloride, calcium chloride, and ammonium chloride. Preferably, the inorganic salt is sodium chloride. Typically, the inorganic salt is present in the film in a total concentration of at least 0.05 wt%, preferably in a concentration of from 0.1 to 5 wt%, more preferably from 0.2 to 2 wt%, yet more preferably from 0.25 to 1 wt%, and most preferably about 0.5 wt%.

Typically, the film may additionally comprise at least one excipient, optionally at least one basifying agent or acidifying agent, optionally at least one permeation enhancer, optionally at least one pharmaceutically acceptable solvent, optionally at least one buffering component, optionally a SEDDS (e.g. a SMEDDS or a SNEDDS), optionally at least one flavouring agent and optionally at least one antioxidant. For example, the film may comprise at least one excipient, optionally at least one basifying agent or acidifying agent, optionally at least one permeation enhancer, optionally at least one pharmaceutically acceptable solvent and optionally at least one flavouring agent. Preferably, the film may comprise glycerol, sorbitol, optionally at least one basifying agent or acidifying agent, optionally at least one permeation enhancer, optionally at least one pharmaceutically acceptable solvent and optionally at least one flavouring agent. More preferably, the film may comprise glycerol, sorbitol, optionally at least one basifying agent, preferably sodium hydroxide, optionally at least one pharmaceutically acceptable solvent, preferably DMSO or an oil or a mixture thereof, and optionally at least one flavouring agent.

Alternatively, the film may additionally comprise at least one excipient, at least one SEDDS and optionally at least one inorganic salt. Preferably, in this embodiment, the film additionally comprises glycerol, sorbitol, at least one SEDDS and optionally at least one inorganic salt. More preferably, the film additionally comprises glycerol, sorbitol, at least one SEDDS and at least one inorganic salt. Yet more preferably, the film additionally comprises glycerol, sorbitol, Caproyl PGMC, Chremophor EL and at least one inorganic salt. Still more preferably, the film additionally comprises glycerol, sorbitol, Caproyl PGMC, Chremophor EL, transcutol and sodium chloride. Most preferably, the film additionally comprises glycerol, sorbitol, xylitol, Caproyl PGMC, Chremophor EL, transcutol and sodium chloride.

Alternatively, the film may additionally comprise at least one excipient and at least one non-aqueous pharmaceutically acceptable solvent. Preferably, in this embodiment, the film additionally comprises glycerol, sorbitol and triacetin. More preferably, the film additionally comprises glycerol, sorbitol, xylitol and triacetin.

Preferably, the film according to the present invention comprises from 25% to 99% by weight of the alginate salt of a monovalent cation or the mixture of alginate salts containing at least one alginate salt of a monovalent cation, from 0% to 20% by weight of water, from 0.001% to 75% by weight of the API, from 0% to 40% by weight of glycerol, from 0% to 40% by weight of sorbitol, optionally from 0% to 40% by weight of xylitol, optionally a basifying agent or an acidifying agent, optionally from 0.01% to 5% by weight of a permeation enhancer, optionally from 0.1% to 10% by weight of a SEDDS (e.g. a SMEDDS or a SNEDDS), optionally from 0.01% to 5% by weight of a flavouring agent, optionally from 0.001% to 4% by weight of a chelating agent, and optionally from 0.001% to 4% by weight of at least one antioxidant. More preferably, the film according to the present invention comprises from 30% to 91% by weight of the alginate salt of a monovalent cation or the mixture of alginate salts containing at least one alginate salt of a monovalent cation, from 9% to 11% by weight of water, from 0.2% to 45% by weight of the API, from 10% to 20% by weight of glycerol, from 10% to 20% by weight of sorbitol, optionally from 10% to 20% by weight of xylitol, optionally from 0.1% to 3% by weight of a flavouring agent, and optionally a basifying agent or an acidifying agent.

Alternatively, the film according to the present invention consists of from 25% to 99% by weight of the alginate salt of a monovalent cation or the mixture of alginate salts containing at least one alginate salt of a monovalent cation, from 0% to 20% by weight of water, from 0.001% to 75% by weight of the API, from 0% to 40% by weight of glycerol, from 0% to 40% by weight of sorbitol, optionally from 0% to 40% by weight of xylitol, optionally a basifying agent or an acidifying agent, optionally from 0.01% to 5% by weight of a permeation enhancer, optionally from 0.1% to 10% by weight of a SEDDS (e.g. SMEDDS or SNEDDS), optionally from 0.01% to 5% by weight of a flavouring agent, optionally from 0.001% to 4% by weight of a chelating agent, and optionally from 0.001% to 4% by weight of at least one antioxidant. More preferably, the film according to the present invention consists of from 30% to 91% by weight of the alginate salt of a monovalent cation or the mixture of alginate salts containing at least one alginate salt of a monovalent cation, from 9% to 11% by weight of water, from 0.2% to 45% by weight of the API, from 10% to 20% by weight of glycerol, from 10% to 20% by weight of sorbitol, optionally from 10% to 20% by weight of xylitol, optionally from 0.1% to 3% by weight of a flavouring agent, and optionally a basifying agent or an acidifying agent.

A film according to the invention preferably has a thickness before drying of 200 to 2000 µm, more preferably from 300 to 1750 µm, even more preferably from 400 to 1500 µm, and most preferably about 1000 µm.

A film according to the invention preferably has a surface area on each of its two largest faces of from 0.1 to 20 cm², more preferably from 0.5 to 15 cm², even more preferably from 1 to 10 cm² and most preferably from 2 to 6 cm². Preferably, the surface area of each of the two largest faces of the film is about 3 cm².

The skilled person, having regard for the desired time of dissolution for a given application, will be able to select a suitable film thickness and surface area by simply preparing films of a range of different thicknesses and surface areas and testing the resultant films to measure the dissolution time.

The mechanical properties of a film according to the invention are very satisfactory. In particular, the film is flexible (i.e. it permits bending and folding without breaking), and has a high tensile strength. Importantly, the film of the present invention is not a gel, since the alginate polymer strands are not cross-linked with one another. The film of the invention is bioadhesive; that is to say that the film comprises a natural polymeric material (alginate) which can act as an adhesive. The film is adhesive to moist surfaces, such as mucosa. In particular, the film is adhesive to mucosa of the oral cavity, such as mucosa in the buccal, labial, sublingual, ginigival or lip areas, the soft palate and the hard palate.

The film according to the invention may be provided with printed text matter or printed images thereon, e.g. a brand name, a trade mark, a dosage indication or a symbol.

### Administration and uses of the films in treatment

In general, films of the present invention are administered to a human patients so as to deliver to the patient a therapeutically effective amount of the active pharmaceutical ingredient (API) contained therein.

As used herein, the term "therapeutically effective amount" refers to an amount of the API which is sufficient to reduce or ameliorate the severity, duration, progression, or onset of a disorder being treated, prevent the advancement of a disorder being treated, cause the regression of, prevent the recurrence, development, onset or progression of a symptom associated with a disorder being treated, or enhance or improve the prophylactic or therapeutic effect(s) of another therapy. The precise amount of API administered to a patient will depend on the type and severity of the disease or condition and on the characteristics of the patient, such as general health, age, sex, body weight and tolerance to drugs. It will also depend on the degree, severity and type of the disorder being treated. The skilled artisan will be able to determine appropriate dosages depending on these and other factors.

As used herein, the terms "treat", "treatment" and "treating" refer to the reduction or amelioration of the progression, severity and/or duration of a disorder being treated, or the amelioration of one or more symptoms (preferably, one or more discernible symptoms) of a disorder being treated resulting from the administration of a film according to the invention to a patient.

Typically, a film according to the present invention is provided for use in the treatment of a human patient. Preferably, the film according to the invention is provided for use in the treatment of a disease or condition selected from the group consisting of: cancer; dementia; Alzheimer's disease; amyotrophic lateral sclerosis; dystonia; epilepsy; Huntington's disease; multiple sclerosis; Parkinson's disease; spasticity; Tourette's syndrome; irritable bowel disease (IBD); Crohn's disease; ulcerative colitis; anorexia; cachexia; cancer-induced nausea and/or vomiting; cancer-induced cachexia; glaucoma; chronic pain; cancer-induced pain; fibromyalgia; neuropathic pain; addiction; anxiety; bipolar disorder; post-traumatic stress disorder; psychosis; schizophrenia; scleroderma; and type I diabetes. More preferably, the film according to the invention is provided for use in the treatment of a disease or condition selected from the group consisting of: dementia; Alzheimer's disease; epilepsy; inflammatory bowel disease; Crohn's disease; ulcerative colitis; chronic pain; cancer-induced pain; fibromyalgia; and neuropathic pain.

The present invention provides a film according to the invention for use in the treatment of Alzheimer's-related dementia. Dementia refers to a set of symptoms which include impaired thinking and memory. Many conditions can cause dementia, such as Parkinson's disease. Approximately 50-70% of all dementia cases are caused by Alzheimer's disease, which is characterized not only by impaired thinking and memory, but also by difficulty in problem solving, difficulties in completing simple tasks, confusion with respect to place and time, and difficulty in speaking and writing. [16]

The present invention provides a film according to the invention for use in the treatment of epilepsy. Epilepsy affects approximately 1% of the population, and is a chronic disorder characterised by recent seizures. An epileptic seizure is defined as a clinical event associated with a transient, hypersynchronous neuronal discharge. There are several different types of epileptic seizure, included absence seizures (also known as petit mal seizures), tonic-clonic or convulsive seizures (formerly known as grand mal), atonic seizures (also known as drop attacks), clonic seizures, tonic seizures, myoclonic seizures, and partial-onset seizures. Some seizures are more generalised, affecting the whole of the brain (e.g. absence seizures), whilst other seizures tend to remain more localised, affecting only part of the brain at onset (e.g. partial-onset seizures).

The present invention provides a film according to the invention for use in the treatment of inflammatory bowel disease (IBS). IBS is an umbrella term to describe inflammation in the small and/or large intestine. There are subtypes of IBS, depending on symptomatology and location, e.g., IBS with constipation, with diarrhoea, etc. The precise prevalence is uncertain, given the vague symptoms of the disease. Some suggest that 7-21% the adult population in the US has some form of IBS. [17] In some cases, changes in lifestyle, diet, and medications are sufficient to alleviate symptoms. However, for many, IBS remains a constant problem which can negatively impact quality of life.

The present invention provides a film according to the invention for use in the treatment of Crohn's disease. Crohn's is defined as chronic inflammation of the small intestine and the beginning of the ascending large intestine. An estimated 0.5 million people in the US suffer from Crohn's Disease, and prevalence is increasing for unknown reasons. Crohn's Disease patients have a very high likelihood of developing colon cancer. Current therapies range from oral anti-inflammatory agents (salicylic acid derivatives, steroids) to anti-TNF and other monoclonal antibodies. In severe cases, surgical resection of the affected areas is performed. [18]

The present invention provides a film according to the invention for use in the treatment of ulcerative colitis. As in Crohn's, UC is a chronic inflammatory condition, but of the large intestine. Over 900,000 Americans have ulcerative colitis, making it far more prevalent than Crohn's Disease. [19] Signs, symptoms, and treatment are largely the same as for Crohn's Disease.

The present invention provides a film according to the invention for use in the treatment of chronic pain. Pain relief is currently the most common condition being treated by medicinal cannabis. In Colorado, for example, 94% of the requests for medical marijuana identification cards are for the treatment of pain. [20] Not only are cannabinoids useful for pain reduction, but also for a reduced use/exposure to habit-forming opioids, which constitute an alternative therapy for this patient subtype.

The present invention provides a film according to the invention for use in the treatment of fibromyalgia. Fibromyalgia is a complex disease, in which pain is present alongside other symptoms such as fatigue, muscle stiffness, insomnia and mood disorders. It is believed that cannabinoid treatment might enable a multi-faceted treatment for fibromyalgia, because cannabinoids have been implicated in treatment of several of the individual symptoms of the disease.

The present invention provides a film according to the invention for use in the treatment of neuropathic pain. Neuropathic pain is pain caused by damage or disease affecting the somatosensory nervous system. It may be associated with abnormal sensations called dysesthesia or pain from normally non-painful stimuli (allodynia). It may have continuous and/or episodic (paroxysmal) components. The latter resemble stabbings or electric shocks. Common qualities include burning or coldness, "pins and needles" sensations, numbness and itching. Up to 7% to 8% of the European population is affected, and in 5% of persons the condition may be severe. [21]

Typically, the patient to be treated is an adult. Alternatively, the patient to be treated may be a child. The patient to be treated may be an elderly patient. The patient to be treated may be undergoing chemotherapy, and may therefore suffer from chemotherapy-induced nausea and/or vomiting, or cancer-related pain.

Typically, the film is administered to the oral cavity of the patient. The film is preferably applied to an oral mucosa in the buccal or labial or sublingual areas or to the soft palate. The film is typically applied by the patient themselves. Alternatively, the film is administered to the patient by another person, e.g. a medical practitioner, a nurse, a carer, a social worker, a colleague of the patient or a family member of the patient.

The film is bioadhesive and adheres to the surface of the oral cavity upon application. After application, the alginate film begins to dissolve, releasing the active pharmaceutical ingredient. Typically, the film fully dissolves in a time period of from 0.1 to 60 minutes or more after application to the mucosa of the oral cavity. Preferably, the film fully dissolves in a time period of from 0.5 to 30 minutes, more preferably from 1 to 20 minutes, still more preferably from 3 to 10 minutes, and most preferably from 3 to 5 minutes after application to the mucosa of the oral cavity.

Without wishing to be bound by any particular theory, it is believed that as the film dissolves within the oral cavity, the active pharmaceutical ingredient which is concomitantly released may enter the bloodstream by one or both of two different routes: (a) *via* absorption across the oral mucosa directly into the bloodstream (the "oral transmucosal route"); and (b) *via* swallowing into the stomach and subsequent absorption across the epithelium of the intestines into the bloodstream. Typically the peak plasma concentration of the API in a patient may be achieved within 120 minutes from adhesion of the film to the mucosa of the oral cavity, preferably within 60 minutes from adhesion, more preferably within 45 minutes, even more preferably within 30 minutes or 20 minutes from adhesion, and most preferably within 10 minutes from adhesion.

Typically, a single film is applied to the patient, generally to the mucosa of the oral cavity, at a given time. However, in some cases it may be desirable to apply two films simultaneously to achieve the correct dose for an individual patient. In some cases it may be desirable to apply more than two films simultaneously to achieve the correct dose for an individual patient, for example, three, four, five, six, seven, eight, nine, ten or more. Typically, a single dose given to a patient contains from 1 to 20 mg of the API, preferably from 2 to 15 mg of the API.

The present invention also provides a product comprising one or more films according to the invention, and packaging. Each of the films may individually be wrapped within a pouch, or multiple films may be wrapped together within the same pouch. Optionally, said pouch is made from PET-lined aluminium. The product may further comprise instructions for use of the film. These instructions may contain information on the recommended frequency or timing of use of the film by a patient, how to use remove the film from its pouch or packaging, how to adhere the film to a mucous membrane, and where within the oral cavity to adhere the film to a mucous membrane.

Any film or films of the present invention may also be used in combination with one or more other drugs or pharmaceutical compositions in the treatment of disease or conditions for which the films of the present invention and/or the other drugs or pharmaceutical compositions may have utility.

The one or more other drugs or pharmaceutical compositions may be administered to the patient by any one or more of the following routes: oral, systemic (e.g. transdermal, intranasal, transmucosal or by suppository), or parenteral (e.g. intramuscular, intravenous or subcutaneous). Compositions of the one or more other drugs or pharmaceutical compositions can take the form of tablets, pills, capsules, semisolids, powders, sustained release formulations, solutions, suspensions, elixirs, aerosols, transdermal patches, bioadhesive films, or any other appropriate compositions. The choice of formulation depends on various factors such as the mode of drug administration (e.g. for oral administration, formulations in the form of tablets, pills or capsules are preferred) and the bioavailability of the drug substance.

### Manufacture of the films

The films according to the invention may be manufactured by preparing a film-forming solution by addition and mixing of the constituent components of the film, distributing this solution onto a solid surface, and permitting the solution to dry on the surface to form a film. To distribute a solution or composition onto a solid surface the solution or composition may simply be poured onto and/or spread evenly over the surface, e.g. by use of a draw-down blade or similar equipment.

A typical method includes the process steps of:
(a) optionally, mixing at least one antioxidant in water, or in a mixed aqueous/organic solvent, or in one or more organic solvent(s);
(b) mixing the API in water, or in a mixed aqueous/organic solvent, or in one or more organic solvents to which water is subsequently added, or in the solution obtained in step (a), optionally wherein the pH of the solution is adjusted either before or after the addition of the API to the desired level by addition of an appropriate acid or base, typically a diluted aqueous acid or alkali, more typically a diluted aqueous alkali, and preferably wherein the pH of the solution is adjusted to from 3.0 to 13.5, more preferably from 8.0 to 13.5, and yet more preferably from 10.0 to 13.0;
(c) optionally, sonicating the solution, e.g. with an ultra-sonicating rod, until an emulsion is achieved;
(d) optionally, adding one or more excipients, flavouring agents, buffering components, permeation enhancers, SEDDS (e.g. SMEDDS or SNEDDS), chelating agents, antioxidants and/or antimicrobial agents;
(e) adding the alginate salt of monovalent cation under suitable conditions to result in the formation of a viscous cast, e.g. by mixing for about 30 minutes or until a lump free dispersion is achieved, optionally wherein further acid or base is added to the solution during the mixing process to maintain the pH of the solution at the desired level;
(f) optionally, adding one or more further excipients, flavouring agents, buffering components, permeation enhancers, SEDDS (e.g. SMEDDS or SNEDDS), chelating agents, antioxidants and/or antimicrobial agents;
(g) optionally, leaving the cast to de-aerate, typically for from 5 to 14 hours;
(h) pouring the cast onto a surface, e.g. a plate, preferably a glass plate, and spreading the cast out to the desired thickness, e.g. about 1 mm, typically by means of an applicator;
(i) drying the cast layer, typically at a temperature of from 30 to 70 °C, and preferably from 40 to 60 °C, until the residual water content of the film is from 0 to 20% by weight, preferably from 5 to 15% by weight, and more preferably from 8 to 10% by weight, and a solid film is formed; and
(j) optionally, cutting the solid film into pieces of the desired size, further optionally placing these pieces into pouches, preferably wherein the pouches are made from PET-lined aluminium, sealing the pouches and further optionally, labelling them.

An alternative method for manufacturing a film according to the invention includes the process steps of:
(a) mixing the alginate salt of monovalent cation in water, until a lump free dispersion is achieved, and optionally adding one or more excipients, flavouring agents, buffering components, permeation enhancers, SEDDS (e.g. SMEDDS or SNEDDS), chelating agents, antioxidants and/or antimicrobial agents to the aqueous solution either before or after the addition of the alginate salt;
(b) separately, dissolving the API in water, a mixed aqueous/organic solvent or one or more organic solvent(s), optionally wherein at least one antioxidant is pre-dissolved in the solvent, optionally wherein the pH of the solution is adjusted either before or after the addition of the API to the desired level by addition of an appropriate acid or base, typically a diluted aqueous acid or alkali, more typically a diluted aqueous alkali, and preferably wherein the pH of the solution is adjusted to from 3.0 to 13.5, more preferably from 8.0 to 13.5, and yet more preferably from 10.0 to 13.0;
(c) adding the solution obtained in step (a) to the solution obtained in step (b) under suitable conditions to result in the formation of a viscous cast, e.g. by mixing for about 20 minutes or until a lump free dispersion is achieved;
(d) optionally, adding one or more further excipients, flavouring agents buffering components, permeation enhancers, SEDDS (e.g. SMEDDS or SNEDDS), chelating agents, antioxidants and/or antimicrobial agents;
(e) optionally, leaving the cast to de-aerate, typically for from 5 to 14 hours;
(f) pouring the cast onto a surface, e.g. a plate, preferably a glass plate, and spreading the cast out to the desired thickness, e.g. about 1 mm, typically by means of an applicator;
(g) drying the cast layer, typically at a temperature of from 30 to 70 °C, and preferably from 40 to 60 °C, until the residual water content of the film is from 0 to 20% by weight, preferably from 5 to 15% by weight, and more preferably from 8 to 10% by weight; and
(h) optionally, cutting the solid film into pieces of the desired size, further optionally placing these pieces into pouches, preferably wherein the pouches are made from PET-lined aluminium, sealing the pouches and further optionally, labelling them.

Typically, in step (b) of the method for manufacturing a film according to the invention, the API is dissolved in water or in a solution obtained by mixing an antioxidant in water. In this case, it is preferable to either pre-adjust or subsequently adjust the pH of the solution to an alkaline pH by the addition of a base, preferably an aqueous alkali, more preferably a diluted aqueous alkali, and most preferably diluted sodium hydroxide. Without wishing to be bound by any particular theory, it is believed that use of an alkaline pH increases the solubility of cannabinoids in aqueous solution, which assists with the preparation of films in which a uniform dispersion of cannabinoid throughout the film is achieved.

Alternatively, in step (b) of the method for manufacturing a film according to the invention, the API is dissolved in a mixed aqueous/organic solvent or in a solution obtained by mixing an antioxidant in a mixed aqueous/organic solvent. In this case, the organic solvent used may be any suitable non-toxic solvent, including e.g. ethanol, acetone, benzyl alcohol, diethylene glycol monoethyl ether, glycerine, hexylene glycol, isopropyl alcohol, polyethylene glycols, methoxypolyethylene glycols, diethyl sebacate, dimethyl isosorbide, propylene carbonate, dimethyl sulfoxide (DMSO), fatty acid esters, and oils such as soybean oil, peanut oil, olive oil, palm oil, rapeseed oil, corn oil, other vegetable oils and the like. The mixed aqueous/organic solvent system may also comprise two, three, four or more organic solvents. Preferably, the organic solvent is selected from ethanol, dimethyl sulfoxide and oils such as soybean oil, peanut oil, olive oil, palm oil, rapeseed oil, corn oil, other vegetable oils and the like. Most preferably, the mixed aqueous/organic solvent system comprises both ethanol and an oil, e.g. olive oil. Alternatively, the mixed aqueous/organic solvent system comprises both dimethyl sulfoxide and an oil, e.g. olive oil. In the mixed aqueous/organic solvent system, the ratio of aqueous:non-aqueous solvent may be from 1:10,000 to 10,000:1, preferably from 1:100 to 100:1, more preferably from 1:10 to 100:1, even more preferably from 1: 1 to 100:1, still more preferably from 2: 1 to 100:1, yet more preferably from 5:1 to 50:1, and most preferably from 4: 1 to 20: 1.

Alternatively, in step (b) of the method for manufacturing a film according to the invention, the API is dissolved in one or more organic solvents or in a solution obtained by mixing an antioxidant in one or more organic solvents. In this case, water is added to the organic solution comprising the API and optionally an antioxidant after addition of the API to the organic solvent(s). Each organic solvent used may be any suitable non-toxic solvent, such as those listed in the paragraph above. Typically, the API is dissolved in a single organic solvent, preferably ethanol, dimethyl sulfoxide or an oil such as soybean oil, peanut oil, olive oil, palm oil, rapeseed oil, corn oil, other vegetable oils and the like. Alternatively, the API is dissolved in a mixture of two, three, four or more organic solvents, preferably a mixture comprising ethanol and an oil, e.g. olive oil, or a mixture comprising dimethyl sulfoxide and an oil, e.g. olive oil.

Typically, the solution is sonicated in step (c) of the first method above when the solution obtained in the preceding step comprises both aqueous and organic solvents.

Typically, in the method for manufacturing a film according to the invention, when an antioxidant is present in the film, the antioxidant is dissolved in aqueous solution prior to the addition of the API.

In an alternative variant of any of the above methods, after the viscous cast is poured onto a surface, it is first spread out to a thickness of about 2 mm by means of an applicator with a slit height of about 2 mm, and is then subsequently spread out to a thickness of about 1 mm by means of an applicator with a slit height of about 1 mm.

When the films are to be formulated as emulsion-based films, an alternative method for manufacturing a film according to the invention that is particularly preferred includes the process steps of:
(a) mixing the API in an oil phase;
(b) premixing a surfactant and a cosolvent, and then adding this to the solution obtained in step (a) under mixing;
(c) optionally, adding one or more excipients, flavouring agents, buffering components, permeation enhancers, chelating agents, antioxidants and/or antimicrobial agents to water;
(d) adding water, or the solution obtained in step (c), to the solution obtained in step (b) under stirring, preferably continuous stirring, and more preferably wherein the water or the solution obtained in step (c) is added in a dropwise fashion;
(e) optionally, storing the solution obtained in step (d) overnight and subsequently evaluating its physical stability;
(f) mixing the alginate salt of monovalent cation in the solution, until a lump free dispersion is achieved, and optionally adding further water to modulate the viscosity of the cast formed;
(g) pouring the cast onto a surface, e.g. a plate, preferably a glass plate, and spreading the cast out to the desired thickness, e.g. about 1 mm, or about 1.2 mm if further water was added in step (f), typically by means of an applicator;
(h) drying the cast layer, typically at a temperature of from 30 to 70 °C, preferably from 30 to 50 °C, and most preferably about 40 °C, until the residual water content of the film is from 0 to 20% by weight, preferably from 5 to 15% by weight, and more preferably from 9 to 11% by weight; and
(i) optionally, cutting the solid film into pieces of the desired size, further optionally placing these pieces into pouches, preferably wherein the pouches are made from PET-lined aluminium, sealing the pouches and further optionally, labelling them.

When the films are to be formulated as non-emulsion-based films, an alternative method for manufacturing a film according to the invention that is particularly preferred includes the process steps of:
(a) mixing the API in a solubilizing agent;
(b) adding the resultant solution to water, preferably under high shear mixing;
(c) optionally, adding one or more excipients, flavouring agents, buffering components, permeation enhancers, chelating agents, antioxidants and/or antimicrobial agents;
(d) mixing the alginate salt of monovalent cation in the solution, until a lump free dispersion is achieved, and optionally adding further water to modulate the viscosity of the cast formed;
(e) pouring the cast onto a surface, e.g. a plate, preferably a glass plate, and spreading the cast out to the desired thickness, e.g. about 1 mm, typically by means of an applicator;
(f) drying the cast layer, typically at a temperature of from 30 to 70 °C, preferably from 50 to 70 °C, and most preferably about 60 °C, until the residual water content of the film is from 0 to 20% by weight, preferably from 5 to 15% by weight, and more preferably from 9 to 11% by weight; and
(g) optionally, cutting the solid film into pieces of the desired size, further optionally placing these pieces into pouches, preferably wherein the pouches are made from PET-lined aluminium, sealing the pouches and further optionally, labelling them.

Typically, the alginate salt(s) are added to the API-containing water solution. Alternatively, the API and the alginate salt(s) are both dissolved together in solution. Alternatively, the API may be added to the alginate solution so as to give an emulsion or suspension of the API in the alginate solution. Alternatively, the film-forming composition of the invention may comprise both dissolved and non-dissolved active ingredients. For example, a film-forming composition may comprise a combination of active ingredient dissolved in the alginate solution and active ingredient suspended in the solution.

Additional API may be applied to the surface of the film before or after drying, e.g. as an aerosol spray onto a dry or wet film. An active ingredient may also be applied as a powder onto the surface of the film. A flavouring agent may additionally be applied in such a way.

Herein, any reference to a term in the singular also encompasses its plural. Where the term "comprising", "comprise" or "comprises" is used, said term may substituted by "consisting of', "consist of" or "consists of" respectively, or by "consisting essentially of", "consist essentially of" or "consists essentially of" respectively. Any reference to a numerical range or single numerical value also includes values that are about that range or single value. Any reference to a compound of Formula (I) to (XLVI) also encompasses a physiologically acceptable salt thereof unless otherwise indicated. Any reference to alginate encompasses any physiologically acceptable salt thereof unless otherwise indicated. Unless otherwise indicated, any % value is based on the relative weight of the component or components in question.

### Examples

The following are Examples that illustrate the present invention. However, these Examples are in no way intended to limit the scope of the invention.

### Example 1: Preparation of cannabidiol-containing films

Four basic film formulation protocols were developed. The first film formulation protocol (method A) produced cannabidiol-containing films by mixing the film components in an aqueous solution without pH adjustment. The second film formulation protocol (method B) produced cannabidiol-containing films by mixing the film components in an alkaline aqueous solution, wherein the pH of the film formulation prior to coating and drying was adjusted to about 12.5-13. The third and fourth film formulation protocols (methods C and D) produced cannabidiol-containing films by mixing the film components in a water/DMSO or water/oil mixture, respectively, but without pH adjustment.

Batch formulae comprising cannabidiol as the API at 5 mg/dose are set out in Table 1 below. Calculations are based on yields of 2000 doses/batch (dose size = 6 cm²).

**Table 1. Batch formulae for production of cannabidiol-containing films.**

| **Component** | **Batch formulae** | | | | **Function** |
|---|---|---|---|---|---|
| | **A** | **B** | **C** | **D** | |
| Cannabidiol (g) | 10 | 10 | 10 | 10 | API |
| Water (mL) | 195 | 195 | 195 | 195 | Solvent |
| DMSO (mL) | - | - | 10 | - | Solvent |
| Olive oil (mL) | - | - | - | 20 | Solvent |
| Sorbitol (g) | 7 | 7 | 7 | - | Plasticizer |
| Glycerol (g) | 6 | 6 | 6 | - | Plasticizer |
| Peppermint oil (mL) | 2 | 2 | 2 | 2 | Flavouring agent |
| Sodium alginate (Protanal^{®} LFR 5/60) (g) | 26.7 | 26.7 | 26.7 | 26.7 | Film-forming polymer |
| 4 M sodium hydroxide (mL) | - | 10 | - | - | pH adjustment |

The films in batch formula A were prepared according to the following method (method A):
- The cannabidiol was added to the purified water and mixed for 10 minutes.
- The sodium alginate, glycerol, sorbitol and peppermint oil were added to the solution under mixing, and mixing was continued for at least 15 minutes.
- The cast was left overnight for de-aeration.
- The cast was poured onto a glass plate and spread out to a thickness of 1 mm by means of an applicator.
- The cast layer was dried in a drying cabinet heated to approximately 60 °C until a residual water content of from 8% to 10% by weight was achieved and a solid film was formed.
- The solid film was cut into pieces measuring 20 x 30 mm with a knife.
- The resulting films were placed individually into PET-lined aluminium pouches, sealed with a heat sealer and labelled.

The films in batch formula B were prepared according to the following method (method B):
- Purified water was added to the mixing vessel, and the pH was adjusted to from 12.5 to 13 by addition of 4 M sodium hydroxide.
- The cannabidiol was added to the alkaline solution and was mixed until a clear solution was obtained.
- The sodium alginate was added to the mixture under mixing until a lump free dispersion was achieved. Throughout the mixing process, the pH was continually maintained at from 12.5 to 13 by the addition of further 4 M sodium hydroxide.
- The glycerol, sorbitol, and peppermint oil were added to the solution under mixing, and mixing was continued for at least 15 minutes.
- The cast was left overnight for de-aeration.
- The cast was poured onto a glass plate and spread out to a thickness of 1 mm by means of an applicator.
- The cast layer was dried in a drying cabinet heated to approximately 60 °C until a residual water content of from 8% to 10% by weight was achieved and a solid film was formed.
- The solid film was cut into pieces measuring 20 x 30 mm with a knife.
- The resulting films were placed individually into PET-lined aluminium pouches, sealed with a heat sealer and labelled.

The films in batch formula C were prepared according to the following method (method C):
- The sodium alginate was dissolved in the purified water under mixing until a lump free dispersion was achieved.
- The glycerol and sorbitol were then added to this solution under mixing.
- Separately, the cannabidiol was dissolved in DMSO under mixing.
- The cannabidiol/DMSO solution was added to the aqueous alginate-containing solution under mixing.
- The peppermint oil was added to the solution under mixing, and mixing was continued for at least 15 minutes or until a smooth and homogeneous solution was obtained.
- The cast was left overnight for de-aeration.
- The cast was poured onto a glass plate and spread out to a thickness of 1 mm by means of an applicator.
- The cast layer was dried in a drying cabinet heated to approximately 60 °C until a residual water content of from 8% to 10% by weight was achieved and a solid film was formed.
- The solid film was cut into pieces measuring 20 x 30 mm with a knife.
- The resulting films were placed individually into PET-lined aluminium pouches, sealed with a heat sealer and labelled.

The films in batch formula D were prepared according to the following method (method D):
- The cannabidiol was dissolved in the olive oil under mixing to obtain a clear solution.
- This cannabidiol/oil solution was added to the purified water.
- The oil/water mixture was sonicated using an ultra-sonicating rod until an emulsion was created.
- The sodium alginate was added to the solution/emulsion under mixing until a lump free and smooth solution/emulsion was achieved.
- The peppermint oil was added to the solution under mixing, and mixing was continued for at least 15 minutes or until a smooth and homogeneous solution was obtained.
- The cast was left overnight for de-aeration.
- The cast was poured onto a glass plate and spread out to a thickness of 1 mm by means of an applicator.
- The cast layer was dried in a drying cabinet heated to approximately 60 °C until a residual water content of from 8% to 10% by weight was achieved and a solid film was formed.
- The solid film was cut into pieces measuring 20 x 30 mm with a knife.
- The resulting films were placed individually into PET-lined aluminium pouches, sealed with a heat sealer and labelled.

### Example 2: Physical evaluation of cannabidiol-containing films

After manufacture, each of the batches of cannabidiol-containing films may be evaluated with respect to the following criteria:

| **Property** | **Criteria** |
|---|---|
| 1. Cast texture: | lump free, homogenous viscous cast (visual inspection) free of bubbles prior to coating (visual inspection) |
| 2. Residual moisture*: | 8-10% (in process control) |
| 3. Film appearance: | - translucent, transparent and colour homogenous (visual inspection) |
| | - smooth and flat surface structure (visual inspection) |
| | - pliable and flexible (visual inspection) |
| 4. Dose weight homogeneity: | weighing of doses randomly selected within a film batch |
| 5. Cannabidiol content**: | target dose strength within ±10% by weight (RP-HPLC analysis) |

| | |
|---|---|
| ^{∗} *Residual moisture:* IR-induced water vaporization combined with real-time weight measurement was used. Percentage of change in weight at start until no further change was observed as the measure of residual moisture. ^{∗∗} *Cannabidiol content and homogeneity:* Reverse phase high-performance liquid chromatography (RP-HPLC) separation with detection at 220 nm was used. Amount of cannabidiol/dose was calculated using a cannabidiol standard curve. [22] | |

It was noted that the cannabidiol-containing films produced using method A contained crystals of cannabidiol, which is undesirable. The films produced using methods B, C and D were of higher quality. This suggests that use of an alkaline solution and/or a mixed aqueous/organic solvent system is required for optimal preparation of cannabidiol-containing films.

### Example 3: Preparation of further cannabidiol-containing alginate films

Further developmental work was subsequently carried out on the manufacture of cannabidiol-containing alginate films, to explore various formulation conditions for the development of prototypes on a lab scale for preclinical studies and as a basis for scale-up to full-scale product protocols.

An initial batch formula as set out in Table 2 below was used as the starting point for these investigations.

**Table 2. Basic batch formula for alginate-based films. A basic batch formula corresponds to approximately 200 mL cast with a yield of about 1000 doses at 3 cm², if coated at a thickness of 1.0 mm.**

| **Ingredient** | **Amount** | **Function** |
|---|---|---|
| Sodium alginate (Protanal 5/60) | 26.7 g | Film-Forming Polymer |
| Water | 197 mL | Solvent |
| Glycerol | 6 g | Plasticizer |
| Sorbitol | 7 g | Plasticizer |

The dimension of the doses was set to 1.5 x 2.0 cm (3 cm²), a size that was considered easy to handle and easy to apply to the oral mucosa.

The target dose strength of CBD buccal film in this study was set to as high as possible, i.e. 10-15 mg/dose, where the desired drug product is physically and chemically stable (crystal free as well as no oil release in the film). However, initial experiments were carried out at lower dose strength 5 mg/dose.

The alginate selected for use was Protanal 5/60, in order to maximize the possibility for mucoadhesion. Protanal 5/60 generally has an average molecular weight within a range from about 20,000 g/mol to about 90,000 g/mol. Protanal 5/60 has an average guluronic acid content of from 65 to 75% and an average mannuronic acid content of from 25 to 35%. This sodium alginate further has a viscosity of 300-700 mPas as measured in a 10% aqueous solution at a temperature of 20 °C and at a shear rate of 20 rpm by use of a Brookfield viscometer with a spindle No. 2.

Cannabidiol (CBD), with an aqueous solubility of 0.0126 mg/mL and log P value of 6.33, is considered a poorly water-soluble or lipophilic drug. However, CBD has a *p*Kₐ value of 9.13 which indicates that it is soluble at concentrations up to 300 mg/mL at higher pH i.e. above pH 12. Thus, it is possible to formulate CBD films at higher pH, but it was considered more desirable to investigate lipid-based CBD film formulations.

Lipid-based CBD films can be emulsion or oil (non-emulsion)-based formulations. In this regard, self-emulsifying drug delivery systems or formulations (SEDDS) have been used for emulsion-based CBD films which can be either in micron size, i.e. self-microemulsifying drug delivery system (SMEDDS), or in nanometer size, i.e. self-nanoemulsifying drug delivery system (SNEDDS). In the present study, emulsion-based CBD formulations were considered SEDDS or SMEDDS depending on whether the drug-containing lipid mixture spontaneously forms emulsions or microemulsions upon dilution with water. Generally, SEDDS and SMEDDS are mixtures of lipid, surfactant and cosolvent (also known as cosurfactant) with dissolved drug which disperse in water spontaneously or upon mild agitation to form microemulsion (oil droplet size <250 nm), fine emulsion (250-1000 nm) or regular emulsion (oil droplet size >1000 nm).

Often short chain alcohols such as ethanol, propylene glycol etc. are added in the formulations as cosolvents to increase drug loading, decrease gel phase formation and reduce the size of droplets in emulsion or microemulsion. In addition, surfactants with higher HLB values (10-15) were considered to obtain stable emulsions with a finer droplet size. In this regard, nonionic surfactants were selected due to their lower toxicity on human cells as compared to the ionic surfactants. Nonionic surfactants such as cremophor EL, Tween 80, Tween 20, labrasol or combinations thereof were used in the formulations as surfactant.

The solubility of CBD in various oils, surfactants and cosolvents, as set out in Table 3 below, was determined by adding a known amount of drug in 1 g of each oil, surfactant and cosolvent in different addition steps. For that, 50 mg CBD was added in each step until saturation of the solutions was achieved. Mixtures were kept on magnetic stirring at room temperature. During the first five hours, the vials were inspected every half hour but the substance was not completely dissolved. The vials were then left to stir overnight at room temperature. The next day, test solutions were inspected, and it was found that the solutions were unclear, i.e. already in a saturated condition. The experiment was stopped and the total amount of drug added prior to the saturation condition of each solution was calculated. The calculated value, as can be seen in Fig. 1, presents approximate solubility (g/g) of CBD in the different solvents.

**Table 3. Different types of solvents used for CBD solubility screening experiment, their chemical description and specific functions.**

| **Solvent** | **Chemical description** | **Function** |
|---|---|---|
| Olive oil | Long chain triglycerides | Oil phase |
| Soyabean oil | Long chain triglycerides | Oil phase |
| Capryol PGMC | Medium-chain triglycerides | Oil phase |
| Maisine CC | Glyceryl monolinoleate | Oil phase |
| Labrafil M2125 | Linoleoyl polyoxylglyceride | Oil phase |
| Captex 355 | Medium-chain triglycerides | Oil phase |
| Triacetin | Glycerol triacetate | Oil phase |
| Transcutol | 2-(2-ethoxyethoxy)-ethanol | Cosolvent |
| PEG 400 | Polyethylene glycol | Cosolvent |
| DMSO | Dimethyl sulfoxide | Cosolvent |
| Ethanol | Ethyl alcohol | Cosolvent |
| Cremophor EL | Polyoxyl 35 castor oil | Surfactant |
| Tween 80 | Polyoxyethylene sorbitan fatty acid esters | Surfactant |
| Labrasol | Caprylocaproyl polyoxylglycerides | Surfactant |

Since CBD formulation at higher dose strength is required, higher oil content may be desirable to dissolve more drug in the self-emulsifying mixture and increase its content in the film. However, there is a limit imposed on how much oil may be used, due to possible adverse effects on emulsion stability and film pliability. Therefore, solvents displaying maximum drug solubility, i.e. capryol PGMC (which consists of propylene glycol mono- and di-esters of caprylic acid) as oil phase and transcutol (highly purified diethylene glycol monoethyl ether) as cosolvent, were selected for the emulsion-based formulations. Surfactants displaying higher HLB values such as the polyoxyethylene caster oil derivative Cremophor EL (13.9) and Tween 80 were selected for the emulsion-based formulations.

The use of transcutol as a cosolvent is believed to contribute to the formation of emulsion/microemulsion by multiple mechanisms such as through reducing interfacial tension and viscosity, with the cosolvent molecules positioning themselves in-between the surfactant tails and thus increasing the flexibility and fluidity of the interfacial film.

### Preparation of emulsion for emulsion-based CBD films

Capryol PGMC (oil), Cremophor EL and Tween 80 (surfactants) and transcutol (cosolvent) were thus used to prepare a microemulsion for dissolving CBD (see Table 4 below). Furthermore, placebo formulations containing different concentrations of oil as well as mass ratio of surfactant to cosolvent (Sₘᵢₓ ratio) were also prepared to evaluate the stability of emulsion in the alginate film. In addition, viscosity of cast and pliability of film were also considered as selection criteria and tested on placebo films with the same compositions used for the drug incorporation.

**Table 4. Composition of SMEDDS with the basic formulation for 5 mg CBD films.**

| **Ingredient** | **Amount** | **Concentration (w/w)** | **Function** |
|---|---|---|---|
| Capryol PGMC | 1.5 g | 3% | Oil |
| Cremophor EL/ Tween 80 | 2.5 g | 5% | Surfactant |
| Transcutol | 2.5 g | 5% | Cosolvent |
| NaCl | 0.25 g | 0.5% | Salt |
| Water | 50 mL | | Solvent |
| Glycerol | 1.5 g | | Plasticizer |
| Sorbitol | 1.75 g | | Plasticizer |
| Sodium alginate (Protanal 5/60) | 6.65 g | | Film-Forming Polymer |

### Preparation of vehicle for non-emulsion based CBD films

Based on the above solubility experiments, vehicles displaying maximum CBD solubility were considered for non-emulsion-based CBD formulation. DMSO, transcutol and triacetin were selected for this purpose. As shown in Table 5 below, placebo formulations were prepared with these vehicles to decide on film appearance, texture and pliability.

**Table 5. Basic formulation with different vehicles for the non-emulsion-based CBD film.**

| **Ingredient** | **Amount** | **Concentration (w/w)** | **Function** |
|---|---|---|---|
| DMSO | 2.5 g | 5% | Solubilizer |
| Transcutol | 2.5 g | 5% | Solubilizer |
| PEG 400 | 2.5 g | 5% | Solubilizer |
| Triacetin | 4 g | 8% | Solubilizer |
| Water | 50 mL | | Solvent |
| Glycerol | 1.5 g | | Plasticizer |
| Sorbitol | 1.75 g | | Plasticizer |
| Sodium alginate (Protanal 5/60) | 6.65 g | | Film-Forming Polymer |

### Physical evaluation criteria

After manufacture, each of the batches of cannabidiol-containing films may be evaluated with respect to the following criteria:

| **Property** | **Criteria** |
|---|---|
| 1. Cast texture: | lump free, homogenous viscous cast (visual inspection) free of bubbles prior to coating (visual inspection) |
| 2. Residual moisture^{∗}: | 9-11% (in process control) |
| 3. Film appearance^{∗∗}: | - translucent and colour homogenous (visual inspection) |
| | - smooth and flat surface structure (visual inspection) |
| | - pliable and flexible (visual inspection) |
| 4. Dose weight homogeneity: | weighing of doses randomly selected within a film batch |
| 5. Cannabidiol content^{∗∗∗}: | target dose strength within ± 12% by weight (RP-HPLC analysis) |
| 6. Physical stability | - oil release (visual inspection) |
| | - crystal free film (optical microscopy study) |

| | |
|---|---|
| ^{∗} *Residual moisture*: IR-induced water vaporization combined with real-time weight measurement was used. Percentage of change in weight at start until no further change was observed as the measure of residual moisture. ** Some film batches were inspected and analysed with respect to surface structure under a light microscope. ^{∗∗∗} *Cannabidiol content and homogeneity*: Reverse phase high-performance liquid chromatography (RP-HPLC) separation with detection at 210 nm was used. Amount of cannabidiol/dose was calculated using a cannabidiol standard curve. [22] | |

### Protocol for preparation of formulations

The formulations set out in Table 6 below were evaluated in this study. Formulations were prepared by using different types of vehicles for non-emulsion-based formulations as well as factors affecting emulsion-based CBD formulation.

**Table 6. Formulations evaluated in this study.**

| **Emulsion-based CBD formulations** | **Non-emulsion-based CBD formulations** |
|---|---|
| Effect of salt | Basic recipe with DMSO |
| Effect of buffer | Basic recipe with Transcutol |
| Effect of cosolvent | Basic recipe with PEG 400 |
| Effect of additional surfactant | Basic recipe with Triacetin |
| Effect of plasticizer | - |
| Effect of temperature | - |

### Production of emulsion-based CBD films

The batch formula for 5 mg dose strength of emulsion-based CBD films is listed in Table 4 above. For this, self-emulsifying mixture consists of medium chain triglycerides, capryol PGMC as oil phase, Cremophor EL as surfactant and transcutol as cosolvent. The mass ratio of surfactant to cosolvent (Sₘᵢₓ ratio) was kept constant at 1:1 by weight. For the preparation of the CBD microemulsion, CBD was solubilized in 3% w/w of oil phase (capryol PGMC) and the surfactant and cosolvent were added under continuous stirring. Finally, milliQ water was added to the lipid mixture in dropwise manner. These emulsions were stored overnight and later, subjected to visual assessment of physical stability (i.e. presence of coalescence or phase separation).

Films that were physically stable (no oil release) at 5 mg dose strength were further formulated at increased dose strength, i.e. 10 mg/dose. In this case, the effect of different factors as mentioned in Table 6 above was considered to obtain a physically stable CBD film at the higher dose strength (10 mg/dose).

Since an emulsion-based CBD formulation results in a viscous cast when mixing the alginate in the pre-cast solution, further dilution of the cast was required to achieve a less viscous cast. For that, an additional 10 mL of milliQ water was added to the final cast resulting in a lower CBD dose per film compared to the standard API dose calculation. To compensate for this, the emulsion-based cast was coated at 1.2 mm thickness (as opposed to a 1 mm thickness) to obtain the required dose strength.

### Production of non-emulsion-based CBD films

Non-emulsion-based CBD films, which utilize vehicles as solubilizers for CBD, were also prepared to evaluate basic characteristics such as pliability, tensile strength of CBD films at higher dose strength (10 mg/dose) as well as to avoid obtaining very viscous cast of alginate-based CBD films. The solubilizers used were organic solvents, glycols or oils. These films were prepared using the following protocol:
- CBD was added to the vehicle and mixed for 1 hr under magnetic stirring so that a clear solution is obtained (solution 1).
- Glycerol, sorbitol and xylitol were added to water and mixed (solution 2).
- Solutions 1 and 2 were mixed together using an Ultra-Turrax^{®} tube drive for 5 mins.
- Sodium alginate was added to the precast solution under mixing (in a small food processor) for approximately 20 minutes or until a lump free dispersion was achieved, resulting in a viscous cast.
- The cast was coated immediately on a glass plate at a thickness of 1.2 mm by means of an applicator.
- The cast layer was dried in a drying cabinet heated to approximately 50 °C until a water content of 9-11% was achieved and a solid film was formed.
- The solid film was cut into rectangles with dimension 1.5 × 2.0cm with a knife.
- The resulting films were placed individually into aluminum pouches, sealed with a heat sealer and labeled.

The specific solubilizers used in these formulations are set out in Table 6 above.

### Properties of emulsion-based CBD films

CBD was fully dissolved in the oil phase. Lump free and homogenous (whitish) viscous casts could be prepared with each individual batch formula/protocol. Viscosity was found to increase with increasing content of the microemulsion components i.e. oil phase, surfactant or cosolvent.

It was observed that the viscosity of emulsion-based casts increases with time. Films were thus coated on glass plate immediately after the cast was prepared. Air bubbles generated during preparation of the casts and thus introducing inhomogeneity in the films, were removed by coating the cast on the glass plate and leaving it for 20 mins at room temperature for passive de-aeration prior to drying.

All prepared films had smooth, and flat surface structures with flexible properties when dried to a water content of 9-11%. Particularly, emulsion-based CBD films were whitish, homogeneous in appearance, but more opaque.

Quantitative determination of CBD in films was performed via RP-HPLC in isocratic mode using FAST analytical method (where UV detection at wavelength 210 nm was used). However, more stable (no oil release) CBD formulations at higher dose strength (10 mg) were analyzed using gradient analytical method where UV detection at wavelength 210 nm was used.

Several factors affecting the stability of emulsion containing CBD at different dose strength, under drying condition were considered in the study.

### (a) Effect of salt

To evaluate salt effect on the stability of emulsion-based CBD films, a self-emulsifying formulation concept was utilized. For that, capryol PGMC (oil), Cremophor (surfactant) and transcutol (cosolvent) were used. The batch formulae prepared are as set out in Table 7 below.

**Table 7. Batch formulae for CBD films at different dose strength (5 mg and 10 mg) produced in the study. The batch size is about 250 doses (dose dimension 3 cm²)**

| **Ingredient** | **Concentration (w/w)** | **Amount (g)** | | **Function** |
|---|---|---|---|---|
| | | 5 mg CBD films | 10 mg CBD films | |
| CBD | | 1.25 | 2.5 | API |
| Capryol PGMC | 3% | 1.5 | 1.5 | Oil phase |
| Cremophor EL | 5% | 2.5 | 2.5 | Surfactant |
| Transcutol | 5% | 2.5 | 2.5 | Cosolvent |
| NaCl | 0.5% | 0.25 | 0.25 | Stabilizer |
| Sorbitol | | 1.75 | 1.75 | Plasticizer |
| Glycerol | | 1.5 | 1.5 | Plasticizer |
| Xylitol | | 2.5 | 2.5 | Plasticizer |
| Water | | 50 | 50 | Solvent |
| Sodium alginate (Protanal 5/60) | | 6.65 | 6.65 | Film-Forming Polymer |

Emulsion-based 5 mg CBD films were prepared with and without addition of the NaCl component listed in Table 7. It was found that CBD films without salt resulted in partial sweating of oil droplets in the film during the drying process. The film surface and the glass plate (inspected after removal of the films) were somewhat greasy. In contrast, no such partial oil release was observed with CBD formulation containing NaCl. As could be confirmed using light microscopy, a homogeneous, crystal free emulsion was formed at 5 mg CBD dose strength.

Crystals did not appear in polarized light microscope images of the fresh 5 mg CBD film nor in a film exposed to the normal air for 5 days at room temperature. Together, no oil release was observed in fresh 5 mg CBD films and films exposed to normal air for 5 days at room temperature. However, a few scattered droplets, bigger in size were seen in the CBD film in non-polarized view. These were considered to be oil droplets formed due to coalescence of small oil droplets in the drying process.

Film weight, dose and homogeneity data are presented in Table 8 below. Acceptable dose variation as well as good homogeneity (µg CBD/mg film) among the films within the batch were observed.

**Table 8. Weight, dose and homogeneity data for physically stable 5 mg emulsion-based CBD formulation in presence of small amount of salt, coated at 1.2 mm film thickness.**

| ***Emulsion-based CBD formulation (5 mg*/*dose) (#Batch 25)*** | |
|---|---|
| *Average weight (g)* | 3.941 |
| *Standard deviation* | 0.326 |
| *RSD%* | 8.28 |
| *CBD* (*µg*/*mg of film*) | 66.58 |
| *Number of films analysed* | 3 |

The stabilizing effect of small amount of salt addition to the emulsion was thought to participate in partial screening of charges on the surface of oil droplets as well as serving as an agent that facilitates emulsifiers to coacervate on the droplet surface, hence increasing the emulsion stability in the drying process. However, salt addition at increased concentration may destroy the protective double layer on the oil droplets surface, causing agglomeration and phase separation and thus reducing the stability of emulsions. The process is called "salting out". Thus, addition of salt at lower concentration (< 0.1 M) in the emulsion may be most preferred in terms of stabilizing the oil droplets.

Since increased concentration of emulsion components is linked to increased viscosity of the cast, 10 mg CBD films were formulated using the same amount of emulsion components as the 5 mg CBD films (Table 7). As seen under a light microscope, a homogeneous, stable and crystal free (polarized view) emulsion was formed. The CBD cast at 10 mg dose was coated and subjected to the drying process. Oil release was observed from the film during the drying process. These films were analyzed under optical microscope and the presence of shiny spots was observed under polarized view, thus confirming the presence of crystals in 10 mg CBD films. It seems that addition of a low concentration of salt is not necessarily sufficient to overcome the osmotic pressure at the curvature of oil droplets and optimally stabilize the oil droplet surface at a higher CBD dose strength.

Furthermore, another surfactant with higher HLB value than Cremophor EL, Tween 80 (HLB value 15) was alternatively used with and without salt addition for the preparation of 10 mg CBD films. As observed under light microscopy, a homogeneous and crystal free (polarized view) emulsion, with relatively larger droplet size compared to formulations containing Cremophor EL, was formed in the absence of salt. Again, however, it was observed that oil was released while drying the film, and the presence of crystals in the film was confirmed by light microscopy (polarized view). Similar results were obtained when NaCl was added to the emulsion.

It was concluded that surfactant with higher HLB value in combination with the small amount of salt does not optimally stabilize the oil droplets in the drying process at a higher CBD dose strength.

### (b) Effect of buffer

Another approach to stabilizing the high dose strength CBD films involved buffer incorporation in the 10 mg CBD formulation at neutral pH. Without wishing to be bound by any particular theory, it is believed that the buffer may act as an indirect stabilizing agent for emulsion droplets through interacting as counterions at the droplet surface and thus stabilizing emulsion droplets in the drying process. The droplets may be sufficiently stable to coalescence when the entire interfacial layer is closely enough packed due to drying the film, compensating for the higher osmotic pressure of the droplets caused by higher drug loading in the emulsion droplets.

For this purpose, 50 mM phosphate buffer at pH 6.5 was used alone in the CBD formulation and in combination with 0.5% w/v NaCl, as shown in Table 9.

**Table 9. Batch formula for 10 mg CBD films produced in the study. The batch size is about 250 doses (dose dimension 3 cm²)**

| **Ingredient** | **Concentration (w/v)** | **Amount (g)** | **Function** |
|---|---|---|---|
| CBD | | 2.5 | API |
| Capryol PGMC | 3% | 1.5 | Oil phase |
| Cremophor EL | 5% | 2.5 | Surfactant |
| Transcutol | 5% | 2.5 | Cosolvent |
| ± NaCl | 0.5% | 0.25 | stabilizer |
| Phosphate buffer (50 mM) | | 50 mL | Solvent |
| Glycerol | | 1.5 | Plasticizer |
| Sorbitol | | 1.75 | Plasticizer |
| Xylitol | | 2.5 | Plasticizer |
| Sodium alginate (Protanal 5/60) | | 6.65 | Film-Forming Polymer |

In both cases, partial oil release was observed in the film during the drying process. Therefore, it was concluded that buffer addition in presence of salt has some stabilizing effect on emulsion droplets in the drying process.

### (c) Effect of additional cosolvent

Another strategy employed to optimize the physical stability of 10 mg CBD films was to use additional cosolvent in the formulation. In this regard, DMSO, ethanol or PEG400 were employed in the formulation at higher dose strength (see Table 10 below).

In all cases, oil release was observed in the film during the drying process. Therefore, it was concluded that cosolvent addition in the formulation has little stabilizing effect on emulsion droplets in the film.

**Table 10. Batch formula for 10 mg CBD films containing different types of cosolvent. The batch size is about 250 doses (dose dimension 3 cm²)**

| **Ingredient** | **Concentration (w/v)** | **Amount (g)** | **Function** |
|---|---|---|---|
| CBD | | 2.5 | API |
| Ethanol | 2% | 1 | Additional cosolvent |
| DMSO | 2% | 1 | Additional cosolvent |
| PEG400 | 2% | 1 | Additional cosolvent |
| Capryol PGMC | 3% | 1.5 | Oil phase |
| Cremophor EL | 5% | 2.5 | Surfactant |
| Transcutol | 5% | 2.5 | Cosolvent |
| Water | | 50 mL | Solvent |
| Glycerol | | 1.5 | Plasticizer |
| Sorbitol | | 1.75 | Plasticizer |
| Xylitol | | 2.5 | Plasticizer |
| Sodium alginate (Protanal 5/60) | | 6.65 | Film-Forming Polymer |

### (d) Effect of additional surfactant

Using a mix of surfactants to form the microemulsion was also trialled. For this purpose, labrasol (HLB value 12), a nonionic water-dispersible surfactant, was added in the lipid formulation along with an increased concentration of Cremophor EL, as shown in Table 11.

It was found that in this formulation, oil was not released in the CBD film at 10 mg dose strength, while drying the film. A white, non-greasy film was obtained that was brittle in nature due to higher concentration of surfactants used in the formulation. This experiment suggests that mixed surfactant approach displays an effective stabilizing effect on emulsion droplets in the drying process, thus preventing oil release in the film and enabling production of a more optimal film containing a higher dose strength of CBD.

**Table 11. Batch formula for 10mg CBD films with additional surfactant. The batch size is about 250 doses (dose dimension 3 cm²)**

| **Ingredient** | **Concentration (w/v)** | **Amount (g)** | **Function** |
|---|---|---|---|
| CBD | | 2.5 | API |
| Capryol PGMC | 3% | 1.5 | Oil phase |
| Cremophor EL | 10% | 2.5 | Surfactant |
| Transcutol | 5% | 2.5 | Cosolvent |
| Labrasol | 5% | 0.25 | Additional surfactant |
| Water | | 50 mL | Solvent |
| Glycerol | | 1.5 | Plasticizer |
| Sorbitol | | 1.75 | Plasticizer |
| Sodium alginate (Protanal 5/60) | | 6.65 | Film-Forming Polymer |

### (e) Effect of plasticizer

It was further postulated that addition of an additional plasticizer, xylitol, might lead to an improvement in the pliability of the higher CBD dose strength films. The formulation developed is set out in Table 12 below.

It was found that the addition of xylitol causes partial oil release in the film in the drying process but that this is a temporary effect. Without wishing to be bound by any particular theory, it is believed that xylitol, being crystalline in nature, acts as an internal plasticizer in the formulation that interferes with the structural arrangement of the droplets (e.g. through hydrogen bonding), thus increasing the stability of emulsion droplets in the film.

A similar improvement in pliability could also be observed by increasing the concentration of the external plasticizers (such as glycerol and sorbitol) in the formulation. Concomitantly, the alginate concentration in the formulation was increased to retain an optimal tensile strength. The corresponding water content was also increased to maintain an optimal viscosity of the cast. The resulting formulation is described in Table 13.

**Table 12. Batch formula for 10 mg CBD films with xylitol as plasticizer. The batch size is about 250 doses (dose dimension 3 cm²)**

| **Ingredient** | **Concentration (w/v)** | **Amount (g)** | **Function** |
|---|---|---|---|
| CBD | | 2.5 | API |
| Capryol PGMC | 3% | 1.5 | Oil phase |
| Cremophor EL | 10% | 2.5 | Surfactant |
| Transcutol | 5% | 2.5 | Cosolvent |
| Labrasol | 5% | 0.25 | stabilizer |
| Water | | 50 mL | Solvent |
| Glycerol | | 1.5 | Plasticizer |
| Sorbitol | | 1.75 | Plasticizer |
| Xylitol | | 2.5 | Plasticizer |
| Sodium alginate (Protanal 5/60) | | 6.65 | Film-Forming Polymer |

**Table 13. Batch formula for 10 mg CBD films with improved pliability and good tensile strength. The batch size is about 250 doses (dose dimension 3 cm²)**

| **Ingredient** | **Concentration (w/v)** | **Amount (g)** | **Function** |
|---|---|---|---|
| CBD | | 2.5 | API |
| Capryol PGMC | 4% | 2 | Oil phase |
| Cremophor EL | 10% | 2.5 | Surfactant |
| Transcutol | 5% | 2.5 | Cosolvent |
| Labrasol | 5% | 0.25 | Stabilizer |
| Water | | 85 mL | Solvent |
| Glycerol | | 6.4 | Plasticizer |
| Sorbitol | | 2.9 | Plasticizer |
| Sodium alginate (Protanal 5/60) | | 10.8 | Film-Forming Polymer |

### (f) Effect of drying temperature

The effect of drying temperature was also investigated. Two different temperatures, 40 °C and 60 °C, were used to dry the emulsion-based CBD films. It was found that partial oil leakage occurs at the sides of the film when drying the film at 60 °C but that is a temporary effect. However, no such oil leakage was observed when drying the same formulation at 40 °C. However, other practical factors such as the fact that the film is thinner at the sides than in the middle, as well as a relatively higher temperature on the sides of the film compared to the middle part may also play a role. Without wishing to be bound by any particular theory, it is thought that a higher drying temperature may cause a greater effect on the structural rearrangement of the droplets during the drying process, resulting in partial oil release on the side of the films.

### Properties of non-emulsion-based CBD films

A non-emulsion-based CBD formulation was considered to retain the desirable physical properties of the film (e.g. pliability, tensile strength) as well as to enable a high dose loading of the CBD active agent. DMSO, PEG400 and transcutol were all tested as solubilizing vehicles to formulate the CBD film at higher dose strength (see Table 14 below).

**Table 14. Batch formula for 5mg CBD film with different vehicles used as CBD solubilizers.**

| **Ingredient** | **Concentration (w/v)** | **Amount (g)** | **Function** |
|---|---|---|---|
| CBD | | 2.5 | API |
| DMSO | 5% | 2.5 | Solubilizer |
| PEG400 | 5% | 2.5 | Solubilizer |
| Transcutol | 5% | 2.5 | Solubilizer |
| Water | | 50 mL | Solvent |
| Glycerol | | 1.5 | Plasticizer |
| Sorbitol | | 1.75 | Plasticizer |
| Xylitol | | 2.5 | Plasticizer |
| Sodium alginate (Protanal 5/60) | | 6.65 | Film-Forming Polymer |

CBD was completely dissolved in the vehicle before mixing it with an aqueous solution comprising the plasticizers. As CBD is a hydrophobic drug, drug precipitation was observed as soon as CBD solution was mixed with water.

Subsequently, triacetin was tested as an alternative solubilizer (see Table 15).

**Table 15. Batch formula for 10 mg CBD non-emulsion-based films. The batch size is about 250 doses (dose dimension 3 cm²).**

| **Ingredient** | **Concentration (w/v)** | **Amount (g)** | **Function** |
|---|---|---|---|
| CBD | | 3.65 | API |
| Triacetin | 8.5% | 4.3 | Oil as solubilizer |
| Water | | 50 mL | Solvent |
| Glycerol | | 1.5 | Plasticizer |
| Sorbitol | | 1.75 | Plasticizer |
| Xylitol | | 2.5 | Plasticizer |
| Sodium alginate (Protanal 5/60) | 20% more | 7.99 | Film-Forming Polymer |

CBD was added to triacetin and mixed until a clear solution was obtained. This CBD solution was mixed with an aqueous solution containing the plasticizers using a high shear mixer (Ultraturrax T25, IKA Germany). Alginate was immediately added in the above precast solution and mixed until a homogeneous, viscous, whitish cast is achieved. 20% more alginate was used in this formulation than previously, to prevent the final films from being greasy. The cast was immediately coated on glass plate to avoid any coalescence of oil droplets that might result in phase separation. Drying of this film was then performed at 60 °C.

The resultant CBD films were examined under optical microscopy, which confirmed that no crystals were present in the fresh 10 mg CBD film as well as a film exposed to normal air at room temperature for 5 days. In addition, this non-emulsion-based 10 mg CBD film formulation was found to be crystal free for at least 10 weeks stored in packaging at room temperature (also via optical microscopy).

Film weight, dose and homogeneity data are presented in Table 16 below. Acceptable dose variation as well as good homogeneity (µg CBD/mg film) among the films within the batch were observed.

**Table 16. Weight, dose and homogeneity data for physically stable 10mg non-emulsion-based formulation containing triacetin as solubilizer, coated at 1.2 mm film thickness.**

| ***Non-emulsion-based CBD, formulation (10mg*/*dose) (#Batch 24)*** | |
|---|---|
| *Average weight (g)* | 9.93 |
| *Standard deviation* | 0.2 |
| *RSD%* | 0.27 |
| *CBD (µg*/*mg of film)* | 121.71 |
| *Number of films analysed* | 3 |

### Conclusions

From the above studies, it has been found that it is possible to formulate alginate-based cannabidiol buccal films that can be emulsion-based or non-emulsion-based. In particular, it was found that:
- Lump free, homogenous viscous casts with some air bubbles could be obtained.
- The prepared films were homogenous and had a smooth and flat surface. They were pliable and flexible and easy to handle and considered as being easy to handle and administer for the patient.
- Films with dose strengths up to approximately 5 mg cannabidiol/3 cm² film can be produced as an emulsion of cannabidiol formulation in the film.
- Films with dose strengths up to approximately 10 mg cannabidiol/3 cm² film can be produced as a non-emulsion-based formulation in the film using triacetin as solubilizer.
- The dose-weight variations obtained were considered acceptable for sample preparation in lab scale and the homogeneity data (µg CBD/mg film) showed good consistency within batches.
- Emulsion-based cannabidiol buccal films should preferably be dried at lower temperature, i.e. about 40 °C.
- Alginate casts that contain a cannabidiol formulation become more viscous during overnight storage. Therefore, film coating is preferably carried out with a freshly prepared cast.

It is anticipated that the conclusions drawn from this model study on cannabidiol will also be applicable to other cannabinoids (e.g. THC), due to the similarity in physical properties (and particularly solubility) between different members of the cannabinoid family.

### Example 4: Dog study using cannabidiol films

Two example CBD-containing film formulations, as prepared in Example 3 above, were given to adult beagle dogs (n=3). The first formulation (F1) was a 5 mg emulsion-based CBD alginate film, and the second formulation (F2) was a 10 mg non-emulsion-based CBD alginate film. The films were administered to each of the dogs in the study groups by placement of a single film on the buccal mucosa of the dog. As a control, the first film formulation was also placed in a gelatin capsule (F3) and administered orally to a control group of the dogs. Plasma was withdrawn from each of the two test groups of dogs, and the control group, over a time-course of from 0 to 480 minutes, and the plasma samples analysed for CBD concentration (expressed as ng CBD/mL plasma). For a comparison of absolute exposure levels, the F1 and F3 groups were dose-adjusted to 9.93 mg CBD/film, which was the final CBD concentration given to the F2 group.

Details of formulations F 1 and F2 are provided in Tables 17 and 18 below, respectively.

**Table 17. Batch formula for target 5 mg CBD emulsion-based films (actual final CBD amount per film = 3.92 mg). Batch size = 50 mL; film thickness = 1.2 mm; drying temperature = 40 °C; drying time = 2 hrs 10 mins; pH = 6.28; RH = 11.84%; CBD content = 3.92 ± 0.3 mg/dose.**

| **# B25b CBD (5 mg)(3.92 actual), 50 mL batch** | | | |
|---|---|---|---|
| **Ingredients** | **Calculated amount (g)** | **Amount taken (g)** | **Conc. (%)** |
| Caproyl PGMC | 1.5 | 1.52 | 3% w/w |
| Chremophor EL | 2.5 | 2.53 | 5% w/w |
| Transcutol | 2.5 | 2.52 | 5% w/w |
| Xylitol | 2.5 | 2.5 | - |
| Sorbitol | 1.75 | 1.76 | - |
| Glycerol | 1.5 | 1.5 | - |
| NaCl | 0.25 | 0.25 | 0.5% w/w |
| Water | 50 | 50 | - |
| Alginate (Protonal 5/60) | 6.65 | 6.65 | - |
| CBD | 1.25 | | - |

**Table 18. Batch formula for target 10 mg CBD non-emulsion-based films (actual final CBD amount per film = 9.93 mg). Batch size = 50 mL; film thickness = 1.2 mm; drying temperature = 60 °C; drying time = 1 hr; pH = 5.89; RH = 9.48%; CBD content = 9.92 ± 0.2 mg/dose.**

| **# B24 CBD (10 mg)(9.93 actual), 50 mL batch** | | | |
|---|---|---|---|
| **Ingredients** | **Calculated amount (g)** | **Amount taken (g)** | **Conc. (%)** |
| Triacetin | 4 | 4.3 | 8% w/w |
| Xylitol | 2.5 | 2.5 | - |
| Sorbitol | 1.75 | 1.76 | - |
| Glycerol | 1.5 | 1.5 | - |
| Water | 50 | 50 | - |
| Alginate (Protonal 5/60) | 7.99 (20% more) | 7.97 | - |
| CBD | 3.6 | 3.65 | - |

Dose-adjusted plasma levels over a time period of 480 minutes for each study group F1-F3 are shown in Fig. 2. A partial time-course study showing only the first 60-minute period is shown in Fig. 3. Pharmacokinetic parameters from the study are shown in Tables 19 and 20 below.

**Table 19. Summary of mean pharmacokinetic parameters from CBD formulations given to adult beagle dogs (n=3).**

| **Formulation** | **Dose** | **AUC₀₋₈ₕᵣ ng/ml*min** | **Cₘₐₓ ng/ml** | **Tₘₐₓ min** | **Conc. at 20 min.** |
|---|---|---|---|---|---|
| **F1** | 3.92 mg | 3557 | 22 | 60 | 3.82 |
| **F2** | 9.93 mg | 12597 | 83 | 80 | 18.8 |
| **F3** | 3.92 mg | 2181 | 13 | 80 | 0.86 |

**Table 20. Summary of dose-adjusted mean pharmacokinetic parameters from CBD formulations given to adult beagle dogs (n=3). Dose adjustment of F1 and F3 was made to a 9.93 mg dose (F2).**

| **Formulation** | **Dose** | **AUC₀₋₈ₕᵣ ng/ml*min** | **Cₘₐₓ ng/ml** | **Tₘₐₓ min** | **Conc. at 20 min.** |
|---|---|---|---|---|---|
| **F1** | 3.92mg | 9011 | 55 | 60 | 9.67 |
| **F2** | 9.93mg | 12597 | 83 | 80 | 18.8 |
| **F3** | 3.92mg | 5525 | 32 | 80 | 2.18 |

These studies show that both film formulations that were administered by adhesion to the buccal cavity (F1 and F2) resulted in a higher level of plasma exposure in the dogs to CBD than oral administration of the film (F3). Higher exposure levels were observed when the non-emulsion-based film (F2) was used rather than the emulsion-based film (F1), after adjustment for dose variation. Buccal administration of CBD enabled significantly higher exposure levels to CBD from an early point (20 minutes) in the time course studies.

In conclusion, buccal placement of the CBD formulations appears to provide a surprisingly higher level of plasma exposure to CBD compared with oral administration.

### References

[1] Begg, M.; Pacher, P.; Batkai, S.; Oseihyiaman, D.; Offertaler, L.; Mo, F. M.; Liu, J.; Kunos, G. Evidence for novel cannabinoid receptors. Pharmacology & Therapeutics, 2005, 106(2), 133-145.
[2] Kaminski, N. E. Inhibition of the cAMP signaling cascade via cannabinoid receptors: a putative mechanism of immune modulation by cannabinoid compounds. Toxicology Letters, 1998, 102-103, 59-63.
[3] Basu S.; Ray A.; Dittel B. N. Cannabinoid receptor 2 is critical for the homing and retention of marginal zone B lineage cells and for efficient T-independent immune responses. Journal of Immunology, 2011, 187(11), 5720-5732.
[4] Mechoulam, R.; Peters, M.; Murillo-Rodriguez, E.; Hanuš, L. O. Cannabidiol - Recent Advances. *Chemistry & Biodiversity,* **2007,** 4(8), 1678-1692.
[5] Lim, K. A Systematic Review of the Effectiveness of Medical Cannabis for Psychiatric, Movement and Neurodegenerative Disorders. Clinical Psychopharmacology and Neuroscience, 2017, 15(4), 301-312.
[6] Ben Amar, M. Cannabinoids in medicine: A review of their therapeutic potential. J Ethnopharmacol, 2006, 105(1-2), 1-25.
[7] Stout, S. M.; Cimino, N. M. Exogenous cannabinoids as substrates, inhibitors, and inducers of human drug metabolizing enzymes: a systematic review. Drug Metabolism Reviews, 2014, 46(1), 86-95.
[8] Nadel, J. A. Acute effects of inhalation of cigarette smoke on airway conductance. J Appl Physiol, 1961, 16, 713-716.
[9] Tashkin, D.P. Acute effects of smoked marijuana and oral Δ9-tetrahydrocannabinol on specific airway conductance in asthmatic subjects. Am Rev Respir Dis, 1974, 109, 420-428.
[10] http://www.mannamolecular.com/2016/09/forms-of-cannabis-intake/ (accessed 14 May 2018).
[11] Pertwee, R. G. The pharmacology of cannabinoid receptors and their ligands: an overview. International Journal of Obesity, 2006, 30, 513-518.
[12] Prachayasittikul, V.; Isarankura-Na-Ayudhya, C.; Tantimongcolwat, T.; Nantasenamat, C.; Galla, H.J. EDTA-induced Membrane Fluidization and Destabilization: Biophysical Studies on Artificial Lipid Membranes. Acta biochimica et biophysica Sinica, 2007, 39(11), 901-913.
[13] Managaro, A.; Wertz, P. The effect of permeabilizer on the in vitro penetration of propranolol through porcine buccal epithelium.
[14] Date, A.A.; Desai, N.; Dixit, R.; Nagarsenker, M. Self-nanoemulsifying Drug Delivery Systems: Formulation Insights, Applications and Advances. Nanomedicine, 2010, 5(10), 1595-1616.
[15] Pouton, C.W. Formation of poorly water-soluble drugs for oral administration: Physicochemical and physiological issues and the lipid formulation classification system. European Journal of Pharmaceutical Sciences, 2006, 29(3-4), 278-287.
[16] Alzheimer's Association. 10 Early Signs and Symptoms of Alzheimer's, 2018. https://www.alz.org/10-signs-symptoms-alzheimers-dementia.asp (accessed 6 March 2018).
[17] Chey, W. D. "Irritable Bowel Syndrome: A Clinical Review." JAMA, 2015, 313(9), 949-958.
[18] NIDDK. Definition & Facts for Crohn's Disease, 2017 https://www.niddk.nih.gov/health-information/digestive-diseases/crohns-disease/definition-facts (accessed 27 February 2018).
[19] Nicholson, A. Ulcerative Colitis Statistics, 2016. https://crohnsdisease.com/ulcerative-colitis/ulcerative-colitis-statistics/ (accessed 28 February 2018).
[20] http://www.cannabi sconsumer.org/uploads/9/7 /9/6/97962014/market size and_ demand study_july _9 2014%5B1%5D.pdf (accessed 26 March 2018).
[21] Bouhassira, D.; Lantéri-Minet, M.; Attal, N.; Laurent, B.; Touboul, C. Prevalence of chronic pain with neuropathic characteristics in the general population. Pain, 2008, 136(3), 380-387.
[22] Zgair, A.; Wong, J. C. M.; Sabri, A.; Fischer, P. M.; Barrett, D. A.; Constantinescu, C. S.; Gershkovich, P. Development of a simple and senistive HPLC-UV method for the simultaneous determination of cannabidiol and Δ9-tetrahydrocannibinol in rat plasma. Journal of Biopharmaceutical and Biomedical Analysis, 2015, 114, 145-151.

## Claims

1. A film suitable for administration to an oral cavity comprising:
(i) an alginate salt of a monovalent cation or a mixture of alginate salts containing at least one alginate salt of a monovalent cation; and
(ii) an active pharmaceutical ingredient (API) which is one or more cannabinoids or pharmaceutically acceptable salts thereof;
wherein the alginate salt of a monovalent cation comprises from 25 to 35% by weight of β-D-mannuronate and from 65 to 75% by weight of α-L-guluronate.

2. The film according to claim 1, wherein each cannabinoid present in the film is:
(a) an agonist, inverse agonist or antagonist of the CB₁ receptor; and/or
(b) an agonist, inverse agonist or antagonist of the CB₂ receptor.

3. The film according to claim 1 or claim 2, wherein each cannabinoid present in the film is selected from the group consisting of cannabigerolic acid A, cannabigerolic acid A monomethyl ether, cannabigerol, cannabigerol monomethyl ether, cannabigerovarinic acid A, cannabigerovarin, cannabinerolic acid A, (±)-cannabichromenic acid, (±)-cannabichromene, (±)-cannabichromevarinic acid, (±)-cannabivarichromene, (+)-cannabichromevarin, 2-methyl-2-(4-methyl-2-pentenyl)-7-propyl-2*H*-1-benzopyran-5-ol, cannabidiolic acid, (-)-cannabidiol (CBD), cannabidiol monomethyl ether, cannabidiol-C₄, cannabidivarinic acid, (-)-cannabidivarin, cannabidiorcol, tetrahydrocannabinolic acid A, tetrahydrocannabinolic acid B, tetrahydrocannabinol (Δ⁹-THC), tetrahydrocannabinolic acid-C₄, tetrahydrocannabinol-C₄, tetrahydrocannabivarinic acid A, tetrahydrocannabivarin, tetrahydrocannabiorcolic acid, tetrahydrocannabiorcol, (-)-Δ⁸-*trans*-(6a*R,*10a*R*)-tetrahydrocannabinolic acid A, (-)-Δ⁸-*trans*-(6a*R,*10a*R*)-tetrahydrocannabinol, (±)-(1a*S*,3a*R*,8b*R*,8c*R*)-cannabicyclolic acid, (±)-(1a*S*,3a*R*,8b*R*,8c*R*)-cannabicyclol, (±)-(1a*S*,3a*R*,8b*R*,8c*R*)-cannabicyclovarin, (5a*S*,6*S*,9*R*,9a*R*)-cannabielsoic acid A, (5a*S*,6*S*,9*R*,9a*R*)-cannabielsoic acid B, (5a*S*,6*S*,9*R*,9a*R*)-C₃-cannabielsoic acid B, (5a*S*,6*S*,9*R*,9a*R*)-cannabielsoin, (5a*S*,6*S*,9*R*,9a*R*)-C₃-cannabielsoin, cannabinolic acid A, cannabinol, cannabinol methyl ether, cannabinol-C₄, cannabivarin, cannabinol-C₂, cannabiorcol-C₁, cannabinodiol, cannabinodivarin, (-)-*trans*-cannabitriol, (+)*-trans-*cannabitriol, (±)-*cis*-cannabitriol, (±)-*trans*-cannabitriol-C₃, (-)-*trans*-10-ethoxy-9-hydroxy-Δ^{6a(10a)}-tetrahydrocannabinol, *trans*-10-ethoxy-9-hydroxy-Δ^{6a(10a)}-tetrahydrocannabivarin-C₃, 8,9-dihydroxy-Δ^{6a(10a)}-tetrahydrocannabinol, cannabidiolic acid tetrahydrocannabitriol ester, dehydrocannabifuran, cannabifuran, cannabichromanone, cannabichromanone-C₃, cannabicoumarinone-Cs, cannabicitran, 10-oxo- Δ^{6a(10a)}-tetrahydrocannabinol, (-)- Δ⁹-(6a*S*,10a*R*-cis)-tetrahydrocannabinol, cannabiglendol-C₃, (-)-(6a*R*,9*S*, 10*S,* 10a*R*)-9, 10-dihydroxyhexahydrocannabinol, (-)-6a,7,10a-Trihydroxy-Δ⁹-tetrahydrocannabinol, (±)-Δ⁷-*cis*-(1*R*,3*R*,6*S*)-isotetrahydrocannabivarin-C3, (-)-Δ⁷-*trans*-(1*R*,3*R*,6*R*)-isotetrahydrocannabivarin-C₃, (-)-Δ⁷-*trans*-(1*R*,3*R*,6*R*)-isotetrahydrocannabinol-C₅, anandamide, 2-arachidonoylglycerol, 2-arachidonyl glyceryl ether, *N*-arachidonoyl dopamine, virodhamine, lysophosphatidylinositol, nabilone, rimonabant, dimethylheptylpyran, levonantradol, ajulemic acid,

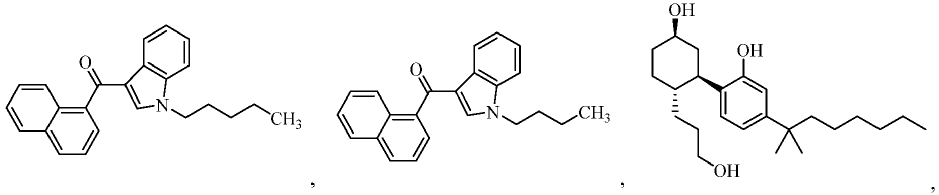

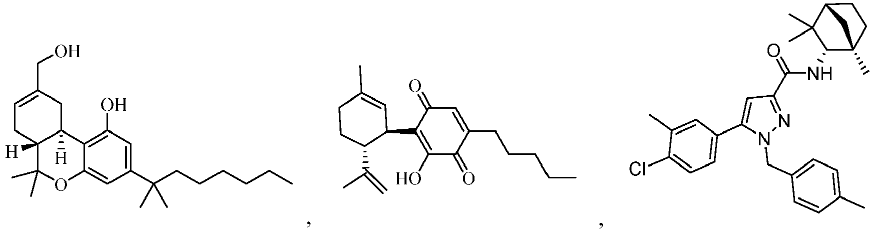

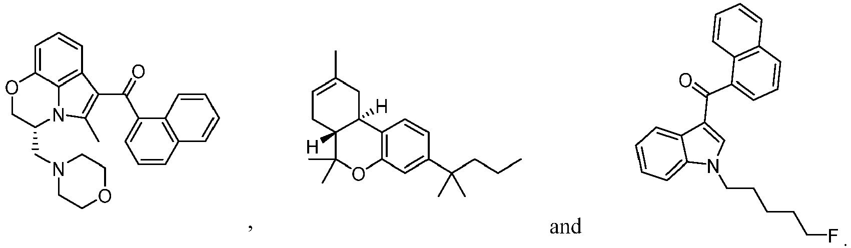

4. The film according to any one of claims 1 to 3, wherein the API is Δ⁹-tetrahydrocannabinol, cannabidiol, or a mixture thereof.

5. The film according to any one of claims 1 to 4, wherein the alginate salt of a monovalent cation is selected from a sodium alginate, a potassium alginate and an ammonium alginate, and is preferably a sodium alginate.

6. The film according to any one of claims 1 to 5, wherein the film comprises from 25% to 99% by weight of the alginate salt of a monovalent cation or the mixture of alginate salts containing at least one alginate salt of a monovalent cation, from 0% to 20% by weight of water, and from 0.001% to 75% by weight of the API.

7. The film according to any one of claims 1 to 6, wherein the film comprises from 29% to 93% by weight of the alginate salt of a monovalent cation or the mixture of alginate salts containing at least one alginate salt of a monovalent cation, from 5% to 15% by weight of water, and from 0.15% to 50% by weight of the API.

8. The film according to any one of claims 1 to 7, wherein the film further comprises at least one plasticizer which is selected from sorbitol, glycerol, and a combination thereof, preferably both sorbitol and glycerol, and:
(a) a basifying agent which is optionally aqueous sodium hydroxide; or
(b) a SEDDS comprising (i) an oil phase, (ii) at least one surfactant, preferably at least two surfactants, and (iii) a solubilizer; or
(c) a non-aqueous pharmaceutically acceptable solvent, preferably triacetin.

9. The film according to claim 8, wherein the film further comprises from 0% to 40% by weight of sorbitol, and from 0% to 40% by weight of glycerol.

10. The film according to claim 8 or claim 9, wherein the film further comprises xylitol.

11. A film according to any one of claims 1 to 10 for use in the treatment of a human patient.

12. A film according to any one of claims 1 to 10 for use in the treatment of a disease or condition in a human patient, wherein the disease or condition is selected from:
cancer; dementia; Alzheimer's disease; amyotrophic lateral sclerosis; dystonia;
epilepsy; Huntington's disease; multiple sclerosis; Parkinson's disease; spasticity;
Tourette's syndrome; irritable bowel disease (IBD); Crohn's disease; ulcerative colitis; anorexia; cachexia; cancer-induced nausea and/or vomiting; cancer-induced cachexia; glaucoma; chronic pain; cancer-induced pain; fibromyalgia; neuropathic pain; addiction; anxiety; bipolar disorder; post-traumatic stress disorder; psychosis;
schizophrenia; scleroderma; and type I diabetes, preferably wherein the disease or
condition is selected from: dementia; Alzheimer's disease; epilepsy; inflammatory bowel disease; Crohn's disease; ulcerative colitis; chronic pain; cancer-induced pain;
fibromyalgia; and neuropathic pain.

13. A film for use according to claim 11 or claim 12, wherein the film is administered to the oral cavity of the human patient.

14. A method of manufacturing a film according to any one of claims 1 to 10, said method comprising the following steps:
(a) either the steps of:
(i) optionally, mixing at least one antioxidant in water, or in a mixed aqueous/organic solvent, or in one or more organic solvents;
(ii) mixing the API in water, or in a mixed aqueous/organic solvent, or in one or more organic solvents to which water is subsequently added, or in the solution obtained in step (i), optionally wherein the pH of the solution is adjusted either before or after the addition of the API to the desired level by addition of an appropriate acid or base, typically a diluted aqueous acid or alkali, more typically a diluted aqueous alkali, and preferably wherein the pH of the solution is adjusted to from 3.0 to 13.5;
(iii) optionally, sonicating the solution;
(iv) optionally, mixing one or more excipients, flavouring agents, buffering components, permeation enhancers, SEDDS (e.g. SMEDDS or SNEDDS), chelating agents, antioxidants, and/or antimicrobial agents into the solution obtained in step (ii) or step (iii); and
(v) adding the alginate salt of monovalent cation under suitable conditions to result in the formation of a viscous cast;
or alternatively the steps of:
(i) mixing the alginate salt of monovalent cation in water, until a lump free dispersion is achieved, and optionally adding one or more excipients, flavouring agents, buffering components, permeation enhancers, SEDDS (e.g. SMEDDS or SNEDDS), chelating agents, antioxidants and/or antimicrobial agents to the aqueous solution either before or after the addition of the alginate salt;
(ii) separately, dissolving the API in water, a mixed aqueous/organic solvent or one or more organic solvent(s), optionally wherein at least one antioxidant is pre-dissolved in the solvent, optionally wherein the pH of the solution is adjusted either before or after the addition of the API to the desired level by addition of an appropriate acid or base, typically a diluted aqueous acid or alkali, more typically a diluted aqueous alkali, and preferably wherein the pH of the solution is adjusted to from 3.0 to 13.5; and
(iii) adding the solution obtained in step (i) to the solution obtained in step (ii) under suitable conditions to result in the formation of a viscous cast;
or alternatively the steps of:
(i) mixing the API in an oil phase;
(ii) premixing a surfactant and a cosolvent, and then adding this to the solution obtained;
(iii) optionally, adding one or more excipients, flavouring agents, buffering components, permeation enhancers, chelating agents, antioxidants and/or antimicrobial agents to water in step (i) under mixing;
(iv) adding water, or the solution obtained in step (iii), to the solution obtained in step (ii) under stirring, preferably continuous stirring, and more preferably wherein the water or the solution obtained in step (iii) is added in a dropwise fashion; and
(v) mixing the alginate salt of monovalent cation in the solution, until a lump free dispersion is achieved, and optionally adding further water to modulate the viscosity of the cast formed;
or alternatively the steps of:
(i) mixing the API in a solubilizing agent;
(ii) adding the resultant solution to water, preferably under high shear mixing;
(iii) optionally, adding one or more excipients, flavouring agents, buffering components, permeation enhancers, chelating agents, antioxidants and/or antimicrobial agents; and
(iv) mixing the alginate salt of monovalent cation in the solution, until a lump free dispersion is achieved, and optionally adding further water to modulate the viscosity of the cast formed;
(b) optionally, adding one or more further excipients, flavouring agents, buffering components, permeation enhancers, SEDDS (e.g. SMEDDS or SNEDDS), chelating agents, antioxidants, and/or antimicrobial agents to the cast obtained in step (a);
(c) optionally, leaving the cast to de-aerate;
(d) pouring the cast onto a surface and spreading the cast out to the desired thickness;
(e) drying the cast layer, typically at a temperature of from 30 to 70 °C, until the residual water content of the film is from 0 to 20% by weight and a solid film is formed; and
(f) optionally, cutting the solid film into pieces of the desired size, further optionally placing these pieces into pouches, preferably wherein the pouches are made from PET-lined aluminium, sealing the pouches and further optionally, labelling them.

15. The method of claim 14, wherein after the viscous cast is poured onto a surface, it is first spread out to a thickness of 2 mm by means of an applicator with a slit height of 2 mm, and is then subsequently spread out to a thickness of 1 mm by means of an applicator with a slit height of 1 mm.

## Patentansprüche

1. Film, der zur Verabreichung in eine Mundhöhle geeignet ist, umfassend:
(i) ein Alginatsalz aus einem einwertigen Kation oder eine Mischung von Alginatsalzen, die mindestens ein Alginatsalz aus einem einwertigen Kation enthält, und
(ii) ein pharmazeutischer Wirkstoff (Active Pharmaceutical Ingredient, API), der ein oder mehrere Cannabinoide oder pharmazeutisch annehmbare Salz davon ist,
wobei das Alginatsalz aus einem einwertigen Kation 25 bis 35 Gew.-% β-D-Mannuronat und 65 bis 75 Gew.-% α-L-Guluronat umfasst.

2. Film nach Anspruch 1, wobei jedes im Film vorhandene Cannabinoid Folgendes ist:
(a) ein Agonist, inverser Agonist oder Antagonist des CB₁-Rezeptors und/oder
(b) ein Agonist, inverser Agonist oder Antagonist des CB₂-Rezeptors.

3. Film nach Anspruch 1 oder Anspruch 2, wobei jedes im Film vorhandene Cannabinoid aus der Gruppe bestehend aus Cannabigerolsäure A, Cannabigerolsäure-A-Monomethylether, Cannabigerol, Cannabigerolmonomethylether, Cannabigerovarinsäure A, Cannabigerovarin, Cannabinerolsäure A, (±)-Cannabichromensäure, (±)-Cannabichromen, (±)-Cannabichromevarinsäure, (±)-Cannabivarichromen, (+)-Cannabichromevarin, 2-Methyl-2-(4-methyl-2-pentenyl)-7-propyl-2*H*-1-benzopyran-5-ol, Cannabidiolsäure, (-)-Cannabidiol (CBD), Cannabidiolmonomethylether, Cannabidiol-C₄, Cannabidivarinsäure, (-)-Cannabidivarin, Cannabidiorcol, Tetrahydrocannabinolsäure A, Tetrahydrocannabinolsäure B, Tetrahydrocannabinol (Δ⁹-THC), Tetrahydrocannabinolsäure-C₄, Tetrahydrocannabinol-C₄, Tetrahydrocannabivarinsäure A, Tetrahydrocannabivarin, Tetrahydrocannabiorcolsäure, Tetrahydrocannabiorcol, (-)-Δ⁸-*trans*-(6a*R,*10a*R*)-Tetrahydrocannabinolsäure A, (-)-Δ⁸-*trans-*(6a*R*,10a*R*)-Tetrahydrocannabinol, (±)-(1a*S*',3a*R*,8b*R*,8c*R*)-Cannabicyclolsäure, (±)-(1a*S*,3a*R*,8b*R*,8c*R*)-Cannabicyclol, (±)-(1a*S*,3a*R*,8b*R*,8c*R*)-Cannabicyclovarin, (5a*S*,6*S*,9*R*,9a*R*)-Cannabielsoinsäure A, (5a*S*,6*S*,9*R*,9a*R*)-Cannabielsoinsäure B, (5a*S*,6*S*,9*R*,9a*R*)-C₃-Cannabielsoinsäure B, (Sa*S*,6*S*,9*R*,9a*R*)-Cannabielsoin, (5a*S*,6S,9R,9aR)-C₃-Cannabielsoin, Cannabinolsäure A, Cannabinol, Cannabinolmethylether, Cannabinol-C₄, Cannabivarin, Cannabinol-C₂, Cannabiorcol-C₁, Cannabinodiol, Cannabinodivarin, (-)-*trans-*Cannabitriol, (+)-*trans*-Cannabitriol, (±)-*cis*-Cannabitriol, (±)-*trans*-Cannabitriol-C₃, (-)-*trans-*10-Ethoxy-9-hydroxy-A^{6a(10a)}-tetrahydrocannabinol, *trans-10-Ethoxy-9-hydroxy-*Δ^{6a(10a)}-tetrahydrocannabivarin-C₃, 8,9-Dihydroxy-Δ^{6a(10a)}-tetrahydrocannabinol, Cannabidiolsäuretetrahydrocannabitriolester, Dehydrocannabifuran, Cannabifuran, Cannabichromanon, Cannabichromanon-C₃, Cannabicoumarinon-Cs, Cannabicitran, 10-Oxo-Δ^{6a(10a)}-tetrahydrocannabinol, (-)-Δ⁹-(6a*S*,10a*R*-*cis*)-Tetrahydrocannabinol, Cannabiglendol-C₃, (-)-(6a*R*,9*S*,10*S*,10a*R*)-9,10-Dihydroxyhexahydrocannabinol, (-)-6a,7, 10a-Trihydroxy-Δ⁹-tetrahydrocannabinol, (±)-Δ⁷-*cis*-(1*R*,3*R*,6*S*)-Isotetrahydrocannabivarin-C3, (-)-Δ⁷*-trans-*(1*R*,3*R*,6*R*)-Isotetrahydrocannabivarin-C₃, (-)-Δ⁷-*trans*-(1*R*,3*R*,6*R*)-Isotetrahydrocannabinol-C₅, Anandamid, 2-Arachidonoylglycerol, 2-Arachidonylglycerylether, *N-*Arachidonoyldopamin, Virodhamin, Lysophosphatidylinositol, Nabilon, Rimonabant, Dimethylheptylpyran, Levonantradol, ajulemischer Säure, 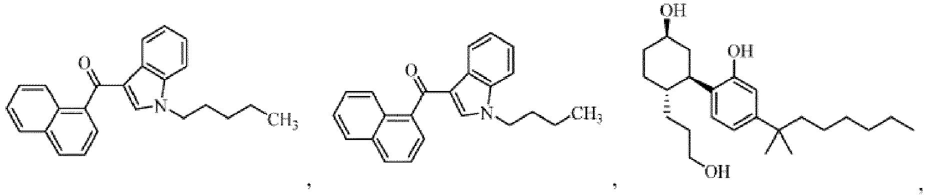 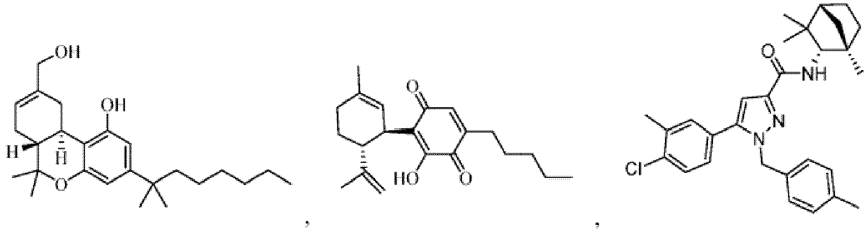 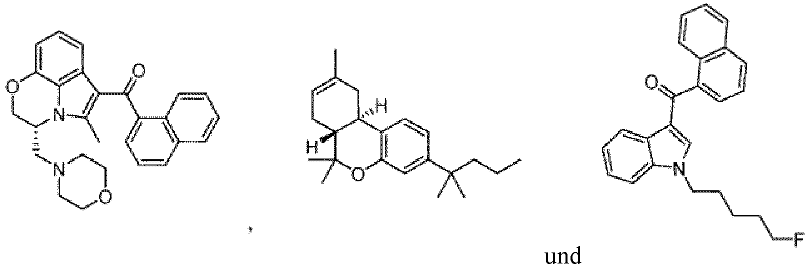 ausgewählt ist.

4. Film nach einem der Ansprüche 1 bis 3, wobei der API Δ⁹-Tetrahydrocannabinol, Cannabidiol oder eine Mischung davon ist.

5. Film nach einem der Ansprüche 1 bis 4, wobei das Alginatsalz aus einem einwertigen Kation aus einem Natriumalginat, einem Kaliumalginat und einem Ammoniumalginat ausgewählt ist und vorzugsweise ein Natriumalginat ist.

6. Film nach einem der Ansprüche 1 bis 5, wobei der Film 25 Gew.-% bis 99 Gew.-% des Alginatsalzes aus einem einwertigen Kation oder der Mischung von Alginatsalzen, die mindestens ein Alginatsalz aus einem einwertigen Kation enthält, 0 Gew.-% bis 20 Gew.-% Wasser und 0,001 Gew.-% bis 75 Gew.-% des API umfasst.

7. Film nach einem der Ansprüche 1 bis 6, wobei der Film 29 Gew.-% bis 93 Gew.-% des Alginatsalzes aus einem einwertigen Kation oder der Mischung von Alginatsalzen, die mindestens ein Alginatsalz aus einem einwertigen Kation enthält, 5 Gew.-% bis 15 Gew.-% Wasser und 0,15 Gew.-% bis 50 Gew.-% des API umfasst.

8. Film nach einem der Ansprüche 1 bis 7, wobei der Film ferner mindestens einen Weichmacher umfasst, der aus Sorbitol, Glycerol und einer Kombination davon, vorzugsweise sowohl Sorbitol als auch Glycerol, ausgewählt ist, und:
(a) ein Alkalisierungsmittel, das optional wässriges Natriumhydroxid ist, oder
(b) ein SEDDS, das (i) eine Ölphase, (ii) mindestens ein Tensid, vorzugsweise mindestens zwei Tenside, und (iii) einen Lösungsvermittler umfasst, oder
(c) ein nicht wässriges pharmazeutisch annehmbares Lösungsmittel, vorzugsweise Triacetin.

9. Film nach Anspruch 8, wobei der Film ferner 0 Gew.-% bis 40 Gew.-% Sorbitol und 0 Gew.-% bis 40 Gew.-% Glycerol umfasst.

10. Film nach Anspruch 8 oder Anspruch 9, wobei der Film ferner Xylitol umfasst.

11. Film nach einem der Ansprüche 1 bis 10 zur Verwendung bei der Behandlung eines menschlichen Patienten.

12. Film nach einem der Ansprüche 1 bis 10 zur Verwendung bei der Behandlung einer Erkrankung oder eines Zustands in einem menschlichen Patienten, wobei die Erkrankung oder der Zustand aus Folgenden ausgewählt ist: Krebs, Demenz, Alzheimer-Krankheit, amyotropher Lateralsklerose, Dystonie, Epilepsie, Huntington-Krankheit, multipler Sklerose, Parkinson-Krankheit, Spastik, Tourette-Syndrom, Reizdarmerkrankung (Irritable Bowel Disease, IBD), Morbus Crohn, Colitis ulcerosa, Anorexie, Kachexie, krebsinduzierter Übelkeit und/oder krebsinduziertem Erbrechen, krebsinduzierter Kachexie, Glaukom, chronischem Schmerz, krebsinduziertem Schmerz, Fibromyalgie, neuropathischem Schmerz, Sucht, Angst, bipolarer Störung, posttraumatischer Belastungsstörung, Psychose, Schizophrenie, Sklerodermie und Diabetes Typ 1, vorzugsweise wobei die Erkrankung oder der Zustand aus Folgenden ausgewählt ist: Demenz, Alzheimer-Krankheit, Epilepsie, entzündlicher Darmerkrankung, Morbus Crohn, Colitis ulcerosa, chronischem Schmerz, krebsinduziertem Schmerz, Fibromyalgie und neuropathischem Schmerz.

13. Film zur Verwendung nach Anspruch 11 oder Anspruch 12, wobei der Film einer Mundhöhle des menschlichen Patienten verabreicht wird.

14. Verfahren zur Herstellung eines Films nach einem der Ansprüche 1 bis 10, wobei das Verfahren die folgenden Schritte umfasst:
(a) entweder die folgenden Schritte:
(i) optionales Mischen von mindestens einem Antioxidans in Wasser oder in einem gemischten wässrigen/organischen Lösungsmittel oder in einem oder mehreren organischen Lösungsmitteln,
(ii) Mischen des API in Wasser oder in einem gemischten wässrigen/organischen Lösungsmittel oder in einem oder mehreren organischen Lösungsmitteln, zu denen anschließend Wasser zugegeben wird, oder in der in Schritt (i) erhaltenen Lösung, optional wobei der pH der Lösung entweder vor oder nach der Zugabe des API auf den gewünschten Wert durch Zugabe einer angemessenen Säure oder Base, typischerweise einer verdünnten wässrigen Säure oder eines verdünnten wässrigen Alkalis, typischer eines verdünnten wässrigen Alkalis, angepasst wird und vorzugsweise wobei der pH der Lösung auf 3,0 bis 13,5 angepasst wird,
(iii) optionales Beschallen der Lösung,
(iv) optionales Mischen von einem oder mehreren Hilfsstoffen, Aromastoffen, Pufferkomponenten, Permeationsverstärkern, SEDDS (z. B. SMEDDS oder SNEDDS), Chelatbildnern, Antioxidantien und/oder antimikrobiellen Mitteln in die in Schritt (ii) oder Schritt (iii) erhaltene Lösung und
(v) Zugeben des Alginatsalzes aus einwertigem Kation unter geeigneten Bedingungen, um zur Bildung eines viskosen Gusses zu führen,
oder alternativ die folgenden Schritte:
(i) Mischen des Alginatsalzes aus einwertigem Kation in Wasser, bis eine klumpenfreie Dispersion erreicht ist, und optionales Zugeben von einem oder mehreren Hilfsstoffen, Aromastoffen, Pufferkomponenten, Permeationsverstärkern, SEDDS (z. B. SMEDDS oder SNEDDS), Chelatbildnern, Antioxidantien und/oder antimikrobiellen Mitteln zur wässrigen Lösung entweder vor oder nach der Zugabe des Alginatsalzes,
(ii) separates Auflösen des API in Wasser, einem gemischten wässrigen/organischen Lösungsmittel oder einem oder mehreren organischen Lösungsmitteln, optional wobei mindestens ein Antioxidans im Lösungsmittel vorgelöst ist, optional wobei der pH der Lösung entweder vor oder nach der Zugabe des API auf den gewünschten Wert durch Zugabe einer angemessenen Säure oder Base, typischerweise einer verdünnten wässrigen Säure oder eines verdünnten wässrigen Alkalis, typischer eines verdünnten wässrigen Alkalis, angepasst wird und vorzugsweise wobei der pH der Lösung auf 3,0 bis 13,5 angepasst wird, und
(iii) Zugeben der in Schritt (i) erhaltenen Lösung zur in Schritt (ii) erhaltenen Lösung unter geeigneten Bedingungen, um zur Bildung eines viskosen Gusses zu führen,
oder alternativ die folgenden Schritte:
(i) Mischen des API in einer Ölphase,
(ii) Vormischen eines Tensids und eines Co-Lösungsmittels und dann Zugeben dieser zur erhaltenen Lösung,
(iii) optionales Zugeben von einem oder mehreren Hilfsstoffen, Aromastoffen, Pufferkomponenten, Permeationsverstärkern, Chelatbildnern, Antioxidantien und/oder antimikrobiellen Mitteln zu Wasser in Schritt (i) unter Mischen,
(iv) Zugeben von Wasser oder der in Schritt (iii) erhaltenen Lösung zur in Schritt (ii) erhaltenen Lösung unter Rühren, vorzugsweise kontinuierlichem Rühren, und besonders bevorzugt wobei das Wasser oder die in Schritt (iii) erhaltene Lösung tropfenweise zugegeben wird, und
(v) Mischen des Alginatsalzes aus einwertigem Kation in der Lösung, bis eine klumpenfreie Dispersion erreicht ist, und optionales Zugeben von mehr Wasser, um die Viskosität des gebildeten Gusses zu modulieren,
oder alternativ die folgenden Schritte:
(i) Mischen des API in einem Solubilisierungsmittel,
(ii) Zugeben der resultierenden Lösung zu Wasser, vorzugsweise unter Mischen mit hoher Scherkraft,
(iii) optionales Zugeben von einem oder mehreren Hilfsstoffen, Aromastoffen, Pufferkomponenten, Permeationsverstärkern, Chelatbildnern, Antioxidantien und/oder antimikrobiellen Mitteln und
(iv) Mischen des Alginatsalzes aus einwertigem Kation in der Lösung, bis eine klumpenfreie Dispersion erreicht wird, und optionales Zugeben von mehr Wasser, um die Viskosität des gebildeten Gusses zu modulieren,
(b) optionales Zugeben von einem oder mehreren weiteren Hilfsstoffen, Aromastoffen, Pufferkomponenten, Permeationsverstärkern, SEDDS (z. B. SMEDDS oder SNEDDS), Chelatbildnern, Antioxidantien und/oder antimikrobiellen Mitteln zum in Schritt (a) erhaltenen Guss,
(c) optionales Entlüftenlassen des Gusses,
(d) Gießen des Gusses auf eine Oberfläche und Verteilen des Gusses in der gewünschten Dicke,
(e) Trocknen der Gussschicht, typischerweise bei einer Temperatur von 30 bis 70 °C, bis der Restwassergehalt des Films 0 bis 20 Gew.-% beträgt und ein fester Film gebildet ist, und
(f) optionales Schneiden des festen Films in Stücke der gewünschten Größe, ferner optionales Platzieren dieser Stücke in Beuteln, vorzugsweise wobei die Beutel aus mit PET ausgekleidetem Aluminium bestehen, Versiegeln der Beutel und ferner optionales Kennzeichnen dieser.

15. Verfahren nach Anspruch 14, wobei, nachdem der viskose Guss auf eine Oberfläche gegossen wurde, er zuerst in einer Dicke von 2 mm mittels eines Applikators mit einer Schlitzhöhe von 2 mm verteilt wird und dann anschließend in einer Dicke von 1 mm mittels eines Applikators mit einer Schlitzhöhe von 1 mm verteilt wird.

## Revendications

1. Film convenant à l'administration dans une cavité buccale comprenant :
(i) un sel d'alginate d'un cation monovalent ou un mélange de sels d'alginate contenant au moins un sel d'alginate d'un cation monovalent ; et
(ii) un ingrédient pharmaceutique actif (API) qui est un ou plusieurs cannabinoïdes ou leurs sels pharmaceutiquement acceptables ;
dans lequel le sel d'alginate d'un cation monovalent comprend de 25 à 35 % en poids de β-D- mannuronate et de 65 à 75 % en poids d'α-L- guluronate.

2. Film selon la revendication 1, dans lequel chaque cannabinoïde présent dans le film est :
(a) un agoniste, agoniste inverse ou antagoniste du récepteur CB₁ ; et/ou
(b) un agoniste, agoniste inverse ou antagoniste du récepteur CB₂.

3. Film selon la revendication 1 ou la revendication 2, dans lequel chaque cannabinoïde présent dans le film est choisi dans le groupe constitué par l'acide cannabigérolique A, l'acide cannabigérolique A monométhyl éther, cannabigérol , cannabigérol monométhyl éther, l'acide cannabigérovarinique A, cannabigérovarine, l'acide cannabinérolique A, l'acide (±)- cannabichroménique , (±)-cannabichromène , (±)-acide cannabichromevarinique , (±)-cannabivarichromène , (+)-cannabichromevarine , 2-méthyl-2 -(4-méthyl-2-pentényl)-7-propyl-2-*H*-benzopyran-5-ol, acide cannabidiolique , (-)- cannabidiol (CBD), cannabidiol monométhyl éther, cannabidiol-C₄, acide cannabidivarinique, (-)- cannabidivarine, cannabidiorcol, l'acide tétrahydrocannabinolique A, l'acide tétrahydrocannabinolique B, tétrahydrocannabinol (Δ⁹-THC), l'acide tétrahydrocannabinolique-C₄, tétrahydrocannabinol-C₄, l'acide tétrahydrocannabivarinique A, tétrahydrocannabivarine, l'acide tétrahydrocannabiorcolique, tétrahydrocannabiorcol, (-)-Δ⁸-*trans*-(6a*R*,10aR)-acide tétrahydrocannabinolique A, (-)-Δ⁸-*trans*-(6a*R*,10a*R*)- tétrahydrocannabinol, (±)-(1aS,3aR,8bR,8cR)-acide cannabicyclolique, (±)-(1a*S*,3a*R*,8b*R*,8c*R*)-cannabicyclol, (±)-(1aS,3aR,8bR,8cR)- cannabicyclovarine, (5aS,6S,9R,9aR)-acide cannabielsoïque A, (5a*S*,6*S*,9*R*,9a*R*)-acide cannabielsoïque B, (5aS,6S,9R,9aR)-C3-acide cannabielsoïque B, (5aS,6S,9R,9aR)-cannabielsoïne , (5a*S*,6S,9*R*,9a*R*)-C₃-cannabielsoïne, acide cannabinolique A, cannabinol, cannabinol méthyl éther, cannabinol-C₄, cannabivarine, cannabinol-C₂, cannabiorcol-C₁, cannabinodiol, cannabinodivarine, (-)-*trans*- cannabitriol, (+)*-trans-*cannabitriol , (±)-*cis*-cannabitriol , (±)-*trans*-cannabitnol-C3, (-)-*trans*-10-éthoxy-9-hydroxy-Δ^{6a(10a)}-tétrahydrocannabinol, *trans*-10-éthoxy-9-hydroxy-Δ^{6a(10a)}-tétrahydrocannabivarine-C₃, 8,9-dihydroxy- Δ^{6a(10a)}-tétrahydrocannabinol, l'acide cannabidiolique tétrahydrocannabitriol ester, déhydrocannabifurane, cannabifurane, cannabichromanone, cannabichromanone-C₃, cannabicoumarinone-Cs, cannabicitran , 10-oxo-Δ^{6a(10a)}-tétrahydrocannabinol, (-)-Δ⁹-(6a*S*,10a*R-cis*)*-* tétrahydrocannabinol, cannabiglendol-C₃, (-)-(6a*R*,9*S*,10*S*,10a*R*)-9,10-dihydroxyhexahydrocannabinol, (-)-6a,7,10a-Trihydroxy-Δ⁹-tétrahydrocannabinol, (±)-Δ⁷-*cis-*(1*R*,3*R*,6*S*)-isotétrahydrocannabivarine-C₃, (-)-Δ⁷-*trans*-(1R,3R,6R)-isotétrahydrocannabivarine-C₃, (-)-Δ⁷- *trans*-(1*R*,3*R*,6*R*)-isotétrahydrocannabinol-C₅, anandamide , 2-arachidonoylglycérol, 2-arachidonyl glycéryl éther, N- arachidonoyl dopamine, virodhamine, lysophosphatidylinositol, nabilone, rimonabant, diméthylheptyl pyrane, lévonantradol, l'acide ajulémique,

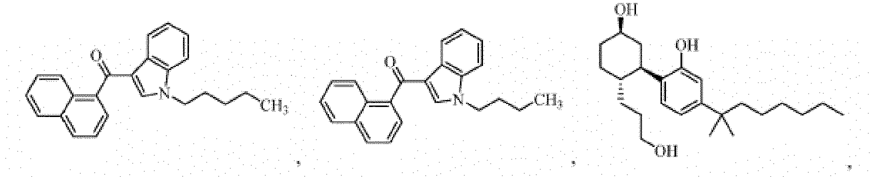

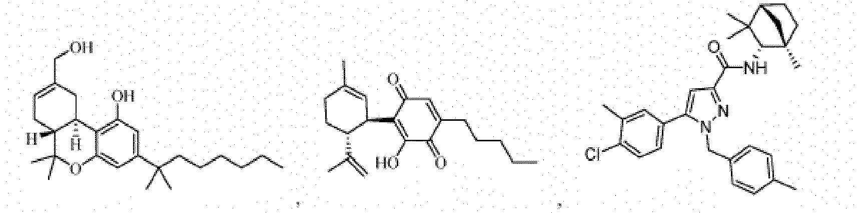

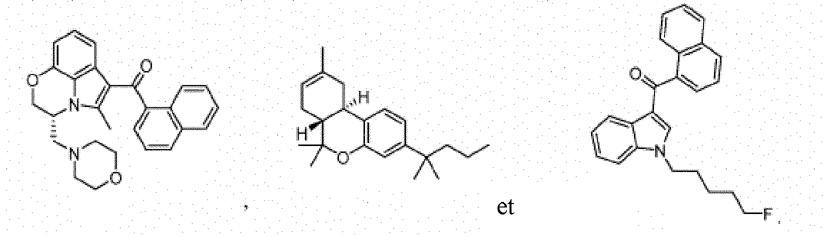

4. Film selon l'une quelconque des revendications 1 à 3, dans lequel l'API est Δ⁹-tétrahydrocannabinol, cannabidiol ou un mélange de ceux-ci.

5. Film selon l'une quelconque des revendications 1 à 4, dans lequel le sel d'alginate d'un cation monovalent est choisi parmi un alginate de sodium, un alginate de potassium et un alginate d'ammonium, et est de préférence un alginate de sodium.

6. Film selon l'une quelconque des revendications 1 à 5, dans lequel le film comprend de 25% à 99% en poids du sel d'alginate d'un cation monovalent ou le mélange de sels d'alginate contenant au moins un sel d'alginate d'un cation monovalent, de 0 % à 20 % en poids d'eau, et de 0,001 % à 75 % en poids de l'API.

7. Film selon l'une quelconque des revendications 1 à 6, dans lequel le film comprend de 29% à 93% en poids du sel d'alginate d'un cation monovalent ou le mélange de sels d'alginate contenant au moins un sel d'alginate d'un cation monovalent, de 5% à 15% en poids d'eau, et de 0,15% à 50% en poids de l'API.

8. Film selon l'une quelconque des revendications 1 à 7, dans lequel le film comprend en outre au moins un plastifiant qui est choisi parmi le sorbitol, le glycérol et une combinaison de ceux-ci, de préférence à la fois le sorbitol et le glycérol, et :
(a) un agent alcalinisant qui est éventuellement l'hydroxyde de sodium aqueux ; ou
(b) un SEDDS comprenant (i) une phase huileuse, (ii) au moins un tensioactif, de préférence au moins deux tensioactifs, et (iii) un solubilisant ; ou
(c) un solvant pharmaceutiquement acceptable non aqueux, de préférence la triacétine.

9. Film selon la revendication 8, dans lequel le film comprend en outre de 0% à 40% en poids de sorbitol et de 0% à 40% en poids de glycérol.

10. Film selon la revendication 8 ou la revendication 9, dans lequel le film comprend en outre le xylitol.

11. Film selon l'une quelconque des revendications 1 à 10 pour une utilisation dans le traitement d'un patient humain.

12. Film selon l'une quelconque des revendications 1 à 10 pour une utilisation dans le traitement d'une maladie ou d'un état chez un patient humain, dans lequel la maladie ou l'état est choisi parmi : le cancer ; la démence ; la maladie d'Alzheimer ; la sclérose latérale amyotrophique ; la dystonie ; l'épilepsie; la maladie de Huntington ; la sclérose en plaques ; la maladie de Parkinson ; la spasticité; le syndrome de Tourette ; la maladie du côlon irritable (IBD) ; la maladie de Crohn ; la rectocolite hémorragique ; l'anorexie ; la cachexie ; les nausées et/ou vomissements d'origine cancéreuse ; la cachexie induite par le cancer ; le glaucome ; la douleur chronique ; la douleur induite par le cancer ; la fibromyalgie ; la douleur neuropathique ; la dépendance ; l'anxiété ; le trouble bipolaire ; le trouble de stress post-traumatique ; la psychose ; la schizophrénie ; la sclérodermie ; et le diabète de type I, de préférence dans lequel la maladie ou l'état est choisi parmi : la démence ; la maladie d'Alzheimer ; l'épilepsie ; la maladie inflammatoire de l'intestin ; la maladie de Crohn ; la rectocolite hémorragique ; la douleur chronique ; la douleur induite par le cancer ; la fibromyalgie ; et les douleurs neuropathiques.

13. Film pour une utilisation selon la revendication 11 ou la revendication 12, dans lequel le film est administré dans la cavité buccale du patient humain.

14. Procédé de fabrication d'un film selon l'une quelconque des revendications 1 à 10, ledit procédé comprenant les étapes suivantes :
(a) soit les étapes consistant à :
(i) éventuellement, mélanger au moins un antioxydant dans de l'eau, ou dans un solvant mixte aqueux/organique, ou dans un ou plusieurs solvants organiques ;
(ii) mélanger l'API dans l'eau, ou dans un solvant mixte aqueux/organique, ou dans un ou plusieurs solvants organiques auxquels l'eau est ensuite ajoutée, ou dans la solution obtenue à l'étape (i), éventuellement dans lequel le pH de la solution est ajusté avant ou après l'ajout de l'API au niveau souhaité par l'ajout d'un acide ou d'une base approprié, généralement un acide ou un alcali aqueux dilué, plus généralement un alcali aqueux dilué, et de préférence dans lequel le pH de la solution est ajusté à 3,0 à 13,5 ;
(iii) éventuellement, soniquer la solution ;
(iv) éventuellement, mélanger un ou plusieurs excipients, agents aromatisants, composants tampons, activateurs de perméation, SEDDS (par exemple SMEDDS ou SNEDDS), agents chélatants, antioxydants et/ou agents antimicrobiens dans la solution obtenue à l'étape (ii) ou à l'étape (iii) ; et
(v) ajouter le sel d'alginate de cation monovalent dans des conditions appropriées pour entraîner la formation d'un moulage visqueux ; ou
en variante les étapes consistant à :
(i) mélanger le sel d'alginate de cation monovalent dans l'eau, jusqu'à l'obtention d'une dispersion sans grumeaux, et éventuellement ajouter un ou plusieurs excipients, agents aromatisants, composants tampons, activateurs de perméation, SEDDS (par exemple SMEDDS ou SNEDDS), agents chélatants, antioxydants et/ou agents antimicrobiens à la solution aqueuse soit avant soit après l'ajout du sel d'alginate ;
(ii) dissoudre séparément l'API dans l'eau, un solvant mixte aqueux/organique ou un ou plusieurs solvants organiques, éventuellement dans lequel au moins un antioxydant est prédissous dans le solvant, éventuellement dans lequel le pH de la solution est ajusté avant ou après l'ajout de l'API au niveau souhaité par l'ajout d'un acide ou d'une base approprié, généralement un acide ou un alcali aqueux dilué, plus généralement un alcali aqueux dilué, et de préférence dans lequel le pH de la solution est ajusté à 3,0 à 13,5 ; et
(iii) ajouter la solution obtenue à l'étape (i) à la solution obtenue à l'étape (ii) dans des conditions appropriées pour conduire à la formation d'un moulage visqueux ; ou
en variante les étapes consistant à :
(i) mélanger l'API dans une phase huileuse ;
(ii) prémélanger un tensioactif et un cosolvant, puis ajouter celui-ci à la solution obtenue ;
(iii) éventuellement, ajouter un ou plusieurs excipients, agents aromatisants, composants tampons, activateurs de perméation, agents chélatants, antioxydants et/ou agents antimicrobiens à l'eau à l'étape (i) sous mélange ;
(iv) ajouter l'eau, ou la solution obtenue à l'étape (iii), à la solution obtenue à l'étape (ii) sous agitation, de préférence une agitation continue, et plus préférablement dans lequel l'eau ou la solution obtenue à l'étape (iii) est ajoutée dans un mode goutte à goutte ; et
(v) mélanger le sel d'alginate de cation monovalent dans la solution, jusqu'à l'obtention d'une dispersion sans grumeaux, et éventuellement l'ajout supplémentaire d'eau pour moduler la viscosité de la coulée formée ; ou
en variante les étapes consistant à :
(i) mélanger l'API dans un agent solubilisant ;
(ii) ajouter la solution résultante à l'eau, de préférence sous mélange à fort cisaillement ;
(iii) éventuellement, ajouter un ou plusieurs excipients, agents aromatisants, composants tampons, activateurs de perméation, agents chélatants, antioxydants et/ou agents antimicrobiens ; et
(iv) mélanger le sel d'alginate de cation monovalent dans la solution, jusqu'à l'obtention d'une dispersion sans grumeaux, et éventuellement l'ajout supplémentaire d'eau pour moduler la viscosité de la coulée formée ;
(b) éventuellement, ajouter un ou plusieurs autres excipients, agents aromatisants, composants tampons, activateurs de perméation, SEDDS (par exemple SMEDDS ou SNEDDS), agents chélatants, antioxydants et/ou agents antimicrobiens au moulage obtenu à l'étape (a) ;
(c) éventuellement, laisser le moulage se désaérer ;
(d) verser le moulage sur une surface et étaler le moulage à l'épaisseur souhaitée ;
(e) sécher la couche coulée, typiquement à une température de 30 à 70°C, jusqu'à ce que la teneur en eau résiduelle du film soit de 0 à 20 % en poids et qu'un film solide soit formé ; et
(f) éventuellement, couper le film solide en morceaux de la taille souhaitée, en outre éventuellement placer ces morceaux dans des sachets, de préférence dans lequel les sachets sont fabriqués à partir d'aluminium doublé de PET, sceller les sachets et en outre éventuellement, les étiqueter.

15. Procédé selon la revendication 14, dans lequel après que le moulage visqueux est versé sur une surface, il est d'abord étalé sur une épaisseur de 2 mm au moyen d'un applicateur avec une hauteur de fente de 2 mm, puis est ensuite étalé sur une épaisseur de 1 mm au moyen d'un applicateur avec une hauteur de fente de 1 mm.
